# EUROPEAN PATENT SPECIFICATION

(11) **EP 3 316 856 B1**
(45) Date of publication and mention of the grant of the patent: **07.04.2021**
(21) Application number: 16733592.6
(22) Date of filing: 30.06.2016
(51) Int. Cl.: A61K 9/00, A61K 9/10, A61K 9/127, A61K 47/24, A61K 47/26, A61K 31/155, A61P 17/00, A61K 8/43

(54) **BLENDED FORMULATIONS**
MISCHFORMULIERUNGEN
FORMULATIONS MÉLANGÉES

(30) Priority: 30.06.2015 GB 201511478; 30.06.2015 GB 201511469
(43) Date of publication of application: 09.05.2018
(73) Proprietor: Sequessome Technology Holdings Limited, Valletta 1436 (MT)
(72) Inventor: HENRY, William, London Greater London WC1B 3RA (GB); GARRAWAY, Richard Wolf, London Greater London WC1B 3RA (GB)
(74) Representative: Lee, Nicholas John
(86) International application number: PCT/EP2016/065415
(87) International publication number: WO 2017/001617

(56) References cited:
- WO-A1-00/45774
- WO-A1-99/22703
- WO-A1-99/36053
- WO-A1-2013/171132
- WO-A1-2015/014965
- WO-A2-2008/039989
- US-A- 5 614 215

## Description

The present invention relates to a formulation comprising blends of formulations (or colloidal dispersions) and its topical application. In particular, the present invention provides a formulation comprising a first colloidal dispersion and a second colloidal dispersion, wherein each colloidal dispersion comprises deformable colloidal particles comprising a surfactant and optionally a phospholipid, and wherein one or more of the deformable colloidal particles of the first colloidal dispersion comprise a modified component comprising a first Agent of Interest (AOI), wherein the modified component is a lipid or surfactant covalently bonded to an AOI, such that the entire AOI is outside the particle membrane, and wherein the first AOI is zinc.

The formulation comprises at least two different types of colloidal dispersion comprising deformable colloidal particles, wherein the deformable colloidal particles comprise a non-ionic surfactant and/or a phospholipid. The deformable colloidal particles of the invention may comprise an agent of interest (AOI) or may be free of an AOI. The formulation may comprise an AOI that is not associated with the deformable colloidal particles. The present invention also includes kits comprising the formulation of the present invention and the use of the formulation in medicine, skin care and cosmetics.

Examples of colloidal dispersions that contain colloidal particles comprising a non-ionic surfactant and a phospholipid, wherein the colloidal dispersions are free of any pharmaceutically active AOI are known from WO 2010/140061, which describes the use of drug-free ("empty") vesicles for the treatment of deep tissue pain, specifically pain associated with osteoarthritis.

Further examples of colloidal dispersions that contain colloidal particles comprising a non-ionic surfactant and a phospholipid, comprising an AOI are known from WO 2015/014965. WO 2015/014965 describes the use of these vesicles for the topical administration of a therapeutic, metabolic or structural AOI that is "tethered" to the lipid and/or the surfactant component of the vesicles, such that the majority of the AOI is external to the vesicles.

None of these documents disclose or teach a formulation comprising blends or mixtures of different types of colloidal dispersions, nor do they disclose or teach such a formulation comprising an AOI that is not associated with the colloidal particles of the colloidal dispersions.

Citation of any reference in this section of the application is not an admission that the reference is prior art to the invention.

Colloidal dispersions comprising colloidal particles have been used in the past in attempts to treat skin conditions or as a cosmetic to improve the appearance of skin. Such colloidal dispersions have also been used to deliver AOIs into the body for the same purposes. In some instances, such as in WO 2013/171131, an active agent is applied directly to the skin and a drug-free colloidal dispersion is applied over the active agent to "push" it through the skin, it is thought, by way of hydrophilic forces. In other instances, such as in WO 2011/022707, drug-free vesicles themselves are used for treating atopic eczema, dishydrotic hand eczema, plaque type psoriasis, seborrheic eczema and acne vulgaris. In WO 2015/014965, examples of colloidal dispersions comprising AOIs are used for different purposes. For example, vesicles comprising anti-oxidants or vitamins are used for enhancing the skin's ability to repair and colloidal dispersions comprising vitamin D may be used to supplement sun cream in order to prevent vitamin D deficiency.

However, these drug-free (i.e. AOI-free) colloidal dispersions and colloidal dispersions comprising AOIs are applied separately. Therefore, if a user requires the effects of multiple formulations, for example, if they have a need to reduce acne-causing bacteria as well as to reduce sebum production, they would be required to first apply a colloidal dispersion comprising zinc to kill the bacteria and allow this to dry before applying a drug-free colloidal dispersion to remove the excess sebum. This results in a number of challenges for the user. For example, where an active agent is applied directly to the skin followed by the application of a colloidal dispersion, there is little or no dose control of the application of the active agent. An indeterminate amount of active agent is applied to the skin followed by an indeterminate amount of colloidal dispersion. It is impossible for the user to be certain about how much active agent is being delivered through the skin. In addition, the application of an active agent directly to the skin means that it is applied at a high concentration and is more likely to cause adverse skin reactions. Also, if two AOIs are applied without the use of colloidal dispersions, they can interact, so that the user does not know exactly what chemical combination of original products and reaction products they are receiving. Crucially, having to individually apply two or more colloidal dispersions is particularly time consuming for the user as each colloidal dispersion may take up to 10 minutes to dry.

The current invention circumvents these problems by combining the different colloidal dispersions into one easy to apply formulation where the individual AOIs have not interacted. The colloidal nature of these blended dispersions allows each dispersion to exist separately and stably until applied to the skin. As such, the colloidal dispersions may have a separate function and also be administered together as one formulation, in one easy application. This saves time for the user, ensures more accurate dosing (on a gram-for-gram basis there is a consistent ratio of the AOI to the colloidal particles) and reduces adverse skin reactions.

Accordingly, in a first aspect, the present invention provides a formulation comprising a first colloidal dispersion and a second colloidal dispersion, wherein each colloidal dispersion comprises deformable colloidal particles comprising a surfactant and optionally a phospholipid, and wherein one or more of the deformable colloidal particles of the first colloidal dispersion comprise a first agent of interest (AOI), wherein the modified component is a lipid or surfactant covalently bonded to an AOI, such that the entire AOI is outside the particle membrane, and wherein the first AOI is zinc.

By "one or more" it is meant that from about 1%, to about 5%, from about 5% to about 10%, from about 10% to about 20%, from about 20% to about 40%, from about 40% to about 60%, from about 60% to about 80%, from about 80% to about 85%, from about 85% to about 90%, from about 90% to about 92%, to about 94%, to about 96%, to about 97%, to about 98%, to about 99% or to about 100%, or from about 98% to about 100%.

Where the colloidal dispersions comprise at least one or more deformable colloidal particles comprising an AOI, it is possible that not all of the deformable colloidal particles will comprise an AOI. The AOI is added during the manufacture of the colloidal dispersions and may tether to some deformable colloidal particles but not others. For example, depending on the amount of AOI added, there may not be sufficient AOI to tether to every colloidal particle.

One or more of the deformable colloidal particles of the second colloidal dispersion may also comprise an AOI, in which case the AOI of the first colloidal dispersion and the AOI of the second colloidal dispersion are different.

One or more of the deformable colloidal particles of the first colloidal dispersion and/or one or more of the deformable colloidal particles of the second colloidal dispersion comprise one or more further AOIs and wherein each AOI is different.

The formulation may comprise one or more further colloidal dispersions comprising deformable colloidal particles comprising a surfactant and optionally a phospholipid and one or more of the particles of each dispersion may comprise a further AOI and the first AOI, second AOI and each further AOI are different. The formulation may include further colloidal dispersions comprising deformable colloidal particles comprising a surfactant and/or a phospholipid with no AOI.

The deformable colloidal particles of the same colloidal dispersion may comprise more than one type of AOI. For example, the deformable colloidal particles of the first colloidal dispersion may comprise a first AOI and a second AOI, wherein the first AOI and second AOI are different. The deformable colloidal particles of the same colloidal dispersion may comprise two, three, four, five or more different types of AOI. The different types of AOI may all be tethered (attached or bonded) to the colloidal particle within the dispersion in different combinations.

The formulation may comprise two or more colloidal dispersions comprising colloidal particles, wherein the colloidal particles of each dispersion comprise the same number or different numbers of different types of AOI's. For example, the formulation may comprise a first colloidal dispersion that comprises colloidal particles, which have two different types of AOI's attached (bonded or tethered) to them and a second colloidal dispersion that comprises colloidal particles, which also have two different types of AOI's attached to them.

Alternatively, the formulation may comprise a first colloidal dispersion that comprises colloidal particles, which have two different types of AOI's attached to them and a second colloidal dispersion that comprises colloidal particles, which have three different types of AOI attached to them. The formulation may comprise two or more colloidal dispersions, which comprise colloidal particles that do not have any AOI tethered to them or that have one, two, three, four, five or more different types of AOI tethered to them.

The colloidal dispersions are also known as "Sequessome™, Transfersome™ or Tethersome intermediates".

The deformable colloidal particles of the invention may be vesicles. The AOI may be incorporated into the vesicle membrane. The AOI may be bonded to a component of the vesicle membrane. More specifically, the AOI may be bonded to a surfactant component of the vesicle membrane or to a lipid component of the vesicle membrane. Both a surfactant component and a lipid component of the vesicle membrane may have an AOI bonded to them. The bond may be a covalent bond.

The AOI may be bonded to a component of the particle such that at least a portion of the AOI is on the external surface of the particle, and is external to the particle membrane. Preferably, the component to which the AOI is bonded is a lipid and/or a surfactant component. At least a portion means that of the total AOI that is external to the particle at least 5%, 10% or 20%, suitably 40%, or more than 50% of each molecule (in terms of size or volume of the molecule) is external to the membrane of the particle. Preferably, the majority of the AOI, more preferably the entire AOI molecule is external to the particle. The AOI may be covalently bonded to a component such that it presents wholly on the external surface of the particle.

By the AOI being "external to the particle", it is meant those AOI that are "facing outwards".

During one method of manufacture of the particles, whereby the surfactant or lipid component that is bonded or otherwise attached to the AOI is mixed with the unmodified components, the orientation of the modified molecule cannot be controlled. Thus, approximately 50% of the molecules to which the AOI is attached will be in the "incorrect" orientation, meaning that a portion of the AOI will be present in the lumen of the particle.

During another method of manufacture of the particles, whereby the surfactant or lipid component that is bonded or otherwise attached to the AOI is mixed with already formed particles, the surfactant or lipid component attached to the AOI will preferentially combine with the existing particle such that AOI is usually left external to the particle. Thus approximately 0% to 10% of the molecules to which the AOI is attached will be in the "incorrect" orientation, meaning that a small portion if any of the AOI will be present in the lumen of the particle. Of the modified molecules that are in the "correct" orientation such that the AOI is external to the particle, at least 50% of the AOI molecule itself, in terms of physical size/volume, is external to the vesicular membrane. The manufacturing process may result in a lower proportion of the AOI being external to the particle i.e. the external concentration of the AOI may be between 1% to 10% (including 2%, 3%, 4%, 5%, 6%, 7%, 8% or 9%) 10% to 50%, 15% to 45%, 20% to 40% or 25% to 30% (wt/vol) of the total AOI in the formulation. By external concentration it is meant the concentration of AOI that is available for release and/or to exert its therapeutic activity once the particles have penetrated the skin.

A particle of the formulation may have a single or a plurality of AOIs bonded to its external surface. Wherein a plurality of AOIs are bonded, the AOIs may all be the same, i.e. homogenous, or the AOIs may be different, i.e. heterogeneous.

The AOI to be bonded may be covalently or otherwise bonded directly to either the phospholipid or surfactant component of the particle or the lipid component of the particle. It may be desirable to use a link or bridge that is covalently or otherwise bonded to both the fatty acid, surfactant or lipid component and the AOI. In one example, if an inorganic AOI were to be added (for example a metal, a metal salt or a metal oxide), an additional linker, for example a metal chelating agent (such as EDTA), stearic acid or palmitic acid might first be conjugated to the particle component. In another example it may be desirous to use a longer molecule, for example a polymer (such as polyethylene glycol; PEG), to facilitate the efficacy of the bonding process and/or the effectiveness of the bound AOI. Such linkers/longer bridging molecules will be particularly of benefit when it is desirable to hold an AOI at such a distance from the particles in order to prevent it interfering with the membrane itself. This may occur if the AOI is particularly lipophilic.

The AOI may be bonded (or attached or tethered) to the surfactant component of the colloidal particle. The bonding to the surfactant may be directly onto the surfactant by ester bond if the molecule has a hydroxyl group. An alternative method of bonding is to substitute an atom or functional group of the surfactant (for example in the case of Tween, a polyethylene glycol polymer) with the AOI. A third method of bonding is directly to a fatty acid, optionally via an ester bond. If the AOI is an inorganic molecule then a further linking molecule can first be conjugated to the vesicle component, for example a metal chelating agent such as EDTA in the case of a metal salt. If it is desirous that the AOI be held at some distance from the vesicle in order to maximise its efficiency (for example to expose an active site on an additional ingredient that is an enzyme), then a linking molecule, for example a polymer chain (for example polyethylene glycol) may be bonded to both a component of the colloidal particle and the AOI.

The AOI may be attached (bonded or tethered) to the lipid component of the colloidal particle. The bonding to the lipid might be achieved via any of the glycerol hydroxyl groups by an ester bond, for example by eliminating a fatty acid and replacing with the AOI. Alternatively, the method of attachment may be by replacement of the phosphatidyl moiety such that the final molecule has two fatty acid chains together with the tethered AOI. The modified lipid inserts in the aliphatic region as normal and with the free rotation available on the glycerol template, the tethered AOI would locate on the outside of the vesicle. An amide bond may be used for a more stable alternative, should the AOI be required to be tethered to the deformable colloidal particle for a longer duration. This may be desirable, for example, if the target for the AOI is deeper tissue, such as the reticular layer of the dermis, rather than the upper dermal layers (e.g. the epidermis). A combination of less stable and more stable bonds may be used (e.g. ester and amide, respectively) to achieve staggered release of the AOI.

The method of bonding to any component may be hydrolysable or non-hydrolysable. If it is desirable that the AOI should be detached once within or under the skin, the link should be hydrolysable. If it is desirable that the bonded AOI should remain bound to the vesicle once within or under the skin, the link should be non-hydrolysable.

The AOI may be covalently bonded or conjugated to a membrane component; the bond may be hydrophilic or hydrophobic or hydrostatic; the bond may be a hydrogen bond or an ionic bond.

The terms "bonding", "attaching" and "tethering" are used herein throughout interchangeably to encompass all the bonds mentioned above.

The AOI may be selected from the group consisting of an element, a peptide, a protein, a micronutrient, an ion, an inorganic salt, a small molecule, an amino acid, a carbohydrate, a lipid, a macromolecule or a macrocyclic molecule.

The AOI may be a skin structural protein (such as elastin or collagen), a therapeutic protein, porphyrin or chromophore containing a macromolecule, a vitamin (such as vitamin C, D or E) titanium dioxide, zinc, zinc oxide, zinc stearate, melanin or a melanin analogue. The AOI may be a peptide or synthetic organic chemical such as an anti-inflammatory drug, such as an NSAID.

The first AOI is zinc. The second AOI and/or each further AOI may be selected from the group consisting of zinc, ascorbate (vitamin C), vitamin D, tocopherol (vitamin E), tetrapeptide, tripeptide, salicylic acid or menthol. Specifically, the AOI may be selected from the group consisting of zinc stearate, palmitoyl ascorbate (or palmitoyl ascorbic acid (PAA)), palmitoyl tripeptide, palmitoyl tetrapeptide, salicylic acid or menthol. More specifically, the AOI may be selected from the group consisting of zinc stearate, palmitoyl ascorbate, palmitoyl tripeptide-1, palmitoyl tetrapeptide-7, tocopherol linoleate, salicylic acid or menthol. The salicylic acid may be myristyl salicylate or tridecyl salicylate.

Peptides, such as tetrapeptide-7 or tripeptide-1, may be bonded to a fatty acid or surfactant component of the vesicle membrane. Tetrapeptide-7 may be useful in fighting inflammation and act to stimulate skin regeneration by way of collagen production. This means that it is particularly useful in skin care, and anti-ageing products. Tripeptide-1 has a similar action. Efficient delivery through the external layer of skin may provide increased effects at lower levels/concentrations thus minimising possible side effects resulting from the suppression of interleukins.

Vitamin C (water-soluble ascorbate) is a cofactor in many enzymatic reactions including several collagen synthesis reactions. These reactions are important in wound healing and in preventing bleeding from capillaries. Therefore, the current invention includes associating ascorbate with a vesicle component, for incorporation both into skincare and sun care preparations to ameliorate damage to collagen, and for incorporation into wound care products.

Vitamin E has important anti-oxidant properties and is able to reduce the signs of ageing and sun damage. Linoleate is the ester of linoleic acid, an omega-6 fatty acid, which has anti-inflammatory and moisturisation properties. Tocopheryl linoleate combines the properties of both vitamin E and linoleate to reduce the signs of ageing and to moisturise the skin. Therefore, vitamin E or tocopheryl linoleate may be associated with a vesicle component for incorporation into anti-ageing products and skin repair products.

Vitamin D may be incorporated into formulations in accordance with the present invention by association with a vesicle component.

Mammalian skin makes vitamin D₃ through the action of UV radiation on its precursor, 7-dehydrocholesterol, and supplies about 90 percent of our vitamin D. Sunscreen absorbs ultraviolet light and prevents it from reaching the skin.

When the formulation comprises vitamin D₃ (which is not to say that it also does not comprise vitamin C and/or E) the formulation may be incorporated into a sunscreen product, a sun block product, an after-sun product or other skincare or cosmetic product to supplement low vitamin D levels. Low vitamin D levels may be caused by low light conditions, or use of sunblock, which can prevent the manufacture of vitamin D within the body.

Zinc is an antioxidant and antimicrobial. It is beneficial for the immune system and helps to prevent ageing, to speed up wound healing, to kill microbes and bacteria and to down-regulate sebum production by sebaceous glands. It is currently used in a wide range of topical skin preparations, such as in sun creams to protect against sunburn and in antidandruff shampoo to kill dandruff causing fungus. It is also used in the treatment of acne. In the present invention, zinc is tethered to vesicle components for incorporation into skin care products that can, for example, be applied to red, dry, itchy skin, to red, irritated, scaly skin or to acne prone skin in order to improve these conditions.

Salicylic acid is a beta hydroxy acid (BHA) with the formula C₆H₄(OH)COOH. It can be obtained from the bark of willow trees. Salicylic acid is an important active metabolite of aspirin (acetylsalicylic acid), which acts in part as a pro-drug to salicylic acid. It is widely used as an analgesic and it also has anti-inflammatory, anti-pyretic, anti-diabetic, bactericidal and antiseptic effects. As a result, it is an important ingredient in pain-killers, topical anti-acne products, rubefacient products, skin-care products for the treatment of conditions such as psoriasis, calluses, ichthyosis and warts, shampoos for the treatment of dandruff, suntan and sunscreen products, mouthwashes and dentifrices. It can also be used for the prevention or treatment of uneven skin tone caused by, for example, darker melanic spots and liver spots. Salicylic acid can be used in the form of myristyl salicylate or tridecyl salicylate.

A plurality of AOIs may be bonded to a lipid or surfactant component to present on the external surface of the particle so that once taken through the skin, they continue to present on the surface of the particle. Examples include anti-oxidants; vitamins; inorganic compounds such as TiO₂ and ZnO; porphyrin molecules for use in photodynamic therapies or any of the AOIs mentioned above.

As will be appreciated, other AOIs may be tethered to the deformable colloidal particles of the invention in order to treat a wide variety of diseases.

The formulation may or may not contain any known therapeutic agent, other than the AOI bonded to the particles. The formulation comprising an AOI may or may not be free of any further AOI. A further AOI may or may not be associated with the colloidal particles.

The AOI may be an agent of interest, such as a cosmetic, that does not have any biological or pharmacological effect or it may be a biologically active or pharmaceutically active agent.

The colloidal dispersions of the formulation comprise at least two phases; a dispersed phase comprising the suspended deformable colloidal particles and a continuous phase, comprising the medium of suspension (or matrix). The formulation may comprise two, three, four or more dispersed phases. The dispersed phases may comprise different types of colloidal particle such as different types of vesicle, for example Sequessomes™, Transfersomes™, Tethersomes, micelles and liposomes. The formulation may comprise one or more dispersed phases comprising deformable colloidal particles such as Sequessomes™,Transfersomes™ or Tethersomes and optionally one or more dispersed phases comprising non-deformable colloidal particles such as micelles and/or non-deformable vesicles such as liposomes. The formulation may comprise two or more types of deformable colloidal particle.

Usually, the term "Transfersome™" is used to refer to a deformable phospholipid and surfactant vesicle, which is associated with an AOI, in particular where the AOI is held either in the lumen of the vesicle or bound within the vesicle's membrane. As described herein, the term "Sequessome™" is usually used to refer to the deformable phospholipid and surfactant vesicle itself. If an AOI is 'tethered' to the vesicle, such that it is held outside the external surface of the membrane, the vesicle (or Sequessome™) may be referred to as a 'Tethersome'.

The AOI may be present in the continuous phase. Preferably, this continuous phase comprises a non-lipid phase, most preferably an aqueous phase.

Alternatively, the AOI may be present in a second dispersed phase (the first dispersed phase comprising the deformable colloidal particles). For example, the AOI may be associated with non-deformable colloidal particles such as micelles or non-deformable vesicles such as liposomes.

The formulation may be a liquid, cream, lotion, ointment, gel, solution, spray, lacquer or film forming solution. Preferably, the formulation may be a gel.

As mentioned above, the formulation of the first aspect of the invention may comprise an AOI which is not associated with the deformable colloidal particles in the formulation.

As used herein, the phrase "not associated with" means that the AOI is not attached to or contained within the deformable colloidal particle. In particular, the AOI is not tethered, bound or otherwise secured to the deformable colloidal particle, is not contained within the structure of the deformable colloidal particle, including the lipid bilayer or the fluid enclosed by the lipid bilayer, is not encapsulated within the deformable colloidal particle or in any other way directly associated with the deformable colloidal particle. Rather, the AOI is present in a different phase of the composition from the deformable colloidal particles. For example, the AOI may be present within the continuous phase of the composition, for example dissolved in the continuous phase. Alternatively, the AOI may be present within a different dispersed phase of the composition, for example in the form of insoluble aggregates or associated with non-deformable colloidal particles such as micelles and non-deformable vesicles such as liposomes. Preferably, the AOI is in a form in which it would not normally pass through the skin, without the assistance of deformable colloidal particles.

The formulation of the present invention is multiphasic because, as mentioned above, it comprises at least two phases; a dispersed phase comprising the suspended deformable colloidal particles and a continuous phase comprising the medium of suspension. In a multiphasic system there are discrete particles, assemblies or bodies dispersed or suspended within a medium or matrix. Each phase is a separate solid or liquid entity with a detectable phase boundary. In colloidal systems, the particle size of each phase is too small for observation with the naked eye. However, the multiphasic nature of the system can be demonstrated by applying a narrow beam of light, a Tyndall Beam, the passage of which is visible through the solution due to scattering of the light by the phase boundary(ies). The passage of such a beam of light through a single phase solution would not be visible.

The formulation of the present invention may comprise more than two phases. For example, the formulation may comprise two, three, four or more dispersed phases, in addition to the continuous phase. Two such dispersed phases may comprise the deformable colloidal particles which drive the AOI through the skin. A third dispersed phase may comprise the AOI, for example in the form of insoluble aggregates or associated with non-deformable colloidal particles. Other dispersed phases may also be provided comprising further deformable and/or non-deformable colloidal particles.

The formulation may comprise two or more colloidal dispersions.

Preferably, the continuous phase comprises a non-lipid phase. The continuous phase may be selected depending upon the identity of the AOI and whether the AOI is to be dissolved in the continuous phase or provided as a dispersed phase. Preferably, the continuous phase is an aqueous phase.

Whilst an AOI may be provided in a separate phase from the deformable colloidal particles, under certain circumstances, which will be well known to those of skill in the art, some of the AOI may partition into the membrane or the fluid core of the deformable colloidal particles.

Preferably, only a negligible or insignificant amount of the AOI provided in a separate phase from the deformable colloidal particles partitions into the deformable colloidal particles whilst a significant amount of the AOI remains in a separate phase. Preferably, about 5% or less, about 4% or less, about 3% or less, about 2% or less, about 1% or less, about 0.5% or less, about 0.1% or less or about 0% of the AOI partitions into the deformable colloidal particles. Preferably, at least about 95.0%, at least about 96.0%, at least about 97.0%, at least about 98%, at least about 99.0%, at least about 99.5%, at least about 99.9% or about 100% of the AOI in the formulation remains in a separate phase from the deformable colloidal particles.

The formulation according to the first aspect of the present invention may comprise two significant populations of the same AOI; a first population that is not associated with the deformable colloidal particles and a second population that is associated with the deformable colloidal particles, for example, tethered to the deformable colloidal particles. In such embodiments, the first population of the AOI will usually penetrate less deeply into the skin than the second population of the AOI, providing effects at different depths of tissue.

Where the formulation of the first aspect of the present invention comprises two such populations of the same AOI, the proportion of the first population to the second population is designed according to the separate purposes that each of the populations is designated to perform. Preferably, the ratio of the first population to the second population is within the range 20:80 to 80:20, 30:70 to 70:30, 40:60 to 60:40 or approximately 50:50.

As used herein, the phrase "Agent of Interest" or "AOI" includes but is not limited to biologically active or pharmaceutically active agents. A biologically or pharmaceutically active agent is herein defined as an agent that has pharmacological, metabolic or immunological activity. This may include nutraceuticals or pharmaceuticals. Where necessary, the AOI has preferably received regulatory approval.

The AOI may be an antiseptic, an antibiotic, an anaesthetic, an analgesic, a skin lightener, an antihistamine, a steroid, an anti-inflammatory agent, an anti-viral, sun block, moisturiser, nicotine, an anti-fungal, an antimicrobial, a nutraceutical, an essential oil or a hormone.

Preferably, the AOI that is not associated with the deformable colloidal particles is selected from the group including but not limited to chlorhexidine or a salt thereof, capsaicin, salicylic acid or a salt or ester thereof, glucosamine or a salt thereof, an amide of glucosamine or a salt thereof, chondroitin or a salt thereof, caffeine or tocopherol.

If the AOIs listed above are added to the organic phase of the formulation during manufacture (i.e. prior to formation of the deformable colloidal particles), some AOI may be present inside the lumen of the colloidal particles and some AOI may be present in the continuous phase of the formulation (i.e. not associated with the colloidal particles). The distribution of the AOI may depend on the particular nature of the AOI that is included in the formulation.

Chlorhexidine, *N*,*N''''*1,6-Hexanediylbis[*N'*-(4-chlorophenyl)(imidodicarbonimidic diamide)], is a cationic polybiguanide (bisbiguanide). It is an antimicrobial compound that is effective against a wide range of gram-positive and gram-negative bacteria and some fungi and viruses. It is widely used in topical disinfectants, cosmetics and pharmaceutical products, including wound dressings and mouthwashes. It is also used for the treatment of acne.

Chlorhexidine may be frequently used in the form of a salt. Preferably, the salt of chlorhexidine is selected from the group consisting of chlorhexidine dihydrochloride, chlorhexidine diacetate, chlorhexidine gluconate and chlorhexidine digluconate. Most preferably, the formulation comprises chlorhexidine gluconate.

The chlorhexidine or a salt thereof of the present invention may be provided as a 10% solution, a 20% solution, a 30% solution, a 40% solution or a 50% solution. Preferably, chlorhexidine or a salt thereof may be provided as a 20% solution. The formulation of the present invention may comprise 0.1 to 2.5%, more preferably 0.5 to 2.0%, most preferably 1.5% by weight of the 10% solution, 20% solution, 30% solution, 40% solution or 50% solution. Preferably, the formulation of the present invention may comprise 0.1 to 2.5%, more preferably 0.5 to 2.0%, most preferably 1.5% by weight of the 20% solution. The formulation may therefore comprise from 0.02% to 0.1%, from 0.1% to 0.2%, from 0.2% to 0.3%, from 0.3% to 0.4%, from 0.4% to 0.5%, from 0.5% to 1.1% or from 1.1% to 1.25% of chlorhexidine. Preferably, the formulations of the present invention comprise chlorhexidine or a salt thereof at a concentration below its critical micelle concentration.

The chlorhexidine or salt thereof may be present in the formulation in the form of insoluble aggregates or micelles. Where the AOI not associated with the deformable colloidal particles comprises chlorhexidine or a salt thereof, the continuous phase is preferably aqueous.

Capsaicin, 8-methyl-*N*-vanillyl-6-nonenamide, is a capsaicinoid which is produced as a secondary metabolite by the fruits of plants in the genus *Capsicum,* including chili peppers. It is believed to act as a deterrent against predation by certain mammals and also as an anti-fungal agent. In medicine, capsaicin is used as an analgesic, particularly for the temporary relief of pain associated with arthritis, backache, strains and sprains, fibromyalgia and neuralgia. It is also used to reduce itching associated with conditions such as psoriasis.

The formulations of the present invention may comprise 0.01 to 0.1% capsaicin, more preferably 0.01 to 0.05%, most preferably approximately 0.025%.

The capsaicin may be dissolved in the continuous phase, when the continuous phase comprises a suitable solvent, for example ether, benzene and/or an alkane.

Preferably, the capsaicin is present in the formulation in the form of insoluble aggregates or associated with non-deformable colloidal particles such as liposomes comprising one or more phospholipids. Preferably, the capsaicin is bound in the liposome membrane. Under such circumstances, the continuous phase is preferably aqueous.

As mentioned above, salicylic acid is widely used as an analgesic and it also has anti-inflammatory, anti-pyretic, anti-diabetic, bactericidal and antiseptic effects.

Salicylic acid can be used in the above applications in the form of a salt or ester. Salts of salicylic acid include calcium salicylate, magnesium salicyalte, MEA-salicylate, potassium salicylate, sodium salicylate and TEA-salicylate. Esters of salicylic acid include butyloctyl salicylate, C12-15 alkyl salicylate, capryolyl salicylic acid, hexyldecyl salicylate, isocetyl salicylate, isodecyl salicylate, ethylhexyl salicylate, methyl salicylate, myristyl salicylate and tridecyl salicylate.

Preferably, the formulation of the first aspect of the present invention comprises an ester of salicylic acid, most preferably myristyl salicylate or tridecyl salicylate.

The composition of the present invention may comprise salicylic acid or a salt or ester thereof in concentrations appropriate to the intended use and within any regulatory limitations.

Salicylic acid and salts and esters thereof are water soluble and thus, where the continuous phase is aqueous, the salicylic acid or a salt or ester thereof may be dissolved within the continuous phase. At least some of the salicylic acid or salt or ester thereof may also partition into the membrane of the deformable colloidal particles of the present invention and/or into the membrane of one or more non-deformable colloidal particles also present in the formulation. The formulations of the present invention preferably comprise about 0.05% to 2.5% by weight of salicylic acid or a salt or ester thereof.

In solution, chlorhexidine and salts thereof, capsaicin, and salicylic acid and esters and salts thereof may form micelles, vesicles or insoluble aggregates.

Glucosamine, or (3R,4R,5S)-3-Amino-6-(hydroxymethyl)oxane-2,4,5-triol, is an amino sugar and a prominent precursor in the biochemical synthesis of glycosylated proteins and lipids. It is commonly used as a dietary supplement, in particular for the treatment of osteoarthritis.

Glucosamine may be used in the form of an amide such as *N*-acetylglucosamine.

The formulations of the present invention may comprise salts of glucosamine or the amides of glucosamine, such as glucosamine sulfate, glucosamine hydrochloride and N-acetylglucosamine sulphate.

The formulations of the present invention may comprise about 0.01 to 1.0% by weight of glucosamine, amides of glucosamine or salts thereof, more preferably about 0.05 to 0.5% by weight, more preferably about 0.1 to 0.3% by weight, most preferably approximately 0.2% by weight.

Glucosamine, amides of glucosamine and salts thereof are water soluble. Thus, where the continuous phase of the formulation is aqueous, these may be dissolved within the continuous phase.

Chondroitin is a glycosaminoglycan (GAG) composed of a chain of alternating sugars, N-acetylgalactosamine and glucuronic acid. It is usually found attached to proteins as part of a proteoglycan. A chondroitin chain can have over 100 individual sugars, each of which can be sulfated in variable positions and quantities.

Chondroitin may be used in the form of a salt, such as chondroitin sulphate, chondroitin gluconate and chondroitin hydrochloride. Chondroitin sulfate is an important structural component of cartilage and has become a widely used dietary supplement for treatment of osteoarthritis.

The formulations of the present invention may comprise about 0.01 to 1.0% chondroitin or salts thereof by weight, more preferably about 0.05 to 0.5% by weight, more preferably about 0.1 to 0.3% by weight, most preferably approximately 0.2% by weight.

Chondroitin and salts thereof, such as chondroitin sulphate, are water soluble. Thus, where the continuous phase of the formulation is aqueous, these may be dissolved within the continuous phase.

Caffeine, or 1,3,7-Trimethylpurine-2,6-dione, is a methylxanthine alkaloid which is naturally found in the seeds, nuts, or leaves of a number of plants native to South America and East Asia. Whilst commonly used as a stimulant of the central nervous system, caffeine is also a powerful antioxidant and anti-inflammatory that can be topically applied to reduce wrinkles, eye puffiness and dark under-eye circles. It may also be used to prevent sun damage to the skin.

The formulations of the present invention may comprise about 0.001 to 1.0% caffeine by weight, more preferably about 0.01 to 0.5% by weight, more preferably about 0.02 to 0.1% by weight, more preferably about 0.02 to 0.08% by weight, most preferably approximately 0.05% by weight.

Caffeine is water soluble. Thus, where the continuous phase of the formulation is aqueous, caffeine may be dissolved within the continuous phase. At least some of the caffeine may also partition into the membrane of the deformable colloidal particles of the present invention and/or one or more non-deformable colloidal particles also present in the formulation.

The formulations of the present invention may comprise more than one AOI that is not associated with the deformable colloidal particles. For example, a formulation of the present invention may comprise glucosamine or a salt thereof and/or an amide of glucosamine or a salt thereof, in combination with chondroitin or a salt thereof. For example, the formulation of the present invention may comprise glucosamine hydrochloride and/or N-acetylglucosamine sulphate in combination with chondroitin sulphate.

Tocopherols are a class of organic chemical compounds (more precisely, various methylated phenols), many of which have vitamin E activity. There are four different forms of tocopherol; alpha, beta, gamma and delta. Tocopherols have a number of functions within the body. They have antioxidant and anti-inflammatory effects, which help protect cells from the damaging effects of free radicals which can lead to cardiovascular disease and cancer. Tocopherols are involved in healthy immune function, gene expression, blood circulation, protecting the health of nerves and preventing mental degeneration. Tocopherols also have an important role in skin care, protecting the skin from oxidative damage resulting from UV rays and pollution. Topical application of tocopherols can help reduce redness, sunburn and skin damage, including UV-induced tumour formation. Tocopherols also help to protect the cells that make collagen and elastin, providing an anti-ageing effect. Tocopherols are also an effective moisturiser, hydrating the skin and preventing further water loss, aiding in the reduction of fine lines and wrinkles. Tocopherols also reduce the healing time of wounds and can be used to help repair skin lesions and dry skin and treat skin conditions such as psoriasis and erythema.

Alpha-tocopherol is the form of vitamin E that is preferentially absorbed and accumulated in humans.

Tocopherol, preferably alpha-tocopherol, may be present in the continuous phase of the compositions of the present invention (as "free" tocopherol).

The compositions of the present invention may comprise about 0.01 to 1.0% by weight of "free" tocopherol, such as alpha tocopherol, more preferably 0.05 to 0.5% by weight, more preferably 0.1% to 0.3% by weight, most preferably approximately 0.2% by weight. A small amount of the "free" tocopherol may partition into the lumen of the deformable colloidal particles, but a significant amount will remain in the continuous phase.

Exemplary compositions according to the first aspect of the present invention comprising alpha tocopherol in the continuous phase are provided in Example 6 and Example 7.

Derivatives of tocopherols, for example esters such as tocopheryl linoleate, may also be used. Such derivatives may be tethered to the deformable colloidal particles in various compositions of the present invention, as discussed herein.

The formulation may comprise a least three colloidal dispersions, at least four colloidal dispersions or at least five or more colloidal dispersions.

The formulation of the first aspect of the invention comprises a surfactant and optionally a phospholipid from which the deformable colloidal particles are formed. The deformable colloidal particles may comprise one or more surfactants and no phospholipids, one or more phospholipids and no surfactant, or one or more surfactants in combination with one or more phospholipids.

Preferably, these deformable colloidal particles comprise a fluid core, for example an aqueous core, enclosed by a membrane, for example a bilayer membrane. Suitable bilayer membranes include phospholipid-surfactant bilayer membranes, non-ionic surfactant bilayer membranes and surfactant-cholesterol bilayer membranes. The term "bilayer" indicates that the membrane is two molecules in depth, wherein each molecule is arranged with its hydrophobic end directed inwards towards the opposite side of the membrane and its hydrophilic end directed outwards towards the surrounding aqueous media. For example, the bilayer membrane may comprise two layers of phosphatidylcholine molecules, wherein the hydrophobic tails of each molecule point toward each other and the hydrophilic heads of each molecule point away from each other. Preferably, the formulation comprises deformable colloidal dispersions comprising colloidal particles (i.e. vesicles) having a lipid bilayer in aqueous media. As used herein, the term "vesicle" includes unilamellar or multilamellar vesicles and micelles. The deformable colloidal particles may also comprise niosomes.

Preferably, these deformable colloidal particles are approximately 50nm to 250nm in diameter, approximately 60nm to 200nm in diameter, approximately 70nm to 180nm in diameter, approximately 80nm to 160nm in diameter, preferably 100 to 150nm in diameter, most preferably approximately 120nm in diameter.

Deformable colloidal particles of this invention are described in both WO 2010/140061 and in WO 2011/022707.

The formulation of the present invention is particularly suitable for topical administration as it comprises ultra-deformable (or flexible) colloidal particles (or microscopic spheres). These flexible forms of colloidal particles ("Sequessomes™, Transfersomes™ or Tethersomes") are vesicles, which are bilayer vesicles composed of surfactant and/or lipid, such as phospholipid. The specific amount of surfactant (when present), preferably non-ionic surfactant, modifies the lipid membrane to such an extent that the resulting particles are in a permanent liquid crystalline state. Since the surfactant (when present) also confers membrane stability, the particles are also ultra-deformable and stable (have reduced rigidity without breaking).

The colloidal dispersions of the formulation form into deformable colloid particles suspended in a suspension media, for example, an aqueous buffer. The particles are highly hydrophilic and this property, together with their ultra-deformability, is key to their ability to be transported across the skin. When the formulation of the invention is applied to the skin and allowed to dry, the rehydration driving force of the particles combined with their deformability gives rise to movement of the particles to areas of higher water content on and below the skin permeability barrier. This drives their movement through skin pores and intracellular gaps. It may be the specific ratio of lipid to surfactant that facilitates transdermal delivery of the particles. As the colloidal particles move through the skin, they push or pull the AOIs with them.

While not wishing to be limited to any mechanism of action, the formulations of the invention are able to form colloidal particles characterized by their deformability. The movement of the deformable colloidal particles through the pores and intracellular gaps carries with it the AOI, increasing the speed, depth and effectiveness of absorption of this compound.

The term "deformable" refers to the ability of the colloidal particles to easily change their properties, such as shape, elongation ratio and surface to volume ratio. The colloidal particles of this invention may be characterized by their ability to adjust their shape and properties to the anisotropic stress caused by crossing narrow pores in a semipermeable barrier such as the skin. Sufficient deformability implies that a colloidal particle can sustain different unidirectional forces or stress, such as one caused by pressure, without extensive fragmentation, which defines a "stable" colloidal particle.

A "barrier" in the context of this invention is a body with through-extending narrow pores, such narrow pores having a radius which is at least 25% smaller than the radius of the colloidal particles (considered as spherical) before said colloidal particles permeate through such pores.

The term "narrow" used in connection with a pore implies that the pore radius is significantly, typically at least 25%, smaller than the radius of the colloidal particle tested with regard to its ability to cross the pore. The necessary difference typically should be greater for the narrower pores. Using a 25% limit is therefore quite suitable for >150 nm diameter, whereas >100% difference requirement is more appropriate for the smaller systems, e.g., with <50 nm diameter.

Preferably, the deformability of the colloidal particles may be determined by the ability of the colloidal particles to penetrate a barrier with pores having an average pore diameter at least 25%, at least 30%, at least 35%, at least 40%, at least 55%, at least 50%, at least 55% or at least 60% smaller than the average particle diameter before the penetration. Most preferably, the deformability of the colloidal particles may be determined by the ability of the colloidal particles to penetrate a barrier with pores having an average pore diameter at least 50% smaller than the average particle diameter before the penetration.

The term "semipermeable" used in connection with a barrier implies that a solution can cross transbarrier openings whereas a suspension of non-adaptable aggregates (large enough for the above definition of "narrow" pores to apply) cannot. Conventional lipid vesicles (liposomes) made from any common phosphatidylcholine in the gel lamellar phase or else from any biological phosphatidylcholine/cholesterol 1/1 mol/mol mixture or else comparably large oil droplets, all having the specified relative diameter, are three examples for such non-adaptable aggregates.

The term "stable" means that the colloidal particles do not change their diameter spontaneously or under the transport related mechanical stress (e.g. during passage through a semipermeable barrier) unacceptably, which most often means only to a pharmaceutically acceptable degree. A 20-40% change is normally considered acceptable; the halving or doubling of the diameter of the colloidal particles is borderline and a greater change in diameter is typically unacceptable. Alternatively and very conveniently, the change in diameter of the colloidal particles resulting from pore crossing under pressure is used to assess system stability; the same criteria are then applied as for "narrow" pores, *mutatis mutandis.* To obtain the correct value for diameter change, a correction for flux/vortex effects may be necessary. These procedures are described in greater detail in the publications of the applicant in Cevc et. al., Biochim. Biophys. Acta 2002; 1564:21-30.

Non-destructing passage of colloidal particles through narrow pores in a semi-permeable barrier is thus diagnostic of deformability. If pore radius is two times smaller than the average radius of the colloidal particles, the colloidal particles must change their shape and surface-to-volume ratio at least 100% to pass without fragmentation through the barrier. An easy and reversible change in shape inevitably implies high deformability of the colloidal particles and requires large surface-to-volume ratio adaptation. A change in surface-tovolume ratio *per se* implies: a) high volume compressibility, *e.g.* in the case of compact droplets containing material other than, and immiscible with, the suspending fluid; b) high membrane permeability, e.g. in the case of colloidal particles that are free to exchange fluid between inner and outer vesicle volume.

Methods of testing deformability which may be used to characterise the composition of the invention are set forth in WO 2010/140061 and US Patent Application Nos. 2004/0071767 and 2004/0105881.

Where a formulation in accordance with the present invention comprises two or more colloidal dispersions comprising deformable colloidal particles comprising an AOI and, in some cases, an AOI not associated with the colloidal particles, the formulation is able to exert its effects in different layers of the skin. The colloidal particles are able to efficiently penetrate the skin and deliver the AOI to deeper layers of the dermis, whilst the AOI agent may remain on the surface of the skin or in the upper layers. This ensures that each area of the skin can be targeted in a way that most effectively treats the specific condition.

In a second aspect, the present invention provides a formulation comprising a first colloidal dispersion and a second colloidal dispersion, wherein each colloidal dispersion comprises deformable colloidal particles comprising a surfactant and optionally a phospholipid and one or more of the deformable colloidal particles of the first colloidal dispersion comprise a first agent of interest (AOI), wherein the modified component is a lipid or surfactant covalently bonded to an AOI, such that the entire AOI is outside the particle membrane, and wherein the first AOI is zinc for use in treating or preventing a disease.

In a third aspect, the present invention provides a formulation comprising a first colloidal dispersion and a second colloidal dispersion, wherein each colloidal dispersion comprises deformable colloidal particles comprising a surfactant and optionally a phospholipid and one or more of the deformable colloidal particles of the first colloidal dispersion comprise a first agent of interest (AOI), wherein the modified component is a lipid or surfactant covalently bonded to an AOI, such that the entire AOI is outside the particle membrane, and wherein the first AOI is zinc for cosmetic treatment of a subject or for use in skin care.

The formulation may be used for treating or preventing acne. The formulation may be used for treating or preventing dry skin, itchy skin, red skin, irritated skin, scaly skin, ageing skin, thinning skin, periorbital skin, uneven skin tone, photo-aged skin or dermatitis. The formulation may also be used for cosmetic treatment of these conditions i.e. to improve the appearance of skin affected by these conditions.

Treating or preventing periorbital skin may be the treatment or prevention of wrinkles around eyes (i.e. around the eye sack), fine lines around eyes, dark circles around eyes, eye bags, sagging skin around eyes, hyperpigmentation around eyes or puffiness around eyes.

The use for which the formulation of the present invention is provided will depend at least in part on the identity of the first AOI and/or the second AOI and/or any further AOI included in the formulation.

For example, where a formulation in accordance with the first aspect of the invention comprises a first drug-free colloidal dispersion, a second colloidal dispersion comprising deformable colloidal particles tethered to zinc stearate and a further AOI that is chlorhexidine or a salt thereof, the formulation may be used for treating or preventing acne or dermatitis. This formulation can also be used to treat acne-prone skin, in particular to prevent the appearance of acne in acne-prone skin.

Acne is a common skin condition that affects many people at some point in their lives. It causes spots to develop on the skin, usually on the face, back and chest. The spots can range from surface blackheads and whiteheads, which are often mild, to deep, inflamed, pus-filled pustules and cysts, which can be severe and lead to scarring. The condition is caused by over-production of sebum which in turn results in increased and changed bacterial activity leading to inflammation and pus.

Current treatment options for acne range from simple cleansers to topical exfoliators and antibiotics, to oral retinoid preparations, which can be associated with serious side effects. Many patients either find the treatments ineffective or limited by side effects. The formulation of the present invention circumvents these problems.

The formulation may be useful for the treatment or prevention of acne, not only because the deformable colloidal particles increase the speed, depth and effectiveness of absorption of the zinc stearate and the chlorhexidine or salt thereof into the skin of the patient, but also because the colloidal particles of the first drug-free colloidal dispersion can facilitate the clearance of lipids from the skin by facilitating sebum removal. Furthermore, as discussed above, tethering zinc stearate to the deformable colloidal particles down-regulates sebum production by sebaceous glands and including chlorhexidine in the formulation kills bacteria and controls bacterial hyper-proliferation. As such, this formulation is able to clear existing sebum and reduce its production by denying microbes of their 'nutrient source', as well as killing exist microbes. These multiple functions help to calm inflamed spots, support epidermal renewal, remove impurities and tighten and constrict pores, resulting in a reduction in symptom recurrence (such as spots, hormonal blemishes, blackheads and oily skin) and further spread of infection.

Where a formulation in accordance with the first aspect of the invention comprises a first drug-free colloidal dispersion and a second colloidal dispersion comprising deformable colloidal particles tethered to zinc stearate, the formulation may be used for treating or preventing red, irritated, scaly skin. The colloidal particles of the first colloidal dispersion in this formulation sequester sebum and the zinc stearate attached to the colloidal particles of the second colloidal dispersion reduces sebum production. This dual function reduces colonisation of secretions by microbes, maintaining the normal skin environment and also reduces reappearance of symptoms by denying microbes their 'nutrient source'; the sebum on which they grow. In addition, the colloidal particles deliver nutrients to the skin. As such, this formulation reduces redness, swelling, flaking, weeping, blistering and skin erosion caused by excess oil and also promotes healthy skin balance and supports the skin's own renewal and repair mechanism.

For the avoidance of doubt, references herein to "drug-free" or "empty" are to colloidal dispersions that do not contain any non-lipid non-surfactant AOI that has a therapeutic purpose. These colloidal dispersions may comprise active agents such as antimicrobials, stabilisers and preservatives. However, it is to be understood that these agents are simply for improving the stability of the formulations and for increasing their shelf-life; they do not have a therapeutic purpose.

Where a formulation in accordance with the first aspect of the invention comprises a first drug-free colloidal dispersion, a second colloidal dispersion comprising deformable colloidal particles comprising palmitoyl ascorbate, a third colloidal dispersion comprising deformable colloidal particles comprising palmitoyl tetrapeptide-7 and a fourth colloidal dispersion comprising deformable colloidal particles comprising palmitoyl tripeptide-1, the formulation may be used for treating or preventing ageing and thinning skin.

The symptoms associated with ageing and thinning skin are well documented and publicised. Equally, there are numerous cosmetic treatments available that target such conditions but many of these treatments are not supported by robust data. In addition, each of these cosmetic treatments has to be applied separately to target each symptom of ageing, thinning skin. The formulations of the present invention simplify this and ensure that all of the symptoms of ageing can be effectively treated in one application of the formulation.

Palmitoyl ascorbate is a form of vitamin C that acts as antioxidant; shielding skin from free radical damage and reducing the appearance of brown spots from sun damage, boosting healthy collagen production and reducing inflammation and irritation. Palmitoyl tetrapeptide-7 and Palmitoyl tripeptide-1 both supress the production of excess interleukins and thus inhibit unnecessary inflammatory responses that lead to glycation damage and stiffening of tissues. In addition, these peptides boost the growth of connective tissues, naturally increasing the production of collagen in the skin and allowing the skin to heal and rejuvenate itself. Zinc stearate and tocopherol linoleate may be optionally added to enhance the performance of this blend: zinc is important in slowing the breakdown of collagen and tocopherol (vitamin E) is an important anti-oxidant that can stabilise vitamin C and enhance its effect. Therefore, this formulation can address all visible signs of skin photo-ageing, ensuring a more lifted appearance, better definition and noticeably firmer skin. It helps to build new collagen and elastin, slows collagen breakdown and strengthens the skin's underlying structure. It also preserves the extracellular dermal matrix, prevents free radical damage and hydrates and restores. It reduces fine lines, wrinkles, eye bags, dark circles, skin sagging and hyperpigmentation and improves skin tone, smoothness, firmness, elasticity, complexion.

Where a formulation in accordance with the first aspect of the invention comprises a first drug-free colloidal dispersion, a second colloidal dispersion comprising deformable colloidal particles comprising palmitoyl ascorbate, a third colloidal dispersion comprising deformable colloidal particles comprising palmitoyl tetrapeptide-7, a fourth colloidal dispersion comprising deformable colloidal particles comprising palmitoyl tripeptide-1 and a fifth colloidal dispersion comprising deformable colloidal particles comprising zinc stearate, the formulation may be used for treating or preventing red, dry, itchy skin. Red, dry, itchy skin may be due to conditions such as atopic eczema and dishydrotic hand eczema. As mentioned above, palmitoyl ascorbate works as an antioxidant to prevent skin damage, peptides promote collagen production to enable skin regeneration and zinc acts as an antimicrobial and reduces sebum production and also retards collagen breakdown. The colloidal particles themselves ensure deep dermal hydration with pure water and prevent water loss by forming an intimate evaporation barrier within the skin structure. As such, this formulation functions to defend the skin, to support the skin's own repair mechanism and to deliver nutrients to the skin to improve natural skin function, helping to avoid new flare-ups. This reduces redness, swelling, flaking, weeping, itching, skin thickening, blistering and skin erosion, which are often symptoms of skin conditions such as eczema.

Where a formulation in accordance with the first aspect of the invention comprises a first colloidal dispersion comprising deformable colloidal particles comprising tridecyl salicylate and alpha tocopherol or derivatives thereof and a second colloidal dispersion comprising colloidal particles comprising palmitoyl ascorbic acid, tocopheryl linoleate and alpha tocopherol or derivatives thereof, the formulation may be used for treating or preventing uneven skin tone. The alpha tocopherol in this formulation may be present within the lumen of the colloidal particles or in the continuous phase of the formulation (i.e. not associated with the colloidal particles). The tridecyl salicylate, palmitoyl ascorbic acid and tocopherol linoleate may be tethered to the colloidal particle. The formulation may improve the appearance of uneven skin tone in one or more of the following ways; reducing the appearance of wrinkles, hyperpigmentation, blemishes, dark under-eye circles, improving the appearance of skin texture and increasing skin elasticity.

This formulation may also be used for treating or preventing pain or discomfort by acting as a topical counter-irritant.

Where a formulation in accordance with the first aspect of the invention comprises an AOI that is selected from glucosamine or a salt thereof, an amide of glucosamine or a salt thereof, and/or chondroitin or a salt thereof, the formulation may be used for maintaining and prolonging joint health and/or for treating or preventing joint diseases (arthropathy), in particular degenerative joint diseases such as arthritis and osteoarthritis. These formulations may also be used to treat or prevent the pain associated with poor joint health.

Where a formulation in accordance with the first aspect of the invention comprises an AOI that is selected from glucosamine or a salt thereof, an amide of glucosamine or a salt thereof, and/or chondroitin or a salt thereof, the formulation may also be used for the manufacture of a medicament for maintaining and prolonging joint health and/or for treating or preventing joint diseases (arthropathy), in particular degenerative joint diseases such as arthritis and osteoarthritis, or the pain associated with poor joint health.

Where a formulation in accordance with the first aspect of the invention comprises an AOI that is selected from glucosamine or a salt thereof, an amide of glucosamine or a salt thereof, and/or chondroitin or a salt thereof, the AOI may or may not be associated with (i.e. bonded to) the deformable colloidal particle. If the AOI is associated with the deformable colloidal particle, a derivative a glucosamine or a salt thereof, an amide of glucosamine or a salt thereof, and/or chondroitin or a salt thereof may be used to allowing tethering (i.e. bonding) to the deformable colloidal particle.

The formulations of the present invention may be useful for maintaining and prolonging joint health and for treating or preventing joint diseases or the pain associated with poor joint health, not only because the deformable colloidal particles increase the speed, depth and effectiveness of penetration of the AOI into the joint of the patient, but also because the deformable colloidal particles can themselves alleviate or attenuate pain, such as that associated with osteoarthritis, as discussed above.

The volumetric blending ratio (by weight) of the individual colloidal dispersions (or Sequessome™, Transfersome™ or Tethersome intermediates) may differ in each formulation depending on the specific type of colloidal dispersion. For example, in the formulation that may be used to treat ageing thinning skin, the ratio of the palmitoyl ascorbate intermediate to the tetrapeptide intermediate and the tripeptide intermediate may be from about 1:1:1 to about 2:1:1, from about 1:2:1 to about 1:3:1, from about 1:1:2 to about 1:1:3, preferably from about 3:1:1 to about 5:1:1 and most preferably about 3:1:1. In the formulation that may be used to treat red, dry, itchy skin, the ratio of the palmitoyl ascorbate intermediate to the tetrapeptide intermediate, the tripeptide intermediate and the zinc stearate intermediate may be from about 1:1:1:1 to about 2:1:1:2, from about 3:1:1:3 to about 3:1:1:4, from about 4:2:2:5 to about 5:3:3:7, preferably from about 3:1:1:5 to about 4:2:2:6 and most preferably about 3:1:1:5. In the formulations that may be used to treat red, irritated, scaly skin and acne prone skin the ratio of the drug-free intermediate to the zinc stearate intermediate may be from about 1:1 to about 2:1, from about 3:1 to about 3:2, from about 3:2 to about 4:3 and most preferably from about 1:1.

Where the formulation comprises capsaicin as the AOI not associated with the deformable colloidal particles, the formulation may be used for treating or preventing one or more conditions selected from the group consisting of pain, including muscle pain, joint pain and itching. Capsaicin stimulates the nerve endings in the skin to create a warming sensation (without actually being related to a physical temperature change). As such, capsaicin containing formulations can be applied to the muscle or joint or any other tissue to relieve aches or pains. Formulations comprising capsaicin can be used prophylactically, being applied prior to exercise to create a warming sensation in the skin and underlying muscles and joints and thus prevent muscle soreness. The formulations may also be applied during or post-exercise to relieve aches and pains. Formulations of the invention comprising capsaicin are also provided for use as an analgesic medicament or an antipruritic medicament.

Where the formulation comprises salicylic acid or a salt or ester thereof, which is associated with and/or not associated with the colloidal particles, the formulation may also be used for treating or preventing one or more conditions selected from the group consisting of pain, including muscle pain and joint pain. Salicylate is an analgesic and has anti-inflammatory properties. As such, salicylate containing formulations can be applied to the muscle or joint or any other tissue to relieve aches or pains. Formulations comprising salicylate can be used prophylactically, being applied prior to exercise to relieve pain in the skin and underlying muscles and joints. The formulations may also be applied during or post-exercise to relieve aches and pains and reduce inflammation in the muscle and joint tissue.

The formulations comprising both capsaicin and salicylic acid or a salt or ester thereof may be used for treating or preventing one or more conditions selected from the group consisting of pain, including muscle pain, joint pain and itching. These formulations may have an enhanced effect in treating pain, such as muscle pain and joint pain, as they are able to both warm the skin and underlying muscles and joints and also provide an analgesic and anti-inflammatory effect in these areas.

The formulations comprising capsaicin and/or salicylic acid may be useful for the treatment or prevention of pain, not only because the deformable colloidal particles increase the speed, depth and effectiveness of absorption of the capsaicin and/or salicylic acid into the skin of the patient, but also because the colloidal particles can themselves alleviate or attenuate pain, such as that associated with osteoarthritis, as discussed above.

Formulations of the present invention are particularly suitable for patients with chronic, long-term pain associated with joint diseases such as osteoarthritis, who wish to avoid or minimise the consumption of pharmaceutical painkillers.

Where the AOI comprises glucosamine or a salt thereof, an amide of glucosamine or a salt thereof, and/or chondroitin or a salt thereof, the disease, disorder or condition is preferably a joint disease (arthropathy), in particular a degenerative joint disease such as arthritis or osteoarthritis, or the pain associated with poor joint health.

Also described herein is a method of maintaining and prolonging joint health, comprising topically applying a formulation according to the first aspect of the invention to the skin of a patient in need thereof, wherein the AOI comprises glucosamine or a salt thereof, an amide of glucosamine or a salt thereof, and/or chondroitin or a salt thereof. The AOI may or may not be associated with (i.e. bonded to) the deformable colloidal particles. When associated, derivatives of glucosamine and/or chondroitin will be used.

Formulations of the present invention comprising capsaicin have applications in cosmetics through their mild irritant effect. In particular, the topical application of capsaicin to the skin results in vasodilation. When applied to the lips, capsaicin causes the lips to swell and redden, enhancing their appearance.

Formulations of the present invention comprising salicylic acid or a salt or ester thereof and tocopherol can be used to improve the appearance of uneven skin tone by reducing the appearance of hyperpigmentation such as melanic spots and liver spots. The salicylic acid or a salt or ester thereof and the tocopherol may be present in the formulation, both associated with the colloidal particles and not associated with the colloidal particles. The salicylic acid or salt or ester thereof and the tocopherol which is associated with the colloidal particles may be tethered to the colloidal particles. The tethered salicylic acid may be in the form of myristyl salicylate or tridecyl salicylate, in which the myristyl aliphatic chain or tridecyl aliphatic chain is embedded in the colloidal particles. The tethered tocopherol may be in the form of tocopheryl linoleate, in which the linoleate chain is embedded in the colloidal particles.

Formulations of the present invention may comprise colloidal dispersions comprising colloidal particles comprising myristyl salicylate or tridecyl salicylate, wherein the myristyl salicylate or tridecyl salicylate is tethered to a component of the colloidal particle.

Formulations of the present invention may comprise colloidal dispersions comprising colloidal particles comprising tocopheryl linoleate, wherein the tocopheryl linoleate is tethered to a component of the colloidal particle.

These formulations may not comprise any other form of salicylic acid or tocopherol. These formulations may comprise one or more further colloidal dispersions comprising colloidal particles comprising a different AOI, such as vitamin C. Alternatively, the colloidal particles comprising myristyl salicylate, tridecyl salicylate or tocopheryl linoleate may also comprise vitamin C. The formulations may also comprise tocopherol that is not associated with the colloidal particles (i.e. it may be present in the continuous phase of the formulations). The formulations may also comprise tocopherol that is present in the lumen of the vesicles.

Formulations of the present invention may comprise colloidal dispersions comprising drug-free colloidal particles and myristyl salicylate or tridecyl salicylate, wherein the myristyl salicylate or tridecyl salicylate is not associated with the colloidal dispersions. In these formulations, myristyl salicylate or tridecyl salicylate may form micelles in the media, which may be a separate phase to the colloidal particles.

Such a formulation has a dual effect. The tethered salicylic acid or salt or ester thereof will penetrate more deeply into the stratum basale of the skin (where the melanocytes are located) where it will exert an anti-tyrosinase action to down-regulate melanin production. The salicylic acid or salt or ester thereof which is not associated with the deformable colloidal particles will penetrate less deeply into the skin, remaining in the outer layers of the epidermis where it will exert a mild exfoliating effect, which will increase the speed of turnover of new, now de-melanised, skin. The skin-lightening effects of the tethered salicylic acid or salt or ester thereof will therefore be seen by the patient more quickly than in the absence of untethered salicylic acid or a salt or ester thereof.

A product containing a salicylate compound may find use as a topical counter-irritant.

Formulations of the present invention comprising caffeine can improve the appearance of the skin, particularly periorbital skin. As discussed above, they do so by acting as a powerful antioxidant and anti-inflammatory. In particular, caffeine has vasoconstrictor properties and is thought to shrink the blood vessels that cause under-eye dark circles. As demonstrated in Example 6, the formulations of the present invention can reduce the appearance of puffiness and swelling around the eyes and below the lower eyelid (eye bags), under-eye dark circles and fine lines and deep wrinkles, including crow's feet, around the eye area. The formulations can also result in periorbital skin which looks and feels less thin, looks and feels more firm, feels more elastic, looks smoother and which is more hydrated. The formulations can also lift sagged skin, resulting in a reduction in skin droopiness and sagging. The formulations can make the skin tone look more even, make the eye contour look and feel tighter and look more toned and lifted and make the eyes look rested and less tired. They may also be used to prevent sun damage to the skin.

Without wishing to be bound by any particular theory, it is also believed that the colloidal particles themselves have a beneficial effect on the skin, particularly periorbital skin, as they have a 'nano-stenting' effect that helps this sensitive tissue to drain excess fluid. By 'nano-stenting' effect it is meant that the colloidal particles act as a form of proppant or scaffold and locate in the extracellular spaces of tissues where they facilitate the draining of extracellular/interstitial fluids into the lymph. It is believed that the colloidal particles physically push the interstitial fluid and by-products contained therein. This assists in the removal of undesirable by-products to the lymph and through the lymph, preventing toxic build-up, and also assists in the normal drainage of the lymph where this has been compromised.

Where these formulations comprise palmitoyl ascorbic acid and tocopherol, their antioxidant effect is enhanced. Where these formulations comprise palmitoyl tripeptide-1 and palmitoyl tetrapeptide-7 tethered to the deformable colloidal particles, collagen synthesis is also stimulated. The appearance of the periorbital skin is therefore further improved.

Compositions of the present invention comprising tocopherol in the continuous phase and salicylic acid or a salt or ester thereof tethered to the deformable colloidal particles can improve the appearance of the skin. In particular, the compositions can be used to improve the appearance of skin tone, for example by treating and/or preventing uneven skin tone.

As discussed above, the tocopherol acts as a powerful antioxidant and anti-inflammatory, protecting the cells that make collagen and elastin and acting as an effective moisturiser. As discussed above, salicylic acid or a salt or ester thereof can also improve the appearance of uneven skin tone by reducing the appearance of hyperpigmentation such as melanic spots and liver spots. As demonstrated in Example 7, the compositions of the present invention can reduce the appearance of fine lines and wrinkles and the amount and size of hyperpigmentation/pigmented skin blemishes. The compositions can result in skin which looks significantly smoother and feels significantly more healthy and elastic. The compositions can help the complexion look younger and significantly healthier, be visibly improved and more radiant. The compositions can also make skin tone look significantly more even and significantly lighten hyperpigmentation/pigmented skin blemishes and periorbital dark circles.

Where these compositions comprise palmityol ascorbic acid and tocopheryl linoleate tethered to a second form of deformable colloidal particle, their antioxidant effect is enhanced.

The compositions of the present invention may be useful for improving the appearance of the skin, not only because the deformable colloidal particles increase the speed, depth and effectiveness of absorption of the tocopherol and salicylic acid or salt or ester thereof into the skin of the patient, but also because the deformable colloidal particles themselves can improve the appearance of the skin. As discussed above, the deformable colloidal particles can enhance drainage of the interstitial fluid to the lymph nodes, preventing toxic build-up of by-products in the skin.

The invention also provides the use of a formulation according to the first aspect of the present invention for the manufacture of a medicament for the treatment or prevention of a disease or for cosmetic treatment of a subject or for skin care.

For all of the formulations in accordance with the invention, it is the particular combination (or blend or mixture) of the specific colloidal dispersions and, when present, the AOI that allows the formulation to have multiple effects.

Formulations in accordance with the present invention may be known as a "dermocosmetics" as they are able to address the symptoms of dermatological conditions as well as mask them. Without wishing to be bound by any theory, it is the flexible microscopic spheres (i.e. the colloidal particles) of the formulation of the present invention, containing naturally occurring plant lipids, that enables the formulation to supplement the skin, support the natural recovery process, ensure deep dermal hydration with pure water and prevent water loss by forming an intimate evaporation barrier within the skin structure. When present, the AOI in the continuous phase of the formulation (i.e. that is not associated with a deformable colloidal particle) is pushed through the skin by the deformable colloidal particles in the formulation and any AOI that is associated with a deformable colloidal particle is pulled through the skin by the deformable colloidal particles to which it is attached. These actions ensure that the AOIs penetrate the skin and are delivered in effective amounts to the required areas.

The formulations of the present invention may comprise one or more vitamins. These vitamins may be tethered to the colloidal particles, present in the continuous phase or as a separate phase.

Such vitamins may include vitamin C (ascorbic acid) or esters thereof, for example palmitoyl ascorbic acid or palmitoyl ascorbate. The formulations may comprise 0.01 to 1.0% by weight vitamin C (ascorbic acid) or esters thereof, more preferably 0.05 to 0.5% by weight, most preferably approximately 0.1% by weight. Preferably, the vitamin C or esters thereof are tethered to the deformable colloidal particles.

Another preferred vitamin for inclusion in formulations of the present invention is vitamin E (tocopherol) or esters thereof, for example tocopheryl linoleate. The formulations may comprise about 0.01 to 1.0% by weight vitamin E (tocopherol) or esters thereof, more preferably about 0.05 to 0.5% by weight, most preferably about 0.1% to 0.2% by weight. Preferably, the tocopheryl linoleate is tethered to the deformable colloidal particles. Preferably, tocopherol is present as a separate phase.

Vitamins such as vitamin C and vitamin E and esters thereof provide the vesicles with an anti-oxidant protective effect.

Formulations of the present invention may comprise both vitamin C and vitamin E or esters thereof. Preferably, the vitamin C or esters thereof and the vitamin E or esters thereof, where present, are tethered to separate vesicles.

The tethering of such vitamins is particularly preferred when the AOI which is not associated with the deformable colloidal particles comprises salicylic acid or a salt or ester thereof, glucosamine or a salt thereof, an amide of glucosamine or a salt thereof, chondroitin or a salt thereof, caffeine or tocopherol.

Preferably, when the formulation comprises salicylic acid or a salt or ester thereof, palmitoyl ascorbic acid and tocopheryl linoleate are tethered to the deformable colloidal particles and tocopherol is present as a separate phase. In a particularly preferred embodiment, the formulation comprises two forms of deformable colloidal particle; the first comprising salicylic acid or a salt or ester thereof tethered thereto and the second comprising palmitoyl ascorbic acid and tocopheryl linoleate tethered thereto.

Preferably, when the formulation comprises glucosamine or a salt thereof, an amide of glucosamine or a salt thereof and/or chondroitin or a salt thereof, the formulation additionally comprises palmitoyl ascorbate and tocopheryl linoleate tethered to the deformable colloidal particles. The inclusion of such vitamins into these formulations enhances the efficacy of these formulations in maintaining and prolonging joint health and treating or preventing joint diseases or the pain associated with poor joint health.

Preferably, when the formulation comprises caffeine, palmitoyl ascorbic acid and tocopheryl linoleate are tethered to the deformable colloidal particles and tocopherol is present as a separate phase.

Where the formulations contain tocopherol, a small amount of tocopherol may be present within the lumen of the deformable colloidal particles, with the majority of tocopherol present within the continuous phase of the formulations.

Formulations of the present invention may additionally comprise tripeptide-1, preferably palmitoyl tripeptide-1, and/or tetrapeptide-7, preferably palmitoyl tetrapeptide-7, tethered to deformable colloidal particles.

The formulations may comprise about 0.0001 to 0.1% by weight of tripeptide-1, preferably palmitoyl tripeptide-1, more preferably about 0.001 to 0.01% by weight, more preferably about 0.002 to 0.008% by weight, most preferably approximately 0.006% by weight.

The formulations may comprise about 0.0001 to 0.01% by weight of tetrapeptide-7, preferably palmitoyl tetrapeptide-7, more preferably about 0.001 to 0.01% by weight, more preferably about 0.002 to 0.008% by weight, most preferably approximately 0.006% by weight.

The tethering of tripeptide-1 and tetrapeptide-7, in particular palmitoyl tripeptide-1 and/or palmitoyl tetrapeptide-7, to deformable colloidal particles is particularly preferred when an AOI which is not associated with the deformable colloidal particles is caffeine. In a particularly preferred embodiment, the formulation comprises three types of deformable colloidal particle; the first comprising palmitoyl ascorbic acid tethered thereto and tocopherol, the second comprising palmitoyl tripeptide-1 tethered thereto and the third comprising palmitoyl tetrapeptide-7 tethered thereto. The tocopherol may be present in the lumen of the colloidal particles, or outside the colloidal particles in the continuous phase of the formulation (i.e. not associated with the colloidal particles).

The inclusion of palmitoyl tripeptide-1 and palmitoyl tetrapeptide-7 into these formulations enhances their efficacy by stimulating collagen synthesis, firming the skin and reducing the appearance of wrinkles.

The formulation of the invention may be used to treat any animal. The animal to be treated may be a human, a companion animal (e.g. a cat, a dog or a horse) or an agricultural animal. When the animal to be treated is a human, the invention may be useful for the treatment of patients of any age, for example, patients aged from 15 years old to 100 years old. The human patient may be male or female.

As discussed above, the formulation may be topically applied, enabling the formulation of the invention to be particularly useful as a cosmetic or for treating skin conditions.

The invention comprises a formulation for use as herein described, wherein any amount of the formulation may be topically applied as often as desired, provided this is within any regulatory restrictions imposed on the use of the AOI in the formulations. For example, the formulation may be applied once, twice, three times or more per day. Alternatively the formulation may be administered on alternate days, two or three times per week, once per week or less frequently as needed. The formulation may be administered over a period of one or more weeks, for example, for at least one week, two weeks, three weeks, four weeks, five weeks, six weeks, seven weeks, eight weeks, nine weeks, ten weeks, eleven weeks, or twelve weeks, sixteen weeks, twenty four weeks, four months, six months, eight months, ten months, one year, two or more years, or indefinitely as needed.

Any amount of the formulation sufficient to treat any of the conditions disclosed herein may be administered to the patient. For example, a 0.1 to 10 gram dose of the formulation of the invention may be administered to the patient. The dose may be 1 to 10 grams, or 1 to 5 grams or about 1 gram, 2 grams, 3 grams, 4 grams, 5 grams, 6 grams, 7 grams, 8 grams, 9 grams or 10 grams. The dose may be measured as the total weight of the formulation. The dose can be measured as the total weight of the phospholipid(s) and/or surfactant(s) in the formulation. The dose may be administered once or twice daily or as often as needed. The dose may be administered once, twice, three, four, five, six, or seven times per week, or as often as needed, in accordance with the invention. The dose may be administered every day, every other day, or two to three times a week, or as often as needed, in accordance with the invention. Preferably, the formulation may be applied twice daily. A suitable amount of the formulation (i.e. any amount of the formulation sufficient to treat a condition as herein described) may be spread over the problem area of the skin. The formulation may be left to dry for up to 15 minutes.

Where the formulation is to be used as a cosmetic, any amount of the formulation may be topically applied to the skin as often as necessary to achieve the desired effect.

The lipid in the deformable colloidal particles of the formulation may be phosphatidylcholine.

The surfactant in the deformable colloidal particles of the formulation may be polysorbate 80.

The formulation may include one or more buffers, chelators, humectants, lubricants, antioxidants, preservatives, microbicides, antimicrobials, emollients, co-solvents or thickeners. The thickeners may be Carbopol® (polyacrylamide) or methylcellulose.

In a fourth aspect, the present invention provides a method of cosmetically improving the appearance of a subject comprising administering the formulation of the present invention to the subject. Preferably, the formulation is topically applied to the skin of the subject, who is preferably human.

Where the AOI comprises chlorhexidine or a salt thereof, the method preferably comprises improving the appearance of the skin of the subject, for example by treating or preventing acne.

Where the AOI comprises capsaicin, the method preferably comprises improving the cosmetic appearance of the lips of the subject, for example by plumping the lips.

Where the AOI agent comprises salicylic acid or a salt or ester thereof and/or tocopherol or a derivative thereof, the method preferably comprises improving the appearance of the skin, for example by treating or preventing uneven skin tone, preferably by reducing the appearance of hyperpigmentation.

Where the AOI comprises caffeine, the method preferably comprises improving the appearance of the skin of the subject, preferably the periorbital skin.

Where the AOI comprises glucosamine or salt thereof, an amide of glucosamine or a salt thereof and/or chondroitin or a salt thereof, the method preferably comprises improving the appearance of the skin, for example by enhancing collagen production to reduce the appearance of fine lines and wrinkles.

Also described herein is a method of making the composition of the first aspect of the invention. Preferably, the method comprises a primary manufacturing step in which a colloidal dispersion is made from an "organic phase" containing alcohol-soluble components and an "aqueous phase" consisting of water-soluble components. During a secondary manufacturing step, this initial dispersion is mixed with a thickener to form a gel with the desired consistency. The AOI is preferably added during this secondary manufacturing step. More than one kind of colloidal dispersion may be introduced such that the final composition comprises more than one kind of colloidal particle.

In a sixth aspect, the present invention provides a formulation comprising a first colloidal dispersion and a second colloidal dispersion, wherein each colloidal dispersion comprises deformable colloidal particles comprising a surfactant and optionally a phospholipid and one or more of the deformable colloidal particles of the first colloidal dispersion comprise a first agent of interest (AOI) wherein the modified component is a lipid or surfactant covalently bonded to an AOI, such that the entire AOI is outside the particle membrane, and wherein the first AOI is zinc for use in treating a disease, disorder or condition in a subject by topically applying the formulation.

Where the AOI comprises chlorhexidine or a salt thereof, the disease, disorder or condition is preferably acne or dermatitis.

Where the AOI comprises capsaicin, the disease, disorder or condition is preferably selected from the group comprising pain, including muscle pain, and itching.

Where the AOI agent comprises salicylic acid or a salt or ester thereof and/or tocopherol or a derivative thereof, the disease, disorder or condition is preferably pain or discomfort.

Where the AOI comprises glucosamine or a salt thereof, an amide of glucosamine or a salt thereof, and/or chondroitin or a salt thereof, the disease, disorder or condition is preferably a joint disease (arthropathy), in particular a degenerative joint disease such as arthritis or osteoarthritis, or the pain associated with poor joint health.

Also described herein is a method of maintaining and prolonging joint health, comprising topically applying a composition according to the first aspect of the invention to the skin of a patient in need thereof, wherein the AOI comprises glucosamine or a salt thereof, an amide of glucosamine or a salt thereof, and/or chondroitin or a salt thereof.

The present invention can be used to administer the AOI, such as chlorhexidine or a salt thereof, capsaicin, salicylic acid or a salt or ester thereof, glucosamine or salt thereof, an amide of glucosamine or a salt thereof and/or chondroitin or a salt thereof, caffeine or tocopherol, to or through the skin of an animal. Preferably, the method comprises topically applying the composition to the skin of the patient in an amount sufficient to penetrate the skin to deliver the AOI.

The present invention can also be used to administer an AOI, such as glucosamine or a salt thereof, an amide of glucosamine or a salt thereof and/or chondroitin or a salt thereof, to a joint of an animal, for example a shoulder, elbow, wrist, knuckle, knee or ankle joint.

Preferably, the method comprises topically applying the composition to the skin of the patient in an amount sufficient to penetrate the skin to deliver the AOI to the underlying joint. Any animal can be included, including humans, dogs, cats, horses, food production animals and pets.

In a seventh aspect, the present invention provides a kit comprising one or more compartments, wherein at least one compartment contains a formulation comprising a first colloidal dispersion and a second colloidal dispersion, wherein each colloidal dispersion comprises deformable colloidal particles comprising a surfactant and optionally a phospholipid and one or more of the deformable colloidal particles of the first colloidal dispersion comprise a first agent of interest (AOI).

Preferably, the formulation according to the first aspect of the invention is contained within a single compartment of said kit.

The kit may be formatted such that it is marked to indicate quantity of the formulation remaining or dispensed.

The kit may be in the form of a tube, sachet or pot. The kit may also comprise a dispensing means, preferably selected from group consisting of a pump, nozzle, measuring cup or spatula.

The instructions for administration of the formulation according to the first aspect of the invention preferably comprise instructions for the administration of the formulation in accordance with any of the second, third, fourth, fifth or sixth aspects of the present invention.

Preferably, the instructions comprise instructions for the topical application of the formulation.

The kit may be for use in the treatment of disease.

Where the AOI comprises chlorhexidine or a salt thereof, the instructions for administration thereof preferably comprise instructions for administration thereof to a patient or subject in need thereof for the treatment or prevention of a disease or disorder associated with the skin of a patient, preferably acne or dermatitis. The instructions may alternatively direct the subject to administer the composition to the skin for the purpose of improving its appearance.

Where the AOI comprises capsaicin, the instructions for administration thereof preferably comprise instructions for administration thereof to a patient or subject in need thereof for the treatment or prevention of pain, including muscle pain, and/or itching. The instructions may also direct the subject to administer the composition prior to exercise. The instructions may alternatively direct the subject to administer the composition to the lips for the purpose of providing a cosmetic "plumped" effect.

Where the AOI agent comprises salicylic acid or a salt or ester thereof and/or tocopherol or a derivative thereof, the instructions for administration thereof preferably comprise instructions for administration thereof to a patient or subject in need thereof for the treatment or prevention of pain or discomfort. The instructions may alternatively direct the subject to administer the composition to the skin for the purpose of improving the appearance of uneven skin tone, including hyperpigmentation.

Where the AOI comprises glucosamine or salt thereof, an amide of glucosamine or a salt thereof, and/or chondroitin or a salt thereof, the instructions for administration thereof preferably comprise instructions for administration thereof to a patient or subject in need thereof for maintaining and prolonging joint health and/or for treating or preventing joint diseases (arthropathy), in particular degenerative joint diseases such as arthritis and osteoarthritis, or the pain associated with poor joint health. The instructions may alternatively direct the subject to administer the composition to the skin for the purpose of improving the appearance of the skin, for example by reducing the appearance of fine lines and wrinkles and improving the appearance skin tone, barrier function and hyperpigmentation.

Where the AOI comprises caffeine, the instructions for administration thereof preferably comprise instructions for administration thereof to a patient or subject in need thereof for improving the appearance of the skin, for example the periorbital skin. The formulation for improving the appearance of the skin comprising caffeine may also comprise tocopherol or a derivative thereof.

Where the AOI comprises tocopherol in the continuous phase and salicylic acid or a salt or ester thereof tethered to the deformable colloidal particles, the instructions for administration thereof preferably comprise instructions for administration thereof to a patient or subject in need thereof for improving the appearance of the skin, for example treating and/or preventing uneven skin tone.

An eighth aspect of the present invention comprises a transdermal drug release device comprising a support layer and a layer comprising the composition according to the first aspect of the present invention.

The transdermal drug release device may comprise a strip, plaster, bandage or patch.

The support layer may be made of any suitable material including fabric and silicon.

The transdermal drug release device may be applied to the skin of a patient such that the layer of the composition of the first aspect of the invention is in contact with the skin of the patient.

The support layer may remain on the skin for a set period of time, for example 1 hour to 3 days. Alternatively, the support layer may be removed and the composition layer may remain in contact with the skin.

All preferred features of the first aspect of the invention also apply to the second, third, fourth, fifth, sixth, seventh and eighth aspects of the invention.

Generally, the nomenclature used herein and the laboratory procedures in organic chemistry, medicinal chemistry, and pharmacology described herein are those well-known and commonly employed in the art. Unless defined otherwise, all technical and scientific terms used herein generally have the same meaning as commonly understood by one of ordinary skill in the art to which this disclosure belongs.

As used herein, "skin" includes the general skin of the body and any other external integument, such as the epithelium of the ear, nose, throat and eye, including the sclera of the eye, and other mucosal membranes, such as the vagina and anus/rectum.

As used herein, a "sufficient amount", "amount effective to", "amount sufficient to" or "amount of the formulation sufficient to" achieve a particular result refers to an amount of the formulation of the invention is effective to produce a desired effect, which is optionally a therapeutic effect (i.e., by administration of a therapeutically effective amount). Alternatively stated, a "therapeutically effective" amount is an amount that provides some alleviation, mitigation, and/or decrease in at least one clinical symptom. Clinical symptoms associated with the disorder that can be treated by the methods of the invention are well-known to those skilled in the art. Further, those skilled in the art will appreciate that the therapeutic effects need not be complete or curative, as long as some benefit is provided to the subject. For example, a "sufficient amount" or "an amount sufficient to" can be an amount that is effective to treat the symptoms of any disease or condition as herein described.

As used herein, the terms "treat", "treating" or "treatment" of mean that the severity of a subject's condition is reduced or at least partially improved or ameliorated and/or that some alleviation, mitigation or decrease in at least one clinical symptom is achieved and/or there is an inhibition or delay in the progression of the condition and/or delay in the progression of the onset of disease or illness. The terms "treat", "treating" or "treatment of' also means managing the disease state.

As used herein, the term "pharmaceutically acceptable" when used in reference to the formulation of the invention denotes that a formulation does not result in an unacceptable level of irritation in the subject to whom the formulation is administered. Preferably such level will be sufficiently low to provide a formulation suitable for approval by regulatory authorities.

As used herein with respect to numerical values, the term "about" or "approximately" means a range surrounding a particular numeral value which includes that which would be expected to result from normal experimental error in making a measurement. For example, in certain embodiments, the term "about" when used in connection with a particular numerical value means +-20%, unless specifically stated to be +-1%, +-2%, +-3%, +-4%, +-5%, +-10%. +-15%, or +-20% of the numerical value.

In accordance with this disclosure, the term "comprising" is inclusive or open- ended and does not exclude additional, un-recited elements or method steps; the term "consisting of" excludes any element, step, or ingredient not specified; and the term "consisting essentially of" excludes any element, step, or ingredient that materially changes a basic characteristic of the invention.

The colloidal particles of the colloidal dispersions of the formulation of the invention may comprise a mixture of more than one non-ionic surfactant. The colloidal particles of the colloidal dispersions of the formulation of the invention may comprise a mixture of more than one phospholipid. The colloidal particles of the colloidal dispersions of the formulation of the invention may comprise a mixture of more than one non-ionic surfactant and more than one phospholipid. The colloidal particles of the colloidal dispersions of the formulation of the invention may comprise at least one non-ionic surfactant, at least one phospholipid, a pharmaceutically acceptable carrier, and optionally buffers, antioxidants, preservatives, microbicides, antimicrobials, emollients, co- solvents, and/or thickeners. The colloidal particles of the colloidal dispersions of the formulation of the invention may consist essentially of a non-ionic surfactant and a phospholipid, a pharmaceutically acceptable carrier, and optionally buffers, antioxidants, preservatives, microbicides, antimicrobials, emollients, co-solvents, and/or thickeners. The colloidal particles of the colloidal dispersions of the formulation of the invention may consist of at least one non-ionic surfactant, at least one phospholipid, a pharmaceutically acceptable carrier, and one or more of the following: buffers, antioxidants, preservatives, microbicides, antimicrobials, emollients, co-solvents, and thickeners.

Any lipid known in the art that will form a phospholipid bilayer may be used for making the formulation of the invention.

Table 1 lists preferred phospholipids in accordance with the invention.

**Table 1:**

| |
|---|
| Laur(o)yl |
| Palmit(o)yl |
| Stear(o)yl |
| Bechen(o)yl |
| Eruca(o)yl |
| Arachin(o)yl |
| Gadolen(o)yl |
| Arachindon(o)yl |
| Ole(o)yl |
| Linol(o)yl |
| Linole(n/o)yl |
| Palmitole(o)yl |
| Myrist(o)yl |
| Capr(o)yl |

The preferred lipids in the context of this disclosure are uncharged and form stable, well hydrated bilayers; phosphatidylcholines, phosphatidylethanolamine, and sphingomyelins are the most prominent representatives of such lipids. Any of those can have chains as listed in Table 1; the ones forming fluid phase bilayers, in which phospholipid chains are in disordered state, being preferred.

A preferred phospholipid is, for example, a natural phosphatidylcholine, which was formerly known as lecithin. It can be obtained from egg (rich in palmitic, C16:0, and oleic, C18:1, but also comprising stearic, C18:0, palmitoleic, C16:1, linolenic, C18:2, and arachidonic, C20:4(M, radicals), soybean (rich in unsaturated C18 chains, but also containing some palmitic radicals, amongst a few others), coconut (rich in saturated chains), olives (rich in monounsaturated chains), saffron (safflower) and sunflowers (rich in n-6 linoleic acid), linseed (rich in n-3 linolenic acid), from whale fat (rich in monounsaturated n-3 chains), from primrose or primula (rich in n-3 chains). Preferred, natural phosphatidyl ethanolamines (used to be called cephalins) frequently originate from egg or soybeans. Preferred sphingomyelins of biological origin are typically prepared from eggs or brain tissue.

Preferred phosphatidylserines also typically originate from brain material whereas phosphatidylglycerol is preferentially extracted from bacteria, such as *E. coli,* or else prepared by way of transphosphatidylation, using phospholipase D, starting with a natural phosphatidylcholine. The preferably used phosphatidylinositols are isolated from commercial soybean phospholipids or bovine liver extracts. The preferred phosphatidic acid is either extracted from any of the mentioned sources or prepared using phospholipase D from a suitable phosphatidylcholine.

Synthetic phosphatidylcholines, synthetic phosphatidylethanolamines, synthetic phosphatidic acids, synthetic phosphatidylserines, synthetic phosphatidyl(poly)alcohols, such as phosphatidylinositol, or synthetic phosphatidylglycerol may also be used in accordance with the invention.

Suitable lipids furthermore are a lysophosphatidyl choline analog, such as 1-lauroyl-1,3-dihydroxypropane-3-phosphoryl choline, a monoglyceride, such as monoolein or monomyristin, a cerebroside, ceramide polyhexoside, sulfatide, sphingoplasmalogen, a ganglioside or a glyceride, which does not contain a free or esterified phosphoryl or phosphono or phosphino group in the 3 position. An example of such a glyceride is diacylglyceride or 1 -alkenyl- l-hydroxy-2-acyl glyceride with any acyl or alkenyl groups, wherein the 3 -hydroxy group is etherified by one of the carbohydrate groups named, for example, by a galactosyl group such as a monogalactosyl glycerin.

Lipids with desirable head or chain group properties can also be formed by biochemical means, for example, by means of phospholipases (such as phospholipase Al, A2, B, C and, in particular, D), desaturases, elongases, acyl transferases, etc., from natural or synthetic precursors.

Furthermore, a suitable phospholipid is any phospholipid, which is contained in biological membranes and can be extracted with the help of apolar organic solvents, such as chloroform. Aside from the lipids already mentioned, such lipids also include, for example, steroids, such as estradiol, or sterols, such as cholesterol, beta-sitosterol, desmosterol, 7-keto-cholesterol or beta-cholestanol, fat-soluble vitamins, such as retinoids, vitamins, such as vitamin Al or A2, vitamin E, vitamin K, such as vitamin Kl or K2 or vitamin Dl or D3, etc.

The less soluble amphiphilic components comprise or preferably comprise a synthetic phospholipid, such as myristoleoyl, palmitoleoyl, petroselinyl, petroselaidyl, oleoyl, elaidyl, cis- or trans-vaccenoyl, linolyl, linolenyl, linolaidyl, octadecatetraenoyl, gondoyl, eicosaenoyl, eicosadienoyl. eicosatrienoyl, arachidoyl, cis- or trans-docosaenoyl, docosadienoyl, docosatrienoyl, docosatetraenoyl, lauroyl, tridccanoyl. myristoyl, pentadccanoyl, palmitoyl, heptadecanoyl, stearoyl or nonadecanoyl, glycerophospholipid or corresponding derivatives with branched chains or a corresponding dialkyl or sphingosin derivative, glycolipid or other diacyl or dialkyl phospholipid.

The more soluble amphiphilic components(s) is/are frequently derived from the less soluble components listed above and, to increase the solubility, substituted and/or complexed and/or associated with a butanoyl, pentanoyl, hexanoyl, heptanoyl, octanoyl, nonanoyl, decanoyl or undecanoyl substituent or several, mutually independent, selected substituents or with a different material for improving the solubility.

A further suitable phospholipid is a diacyl- or dialkyl-glycerophosphoethanolamine azo polyethoxylene derivative, a didecanoylphosphatidyl choline or a diacylphosphoolligomaltobionamide.

The amount of phospholipid in the formulations of the invention may range from about 0% to about 12%, about 0.2% to about 10%, about 0.6% to about 8%, about 1.0% to about 7%, about 1.5% to about 6%, about 2% to about 4%, about 3% to about 3.5% or about 0.7%, about 1.4%, about 3.4% or about 3.6% by weight. Preferably, the phospholipid is a phosphatidylcholine.

The formulations of the invention may include a surfactant. The term "surfactant" has its usual meaning. A list of relevant surfactants and surfactant related definitions is provided in EP 0 475 160 Al (see, e.g., p. 6, 1. 5 to p.14. 1.17) and U.S. Pat. No. 6,165,500 (see, e g., col. 7, 1. 60 to col. 19, 1. 64), and in appropriate surfactant or pharmaceutical Handbooks, such as Handbook of Industrial Surfactants or US Pharmacopoeia, Pharm. Eu. The surfactants may be those described in Tables 1-18 of U.S. Patent Application Publication No. 2002/0012680 A1, published January 31, 2002. The following list therefore only offers a selection, which is by no means complete or exclusive, of several surfactant classes that are particularly common or useful in conjunction with present patent application. Preferred surfactants to be used in accordance with the disclosure include those with an HLB greater than 12. The list includes ionized long-chain fatty acids or long chain fatty alcohols, long chain fatty ammonium salts, such as alkyl- or alkenoyl-trimethyl-, -dimethyl- and - methylammonium salts, alkyl- or alkenoyl-sulphate salts, long fatty chain dimethyl- aminoxides, such as alkyl- or alkenoyl-dimethyl-aminoxides, long fatty chain, for example alkanoyl, dimethyl-aminoxides and especially dodecyl dimethyl-aminoxide, long fatty chain, for example alkyl-N-methylglucamide- s and alkanoyl-N-methylglucamides. such as MEGA-8, MEGA-9 and MEGA-IO, N-long fatty chain-N,N-dimethylglycines, for example N-alkyl- N,N-dimethylglycines, 3 -(long fatty chain-dimethylammonio)-alkane- sulphonates, for example 3-(acyidimethylammonio)-alkanesulphonates, long fatty chain derivatives of sulphosuccinate salts, such as bis(2-ethylalkyl) sulphosuccinate salts, long fatty chain- sulphobetaines, for example acyl-sulphobetaines, long fatty chain betaines, such as EMPIGEN BB or ZWITTERGENT-3-16, -3-14, -3-12, -3-10, or-3-8, or polyethylcn-glycol- acylphenyl ethers, especially nonaethylen-glycol-octyl- phenyl ether, polyethylene-long fatty chain-ethers, especially polyethylene-acyl ethers, such as nonaethylen-decyl ether, nonaethylen-dodecyl ether or octaethylene-dodecyl ether, polyethyleneglycol-isoacyl ethers, such as octaethyleneglycol-isotridecyl ether, polyethyleneglycol-sorbitane-long fatty chain esters, for example polyethyleneglycol-sorbitane-acyl esters and especially polyoxyethylene-monolaurate (e.g. polysorbate 20 or Tween 20), polyoxyethylene-sorbitan-monooleate (e.g. polysorbate 80 or Tween 80), polyoxyethylene-sorbitan-monolauroleylate, polyoxyethylene - sorbitan-monopetroselinate, polyoxyethylene -sorbitan- monoelaidate, polyoxyethylene - sorbitan-myristoleylate, polyoxyethylene -sorbitan-palmitoleinylate, polyoxyethylene-sorbitan-p- etroselinylate, polyhydroxyethylene-long fatty chain ethers, for example polyhydroxyethylene-acyl ethers, such as polyhydroxyethylene-lauryl ethers, polyhydroxyethylene-myristoyl ethers, polyhydroxyethylene-cetylst- earyl, polyhyd roxyethylene-palmityl ethers, polyhydroxyethylene-oleoyl ethers, polyhydroxyethylene-palmitoleoyl ethers, polyhydroxyethylene-lino- leyl, polyhydroxyethylen-4, or 6, or 8, or 10, or 12-lauryl, miristoyl, palmitoyl, palmitoleyl, oleoyl or linoeyl ethers (Brij series), or in the corresponding esters, polyhydroxyethylen-laurate, -myristate, -palmitate, -stearate or -oleate, especially polyhydroxyethylen-8-stearate (Myrj 45) and polyhydroxyethylen-8-oleate, polyethoxylated castor oil 40 (Cremophor EL), sorbitane-mono long fatty chain, for example alkylate (Arlacel or Span series), especially as sorbitane-monolaurate (Arlacel 20, Span 20), long fatty chain, for example acyl-N-methylglucamides, alkanoyl-N-methylglucamides, especially decanoyl-N-methylglucamide, dodecanoyl-N-methylglucamide, long fatty chain sulphates, for example alkyl-sulphates, alkyl sulphate salts, such as lauryl-sulphate (SDS), oleoyl-sulphate: long fatty chain thioglucosides, such as alkylthioglucosides and especially heptyl-, octyl- and nonyl-beta-D-thioglucopyranoside; long fatty chain derivatives of various carbohydrates, such as pentoses, hcxoses and disaccharidcs, especially alkyl-glucosides and maltosides, such as hexyl-, heptyl-, octyl-, nonyl- and decyl-beta-D-glucopyranoside or D- maltopyranosidc; further a salt, such as a fatty acid salt, especially oleate, elaidate, linoleate, laurate, or myristate, most often in sodium form, lysophospholipids, n-octadecylene-glycerophosphatidic acid, octadecylene- phosphorylglycerol, octadecylene-phosphorylserine, n-long fatty chain-glycero-phosphatidic acids, such as n-acyl-glycero-phosphatidic acids, especially lauryl glycero-phosphatidic acids, oleoyl-glycero-phosphatidic acid, n-long fatty chain-phosphoryl glycerol, such as n- acyl-phosphorylglycerol, especially lauryl-, myristoyl-, oleoyl- or palmitoeloyl- phosphorylglycerol, n-long fatty chain-phosphorylserine, such as n-acyl-phosphorylserine, especially lauryl-, myristoyl-, oleoyl- or palmitoeloyl-phosphorylserine, n-tetradecyl-glycero- phosphatidic acid, n-tetradecyl-phosphorylglycerol, n-tetradecyl-phosphorylserine, corresponding-, elaidoyl-, vaccenyl-lysophospholipids, corresponding short-chain phospholipids, as well as all surface active and thus membrane destabilising polypeptides. Surfactant chains are typically chosen to be in a fluid state or at least to be compatible with the maintenance of fluid-chain state in carrier aggregates.

The surfactant in the formulations of the invention is a non-ionic surfactant. The surfactant may be present in the formulation in about 0% to about 1%, about 0.04% to about 0.8%, about 0.06% to about 0.6%, about 0.08% to about 0.4%, about 0.1% to about 0.3%, about 0.15% to about 0.2%, about 0.1% to about 5%, about 0.2% to about 3%, about 0.3% to about 4%, about 0.5% to about 10%, about 1% to about 9%, about 1.5% to about 7%, about 2% to about 6% or about 3% to about 8% by weight. The non-ionic surfactant may be selected from the group consisting of: polyoxyethylene sorbitans (polysobate surfactants), polyhydroxyethylene stearates or polyhydroxyethylene laurylethers (Brij surfactants). The surfactant may be a polyoxyethylene-sorbitan- monooleate (e.g. polysorbate 80 or Tween 80) or Tween 20, 40 or 60. The polysorbate can have any chain with 12 to 20 carbon atoms. The polysorbate may be fluid in the formulations, which may contain one or more double bonds, branching, or cyclo-groups.

The formulations of the invention may comprise only one non-ionic surfactant. The formulations of the invention may comprise only one phospholipid. The formulations of the invention may comprise only one non-ionic surfactant and only one phospholipid. The formulations of the invention may comprise one or more than one non-ionic surfactant and/or one or more than one phospholipid, e.g., independently one, two, three, four, or more non-ionic surfactants and/or one, two, three, four or more phospholipids.

The formulations of the invention may have a range of lipid/phospholipid to surfactant ratios. The ratios may be expressed in terms of molar terms (mol lipid/mol surfactant or mol phospholipid/mol surfactant). They may also be expressed as % weight. The weight ratio of lipid or phospholipid to surfactant in the formulation may be from about 20:1 to about 5:1, from about 15:1 to about 10:1, from about 12:1 to about 9:1, from about 18:1 to about 14:1.

Specifically, the weight ratio of lipid or phospholipid to surfactant in the formulation may be about 8:1, about 9:1, about10:1, about 15:1 or about 16:1. There may be no lipid or phospholipid present i.e. a ratio of 0:1. There may be no surfactant present i.e. a ratio of 1:0. Preferably, all of the deformable colloidal particles in a formulation of the present invention have the same lipid or phospholipid to surfactant ratio.

The formulations of the invention may also have varying amounts of total amount of the following components: lipid or phospholipid and surfactant combined (TA). The TA amount may be stated in terms of weight percent of the total composition. The TA may be from about 1% to about 40%, about 5% to about 30%, about 7.5% to about 15%, about 6% to about 14%, about 8% to about 12%, about 5% to about 10%, about 10% to about 20% or about 20% to about 30%. The TA may be 6%, 7%, 8%, 9%, 10%, 12%, 14%, 15% or 20%.

The molar ratio of lipid or phospholipid to surfactant in the formulations of the invention may be from about 1:3 to about 30:0, from about 1:3 to about 30:1, from about 1:2 to about 30:1, from about 1:1 to about 30:0, from about 1:1 to about 30:1, from about 2:1 to about 20:0, from about 2:1 to about 20:1, from about 5:1 to about 30:1, from about 10:1 to about 30:1, from about 15:1 to about 30:1, or from about 20:1 to about 30:1. The molar ratio of lipid or phospholipid to surfactant in the formulation may be from about 1:2 to about 10:1. The molar ratio may be from about 1:1 to about 2:1, from about 2:1 to about 3:1, from about 3:1 to about 4:1, from about 4:1 to about 5:1 or from about 5:1 to about 10:1. The molar ratio may be from about 10.1 to about 30:1, from about 10:1 to about 20:1, from about 10:1 to about 25:1, and from about 20:1 to about 25:1. The lipid or phospholipid to surfactant ratio may be about 1.0:1.0, about 1.25:1.0, about 1.5:1.0, about 1.75:1.0, about 2.0:1.0, about 2.5:1.0, about 3.0:1.0 or about 4.0:1.0.

Selected ranges for total lipid or phospholipid amounts and lipid or phospholipid/surfactant ratios (mol/mol) for the formulations of the invention are described in Table 2 below:

**Table 2: Total Amount and Lipid/Phospholipid to Surfactant Ratios.**

| TA (%) | Lipid/Surfactant or Phospholipid/Surfactant (mol/mol) |
|---|---|
| 5 to 10 | 1.0 to 1.25 |
| 5 to 10 | 1.25 to 1.72 |
| 5 to 10 | 1.75 to 2.25 |
| 5 to 10 | 2.25 to 3.00 |
| 5 to 10 | 3.00 to 4.00 |
| 5 to 10 | 4.00 to 8.00 |
| 5 to 10 | 10.00 to 13.00 |
| 5 to 10 | 15.00 to 20.00 |
| 5 to 10 | 20.00 to 22.00 |
| 5 to 10 | 22.00 to 25.00 |
| 10 to 20 | 1.0 to 1.25 |
| 10 to 20 | 1.25 to 1.75 |
| 10 to 20 | 1.25 to 1.75 |
| 10 to 20 | 2.25 to 3.00 |
| 10 to 20 | 3.00 to 4.00 |
| 10 to 20 | 4.00 to 8.00 |
| 10 to 20 | 10.00 to 13.00 |
| 10 to 20 | 15.00 to 20.00 |
| 10 to 20 | 20.00 to 22.00 |
| 10 to 20 | 22.00 to 25.00 |

The formulations of the invention may optionally contain one or more of the following ingredients: co-solvents, chelators, buffers, antioxidants, preservatives, microbicides, emollients, humectants, lubricants and thickeners. Preferred amounts of optional components are described as follows in Table 3.

| **Table 3** | **Molar (M) or** | **Rel w%*** |
|---|---|---|
| Antioxidant: | | |
| *Primary:* | | |
| Butylated hydroxyanisole, BHA | | 0.1-8 |
| Butylated hydroxytoluene BHT | | 0.1-4 |
| Thymol | | 0.1-1 |
| Metabisulphite | 1-5mM | |
| Bisulsphite | 1-5mM | |
| Thiourea (MW=76.12) | 1-10mM | |
| Monothioglycerol (MW=108.16) | 1-20mM | |
| Propyl gallate (MW=212.2) | | 0.02-0.2 |
| Ascorbate (MW=175.3⁺ ion) | 1-10mM | |
| Palmityl-ascorbate | | 0.01-1 |
| Tocopherol-PEG | | 0.5-5 |
| *Secondary* (chelator) | | |
| EDTA (MW=292) | 1-10mM | |
| EGTA (MW=380.35) | 1-10mM | |
| Desferal (MW=656.79) | 0.1-5mM | |
| *Buffer* | | |
| Acetate | 30-150mM | |
| Phosphate | 10-50mM | |
| Triethanolamine | 30-150mM | |

| | | |
|---|---|---|
| *as a percentage of total phospholipid quantity | | |

The formulation of the invention is typically formulated and/or administered in an aqueous medium. The formulation may be formulated with or without co-solvents, such as lower alcohols. The formulation of the present invention may also comprise a polar liquid medium. The formulation may be in the form of a solution, suspension, emulsion, cream, lotion, ointment, gel, spray, film forming solution or lacquer. Preferably, the formulation of the present invention is in the form of a gel.

The formulation of the invention may include a buffer to adjust the pH of the aqueous solution to a range from pH 3.5 to pH 9, pH 4 to pH 7.5, or pH 6 to pH 7. Examples of buffers include, but are not limited to acetate buffers, lactate buffers, phosphate buffers, and propionate buffers. Preferably, the formulations comprise one or more buffers selected from the group consisting of disodium hydrogen orthophosphate dodecahydrate, disodium hydrogen orthophosphate anhydrous, sodium dihydrogen orthophosphate dehydrate, sodium dihydrogen orthophosphate dodecahydrate and phosphate buffer, for example phosphate (pH6.7) buffer.

A "microbicide" or "antimicrobial"' agent is commonly added to reduce the bacterial count in pharmaceutical formulations. Some examples of microbicides are short chain alcohols, including ethyl and isopropyl alcohol, chlorbutanol, benzyl alcohol, chlorbenzyl alcohol, dichlorbenzylalcohol, hexachlorophene; phenolic compounds, such as cresol, 4-chloro-m-cresol, p-chloro-m-xylenol. dichlorophene, hexachlorophene, povidon- iodine; parabenes. especially alkyl-parabenes, such as methyl-, ethyl-, propyl-, or butyl- paraben, benzyl paraben; acids, such as sorbic acid, benzoic acid and their salts; quaternary ammonium compounds, such as alkonium salts, e.g., a bromide, benzalkonium salts, such as a chloride or a bromide, cetrimonium salts, e.g., a bromide, phenoalkecinium salts, such as phenododecinium bromide, cetylpyridinium chloride and other salts; furthermore, mercurial compounds, such as phenylmercuric acetate, borate, or nitrate, thiomersal, chlorhexidine or its gluconate, or any antibiotically active compounds of biological origin, or any suitable mixture thereof.

Examples of "antioxidants" are butylhydroxyanisole or butylated hydroxyanisol (BHA), butylated hydroxytoluene (BHT) and di-tert-butylphenol (LY178002, LY256548, HWA-131, BF-389, CI-986, PD-127443, E-51 or 19, BI-L-239XX, etc.), tertiary butylhydroquinone (TBHQ), propyl gallate (PG), I-O-hexyl-2,3,5-trimethylhydroquinone (HTHQ); aromatic amines (diphenylamine, p-alkylthio-o-anisidine, ethylenediamine derivatives, carbazol, tetrahydroindenoindol); phenols and phenolic acids (guaiacol, hydroquinone, vanillin, gallic acids and their esters, protocatechuic acid, quinic acid, syringic acid, ellagic acid, salicylic acid, nordihydroguaiaretic acid (NDGA), eugenol); tocopherols (including tocopherols (alpha, beta, gamma, delta) and their derivatives, such as tocopheryl-acylate (e g. -acetate. - laurate. myristate, -palmitate, -oleate, -linoleate. etc., or an y other suitable tocopheryl-lipoate). tocopheryl-POE-succinate; trolox and corresponding amide and thiocarboxamide analogues; ascorbic acid and its salts, isoascorbate, (2 or 3 or 6)-o-alkylascorbic acids, ascorbyl esters (e.g., 6-o-lauroyl, myristoyl, palmitoyl-, oleoyl, or linoleoyl-L-ascorbic acid, etc.). Also useful are the preferentially oxidised compounds, such as sodium bisulphite, sodium metabisulphite, thiourea; chellating agents, such as EDTA, GDTA, desferral: miscellaneous endogenous defence systems, such as transferrin, lactoferrin, ferritin, cearuloplasmin, haptoglobion, heamopexin, albumin, glucose, ubiquinol-10); enzymatic antioxidants, such as superoxide dismutase and metal complexes with a similar activity, including catalase, glutathione peroxidase, and less complex molecules, such as beta-carotene, bilirubin, uric acid; flavonoids (flavones, flavonols, flavonones, flavanonals, chacones, anthocyanins). N-acetylcystein, mesna. glutathione, thiohistidine derivatives, triazoles; tannines, cinnamic acid, hydroxycinnamatic acids and their esters (coumaric acids and esters, caffeic acid and their esters, ferulic acid, (iso-) chlorogenic acid, sinapic acid); spice extracts (e.g., from clove, cinnamon, sage, rosemary, mace, oregano, allspice, nutmeg); carnosic acid, carnosol, carsolic acid; rosmarinic acid, rosmaridiphenol, gentisic acid, ferulic acid; oat flour extracts, such as avenanthramide 1 and 2; thioethers, dithioethers, sulphoxides, tetralkylthiuram disulphides; phytic acid, steroid derivatives (e.g., U74006F); tryptophan metabolites (e.g., 3-hydroxykynurenine, 3-hydroxyanthranilic acid), and organochalcogenides.

"Thickeners" are used to increase the viscosity of pharmaceutical formulations and may be selected from pharmaceutically acceptable hydrophilic polymers, such as partially etherified cellulose derivatives, comprising carboxym ethyl-, hydroxyethyl-, hydroxypropyl-, hydroxypropylmethyl- or methyl-cellulose; completely synthetic hydrophilic polymers such as polyacrylates (e.g. Carbopol®, specifically carbopol 974P), polymethacrylatcs, poly(hydroxyethyl)-, poly(hydroxypropyl)-, poly(hydroxypropylmethyl)methacrylate, polyacrylonitrile, methallyl- sulphonate, polyethylenes, polyoxiethylenes, polyethylene glycols, polyethylene glycol- lactide, polyethylene glycol-diacrylate, polyvinylpyrrolidone, polyvinyl alcohols, poly(propylmethacrylamide), poly(propylene fumarate-co-ethylene glycol), poloxamers, polyaspartamide (hydrazine cross-linked) hyaluronic acid, silicone; natural gums comprising alginates, carrageenan, guar-gum, gelatine, tragacanth, (amidated) pectin, xanthan, chitosan collagen, agarose; mixtures and further derivatives or co-polymers thereof and/or other pharmaceutically, or at least biologically, acceptable polymers. Preferably, the thickeners used in the formulations of the present invention may be selected from Carbopol® (Carbopol® 974P NF), Methylcellulose (Metolose SM 4000), Keltrol CG-T (a personal care grade xanthan gum), Xantural 11k (a pharmaceutical grade xanthan gum), Hydroxypropyl ethylcellulose, Genuvisco CG-131 (carrageenan gum), Kelcogel (gellan gum), Sucraclear HC-31 (cellulose gum, carrageenan, Ceratonia siliqua gum, sucrose), Sucrathix VX (microcrystalline cellulose, cellulose gum, xanthan gum), Lubrajel (glyceryl acrylate/acrylic acid) or Chitosan.

One particularly preferred formulation of the present invention comprises insoluble aggregates or micelles of chlorhexidine or a salt thereof, preferably chlorhexidine digluconate, together with empty deformable colloidal particles, preferably empty Sequessomes™, and deformable colloidal particles, preferably Tethersomes, to which zinc stearate has been tethered.

Another particularly preferred formulation of the present invention comprises liposomes comprising capsaicin, menthol and phospholipids together with deformable colloidal particles, preferably Transfersomes™, the membranes of which comprise menthol.

A particularly preferred formulation comprises myristyl salicylate or tridecyl salicylate dissolved in the continuous phase, and deformable colloidal particles, preferably Tethersomes, to which myristyl salicylate or tridecyl salicylate are tethered. Preferably, a derivative of tocopherol is also tethered to said deformable colloidal particles. This composition additionally comprises a second type of deformable colloidal particle, preferably Tethersomes, to which palmitoyl ascorbic acid and tocopheryl linoleate are tethered. Tocopherol may be present in the continuous phase of the formulation.

Another particularly preferred formulation comprises glucosamine hydrochloride and chondroitin sulphate dissolved in the continuous phase, and deformable colloidal particles, preferably Tethersomes, to which palmitoyl ascorbate and tocopheryl linoleate are preferably tethered.

A further particularly preferred formulation comprises N-acetyl glucosamine sulphate and chondroitin sulphate dissolved in the continuous phase, and deformable colloidal particles, preferably Tethersomes, to which palmitoyl ascorbic acid and tocopheryl linoleate are preferably tethered.

Another particularly preferred composition comprises caffeine dissolved in the continuous phase, and deformable colloidal particles, preferably Tethersomes, to which palmitoyl ascorbic acid and tocopheryl linoleate are preferably tethered. This composition additionally comprises two further types of deformable colloidal particles, a first to which palmitoyl tripeptide-1 is tethered and a second to which palmitoyl tetrapeptide-7 is tethered. Preferably, the composition comprises tocopherol as a separate phase. A small amount of tocopherol may partition into the lumen of the deformable colloidal particles. One embodiment of such a formulation is set out in Example 6.

A further particularly preferred formulation comprises deformable colloidal particles, which comprise tridecyl salicylate and/or alpha tocopherol. Tridecyl salicylate is preferably tethered to the colloidal particles. Alpha tocopherol may be present in the continuous phase of the formulations (i.e. not associated with the colloidal particles) and some may partition into the lumen of the colloidal particles. This composition additionally comprises a further type of deformable colloidal particle, which comprises palmitoyl ascorbic acid, tocopheryl linoleate and/or alpha tocopherol. Palmitoyl ascorbic acid and tocopheryl linoleate are preferably tethered to the colloidal particle. Alpha tocopherol may be present in the continuous phase of the formulations (i.e. not associated with the colloidal particles) and some may partition into the lumen of the colloidal particles. One embodiment of such a formulation is set out in Example 7.

The invention is described below with reference to the following examples and figures in which:

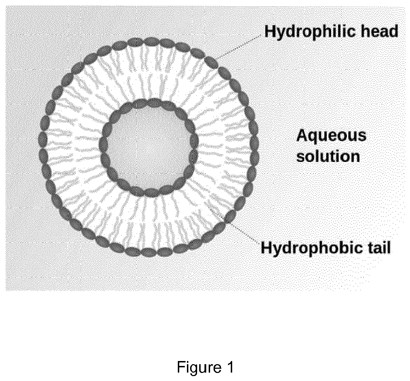

Figure 1 shows the structure of a colloidal particle (vesicle or Sequessome™/Transfersome™/Tethersome) of the invention. The spherical bilayer may comprise a surfactant or a lipid or a surfactant and a lipid. As can be seen from this diagram, the bilayer is two molecules wide;

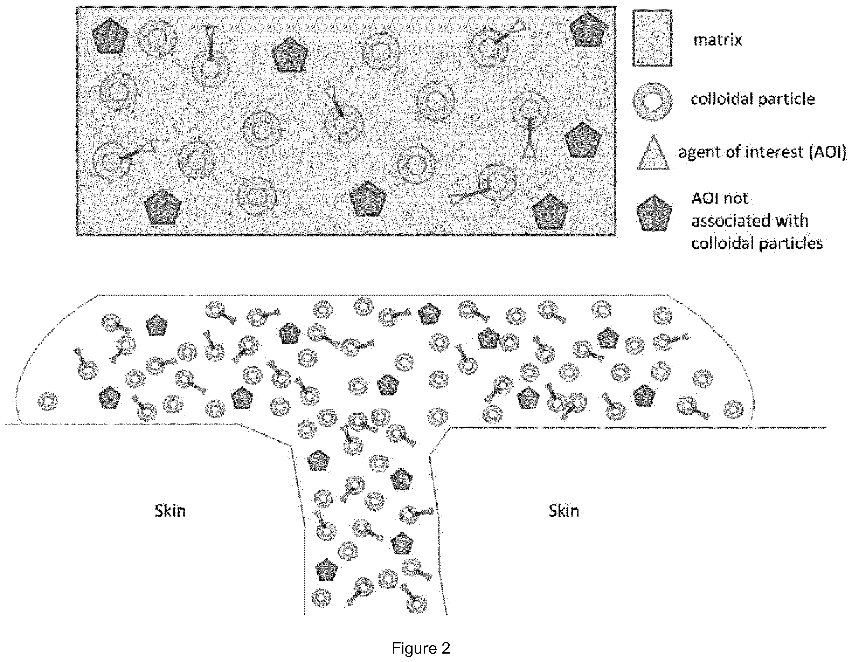

Figure 2 shows a typical formulation in accordance with the first aspect of the invention. This formulation comprises a colloidal dispersion (or Sequessome™/Transfersome™/Tethersome intermediate) comprising colloidal particles, a colloidal dispersion comprising colloidal particles comprising an agent of interest (AOI) (e.g. a peptide, ascorbic acid or zinc) and an AOI that is not associated with the colloidal particles (e.g. chlorhexidine or capsaicin);

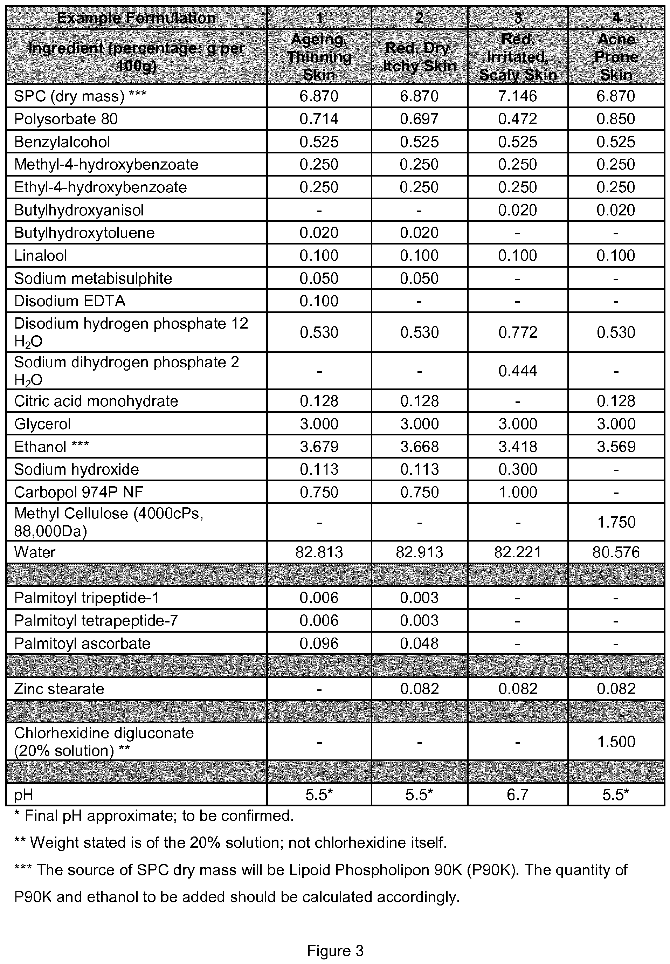

Figure 3 shows a table summarising Example Formulations 1 to 4;
Figure 4 shows the change in sebum levels compared to baseline level on day 0 (=100%);
Figure 5 shows the change in percentage of comedones compared to day 0 occurrence (100%);
Figure 6 shows the change in percentage incidence of pustules compared to day 0 occurrence (100%);
Figure 7 shows the reduction in measured wrinkle volume and the reduction in appearance of fine lines and wrinkles after treatment with a two types of formulation in accordance with the present invention;
Figure 8 shows a comparison of reduction in wrinkle volume (as a percentage of Week 0 baseline volume) after treatment with a formulation in accordance with the present invention, a UK leading own brand (in-market product) and a control;
Figure 9 shows a comparison of reduction in perceived appearance of wrinkles and fine lines (as a percentage of Week 0 baseline volume) after treatment with a formulation in accordance with the present invention, a UK leading own brand (in-market product) and a control; and
Figure 10 shows a comparison of reduction in appearance of peri-orbital dark circles (as a percentage of Week 0 baseline volume) after treatment with a formulation in accordance with the present invention, a UK leading own brand (in-market product) and a control.

### Examples

### Example 1: Method of Manufacture

The simplified generic manufacturing process of formulations in accordance with the present invention is as follows. Firstly, the colloidal dispersions comprising the deformable colloidal particles (for example, the Sequessome™ intermediate, Transfersome™ intermediate or Tethersome intermediate) are made. These colloidal dispersions are each formed from an 'organic phase' containing alcohol-soluble components and an 'aqueous phase' consisting of water-soluble components. Secondly, the gel phase (a thickener) is formed within which the colloidal dispersion(s) are dispersed. During this secondary stage, more than one kind of colloidal dispersion is introduced (each kind containing differing AOIs (or 'extra' ingredients) associated with the colloidal particles to give them unique characteristics), such that the final gelled product is then a blend of one or more individual kinds of vesicles (or colloidal particles). The AOIs or 'extras' may include zinc stearate, palmitoyl peptides, palmitoyl ascorbic acid (PAA), myristyl salicylate, tridecyl salicylate, vitamin D or vitamin E etc. (the 'tethered' ingredients). If desired, an AOI not associated with the colloidal particles can be added to the gel phase during secondary manufacture. If desired the first phase of the manufacturing process can be ordered so that the colloidal dispersions comprising the colloidal particles without any AOI are made in one or more formulations and then to each formulation an AOI (attached to a [phospho]lipid or a surfactant) is added. The second gel phase then occurs as above.

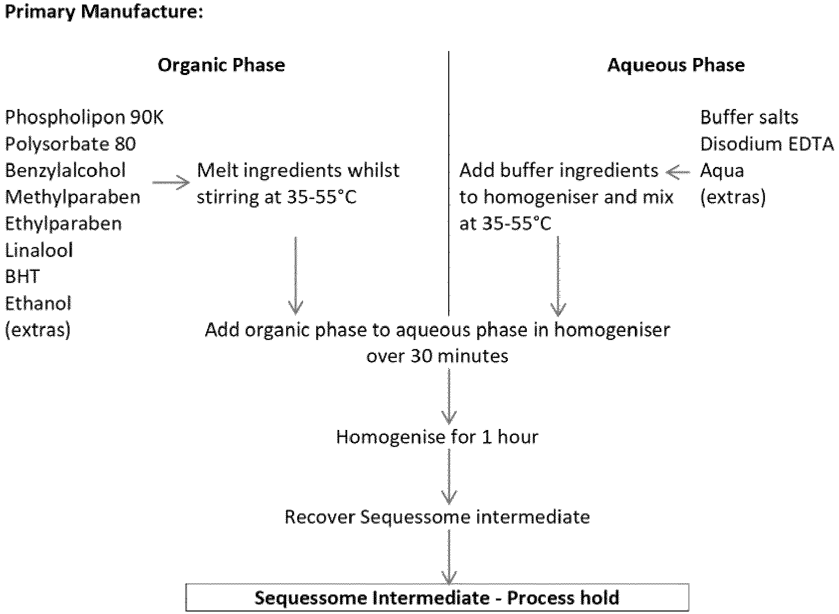

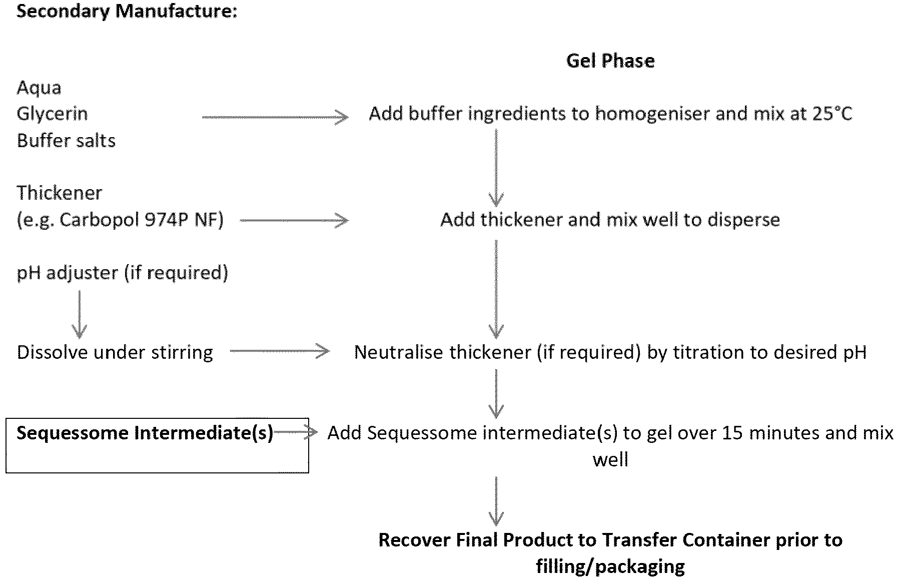

### Example 2: Example formulations

### Example Formulation 1

| **Organic Phase** - **1600ppm PAA Intermediate** | **Quantity Required Per 100g Final Product (g)** |
|---|---|
| SPC | 4.12200 |
| Ethanol | 2.19660 |
| BHT | 0.01200 |
| Methyl-4-hydroxybenzoate | 0.15000 |
| Ethyl-4-hydroxybenzoate | 0.15000 |
| Benzyl alcohol | 0.31500 |
| Polysorbate 80 | 0.40800 |
| PAA | 0.09600 |
| Linalool | 0.06000 |
| **Organic Phase Sub-Total** | **7.50960** |

| **Aqueous Phase** - **1600ppm PAA Intermediate** | |
|---|---|
| Sodium metabisulphite | 0.03000 |
| Citric acid | 0.04320 |
| Na EDTA | 0.06000 |
| diNa hydrogen phos 12H2O | 0.17280 |
| Water | 27.18420 |
| **Aqueous Phase Sub-Total** | **27.49020** |
| **PAA Tethersome Intermediate Total** | **34.99980** |
| | |

| **Organic Phase** - **300ppm Tetrapeptide Intermediate** | |
|---|---|
| SPC | 1.37400 |
| Ethanol | 0.74120 |
| BHT | 0.0040 |
| Methyl-4-hydroxybenzoate | 0.05000 |
| Ethyl-4-hydroxybenzoate | 0.05000 |
| Benzyl alcohol | 0.10500 |
| Polysorbate 80 | 0.15300 |
| Palmitoyl peptide | 0.00600 |
| Linalool | 0.02000 |
| **Organic Phase Sub-Total** | **2.50320** |

| **Aqueous Phase** - **300ppm Tetrapeptide Intermediate** | |
|---|---|
| Sodium metabisulphite | 0.01000 |
| Citric acid | 0.01440 |
| Na EDTA | 0.02000 |
| diNa hydrogen phos 12H2O | 0.05760 |
| Water | 9.06140 |
| **Aqueous Phase Sub-Total** | **9.16340** |
| **Tetrapeptide Tethersome Intermediate Total** | **11.66660** |
| | |

| **Organic Phase** - **300ppm Tripeptide Intermediate** | |
|---|---|
| SPC | 1.37400 |
| Ethanol | 0.74120 |
| BHT | 0.00400 |
| Methyl-4-hydroxybenzoate | 0.05000 |
| Ethyl-4-hydroxybenzoate | 0.05000 |
| Benzyl alcohol | 0.10500 |
| Polysorbate 80 | 0.15300 |
| Palmitoyl peptide | 0.00600 |
| Linalool | 0.02000 |
| **Organic Phase Sub-Total** | **2.50320** |

| **Aqueous Phase - 300ppm Tripeptide Intermediate** | |
|---|---|
| Sodium metabisulphite | 0.01000 |
| Citric acid | 0.01440 |
| Na EDTA | 0.02000 |
| diNa hydrogen phos 12H2O | 0.05760 |
| Water | 9.06140 |
| **Aqueous Phase Sub-Total** | **9.16340** |
| **Tripeptide Tethersome Intermediate Total** | **11.66660** |
| **Sum of Tethersome Intermediates** | **58.33300** |

| **Gel Phase** - **Final Product** | |
|---|---|
| Sodium hydroxide | 0.11300 |
| Carbopol 974P | 0.75000 |
| Glycerol | 3.00000 |
| Citric acid | 0.05600 |
| diNa hydrogen phos 12H2O | 0.24200 |
| Water | 37.50600 |
| **Gel Phase Sub-Total** | **41.66700** |
| **Overall Total** | **100.00000** |

### Example Formulation 2

| **Organic Phase** - **1600ppm PAA Intermediate** | **Quantity Required Per 100g Final Product (g)** |
|---|---|
| SPC | 2.06100 |
| Ethanol | 1.09830 |
| BHT | 0.00600 |
| Methyl-4-hydroxybenzoate | 0.07500 |
| Ethyl-4-hydroxybenzoate | 0.07500 |
| Benzyl alcohol | 0.15750 |
| Polysorbate 80 | 0.20400 |
| PAA | 0.04800 |
| Linalool | 0.03000 |
| **Organic Phase Sub-Total** | **3.75480** |
| **Aqueous Phase** - **1600ppm PAA Intermediate** | |
| Sodium metabisulphite | 0.01500 |
| Citric acid | 0.02160 |
| diNa hydrogen phos 12H2O | 0.08640 |
| Water | 13.62210 |
| **Aqueous Phase Sub-Total** | **13.74510** |
| **PAA Tethersome Intermediate Total** | **17.49990** |
| | |

| **Organic Phase** - **300ppm Tetrapeptide Intermediate** | |
|---|---|
| SPC | 0.68700 |
| Ethanol | 0.37060 |
| BHT | 0.00200 |
| Methyl-4-hydroxybenzoate | 0.02500 |
| Ethyl-4-hydroxybenzoate | 0.02500 |
| Benzyl alcohol | 0.05250 |
| Polysorbate 80 | 0.07650 |
| Palmitoyl peptide | 0.00300 |
| Linalool | 0.01000 |
| **Organic Phase Sub-Total** | **1.25160** |

| **Aqueous Phase** - **300ppm Tetrapeptide Intermediate** | |
|---|---|
| Sodium metabisulphite | 0.00500 |
| Citric acid | 0.00720 |
| diNa hydrogen phos 12H2O | 0.02880 |
| Water | 4.54070 |
| **Aqueous Phase Sub-Total** | **4.58170** |
| **Tetrapeptide Tethersome Intermediate Total** | **5.83330** |
| | |

| **Organic Phase** - **300ppm Tripeptide Intermediate** | |
|---|---|
| SPC | 0.68700 |
| Ethanol | 0.37060 |
| BHT | 0.00200 |
| Methyl-4-hydroxybenzoate | 0.02500 |
| Ethyl-4-hydroxybenzoate | 0.02500 |
| Benzyl alcohol | 0.05250 |
| Polysorbate 80 | 0.07650 |
| Palmitoyl peptide | 0.00300 |
| Linalool | 0.01000 |
| **Organic Phase Sub-Total** | **1.25160** |

| **Aqueous Phase - 300ppm Tripeptide Intermediate** | |
|---|---|
| Sodium metabisulphite | 0.00500 |
| Citric acid | 0.00720 |
| diNa hydrogen phos 12H2O | 0.02880 |
| Water | 4.54070 |
| **Aqueous Phase Sub-Total** | **4.58170** |
| **Tripeptide Tethersome Intermediate Total** | **5.83330** |
| | |

| **Organic Phase - Zinc Stearate Intermediate** SPC | 3.43500 |
|---|---|
| Ethanol | 1.82850 |
| BHT | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.34000 |
| Zinc Stearate | 0.08200 |
| Linalool | 0.05000 |
| **Organic Phase Sub-Total** | **6.25800** |

| **Aqueous Phase - Zinc Stearate Intermediate** | |
|---|---|
| Sodium metabisulphite | 0.02500 |
| Citric acid | 0.03600 |
| diNa hydrogen phos 12H2O | 0.14400 |
| Water | 22.70350 |
| **Aqueous Phase Sub-Total** | **22.90850** |
| **Zinc Tethersome Intermediate Total** | **29.16650** |
| **Sum of Tethersome Intermediates** | **58.33300** |

| **Gel Phase** - **Final Product** | |
|---|---|
| Sodium hydroxide | 0.11300 |
| Carbopol 974P | 0.75000 |
| Glycerol | 3.00000 |
| Citric acid | 0.05600 |
| diNa hydrogen phos 12H2O | 0.24200 |
| Water | 37.50600 |
| **Gel Phase Sub-Total** | **41.66700** |
| **Overall Total** | **100.00000** |

### Example Formulation 3

| **Organic Phase** - **Empty Sequessome Intermediate** | **Quantity Required Per 100g Final Product (g)** |
|---|---|
| SPC | 3.57300 |
| Ethanol | 1.75000 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.23600 |
| Linalool | 0.05000 |
| **Organic Phase Sub-Total** | **6.13150** |
| **Aqueous Phase - Empty Sequessome** | |
| **Intermediate** | |
| Na dihydrogen phos 2H2O | 0.22200 |
| diNa hydrogen phos 12H2O | 0.38600 |
| Water | 34.02100 |
| **Aqueous Phase Sub-Total** | **34.62900** |
| **Empty Sequessome Intermediate Total** | **40.76050** |
| | |

| **Organic Phase** - **Zinc Stearate Intermediate** | |
|---|---|
| SPC | 3.57300 |
| Ethanol | 1.66800 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.23600 |
| Zinc stearate | 0.08200 |
| Linalool | 0.05000 |
| **Organic Phase Sub-Total** | **6.13150** |

| **Aqueous Phase** - **Zinc Stearate Intermediate** | |
|---|---|
| Na dihydrogen phos 2H2O | 0.22200 |
| diNa hydrogen phos 12H2O | 0.38600 |
| Water | 34.02100 |
| **Aqueous Phase Sub-Total** | **34.62900** |
| **Zinc Tethersome Intermediate Total** | **40.76050** |
| **Sum of Sequessome and TethersomeIntermediates** | **81.52100** |

| **Gel Phase** - **Final Product** | |
|---|---|
| Sodium hydroxide | 0.30000 |
| Carbopol 974P | 1.00000 |
| Glycerol | 3.00000 |
| Water | 14.17900 |
| **Gel Phase Sub-Total** | **18.47900** |
| **Overall Total** | **100.00000** |

### Example Formulation 4

| **Organic Phase** - **Empty Sequessome Intermediate** | **Quantity Required Per 100g Final Product (g)** |
|---|---|
| Soy phosphatidylcholine (SPC) | 3.43500 |
| Ethanol | 1.82550 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.42500 |
| Linalool | 0.05000 |
| **Organic Phase Sub-Total** | **6.25800** |

| **Aqueous Phase - Empty Sequessome Intermediate** | |
|---|---|
| Citric acid monohydrate | 0.03600 |
| diNa hydrogen phos 12H2O | 0.14400 |
| Water | 22.72850 |
| **Aqueous Phase Sub-Total** | **22.90850** |
| **Empty Sequessome Intermediate Total** | **29.16650** |
| | |

| **Organic Phase - Zinc Stearate Intermediate** | |
|---|---|
| Soy phosphatidylcholine SPC | 3.43500 |
| Ethanol | 1.74350 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.42500 |
| Zinc stearate | 0.08200 |
| Linalool | 0.05000 |
| **Organic Phase Sub-Total** | **6.25800** |

| **Aqueous Phase** - **Zinc Stearate Intermediate** | |
|---|---|
| Citric acid monohydrate | 0.03600 |
| diNa hydrogen phos 12H2O | 0.14400 |
| Water | 22.72850 |
| **Aqueous Phase Sub-Total** | **22.90850** |
| **Zinc Tethersome Intermediate Total** | **29.16650** |
| **Sum of Sequessome and TethersomeIntermediates** | **58.33300** |

| **Gel Phase** - **Final Product** | |
|---|---|
| Citric acid monohydrate | 0.05600 |
| diNa hydrogen phos 12H2O | 0.24200 |
| Methyl cellulose (4000cPs, 88,000Mw) | 1.75000 |
| Glycerol | 3.00000 |
| Water | 36.00600 |
| **Gel Phase Sub-Total** | **40.16700** |
| **Chlorhexidine digluconate** | **1.50000** |
| **Overall Total** | **100.00000** |

### Example 3: An in-use study in healthy volunteers to investigate the anti-spot efficacy of two Example Formulations comprising chlorhexidine, using objective instrumental assessments of skin sebum levels and subjective visual assessments of lesion prevalence, against a placebo following a 3-week use period.

The effectiveness of Formulation 4 and a second formulation ("Formulation 4.2") was compared with a control product in their ability to control sebum and reduce the incidence of acne in acne-prone individuals.

The Example Formulations (n = 36 and n = 37) contained empty Sequessomes™ and Tethersomes with tethered zinc and other excipients, including chlorhexidine as an anti-microbial preservative. The Sequessomes™ in the two Formulations contained different ratios of SPC:Tween (polysorbate) to assess if this affected the efficacy of the product. The control product contained no vesicles, zinc or chlorhexidine (n = 20).

### Data review

The study assessed the efficacy and safety of treatment with Formulation 4 and Formulation 4.2 compared with placebo (control) in healthy volunteers with oily and spot-prone skin, confirmed by a clinical trials assistant prior to enrolment.

Volunteers were either assessed at the centre on Day 3, 7, 14 and 21 or purely provided their own subjective assessments from home.

In total, 224 volunteers completed the 3-week study: 37 were allocated to Formulation 4, 36 were allocated to Formulation 4.2 and 20 to placebo. Objective assessments included measurement of sebum on the face, Visia CR Photography (used to assess pore size and skin smoothness) and lesion counts (comedones, microcysts, papules and pustules). The subjective assessment was carried out using a subject Self Perception Questionnaire (SPQ).

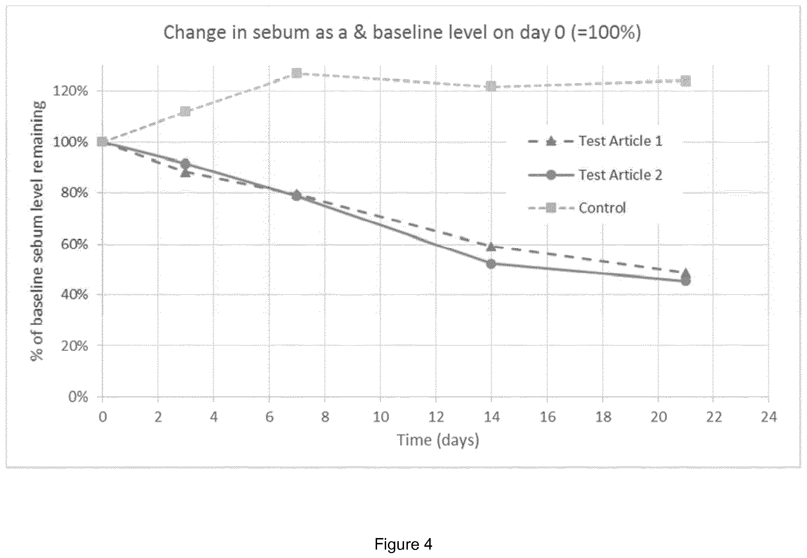

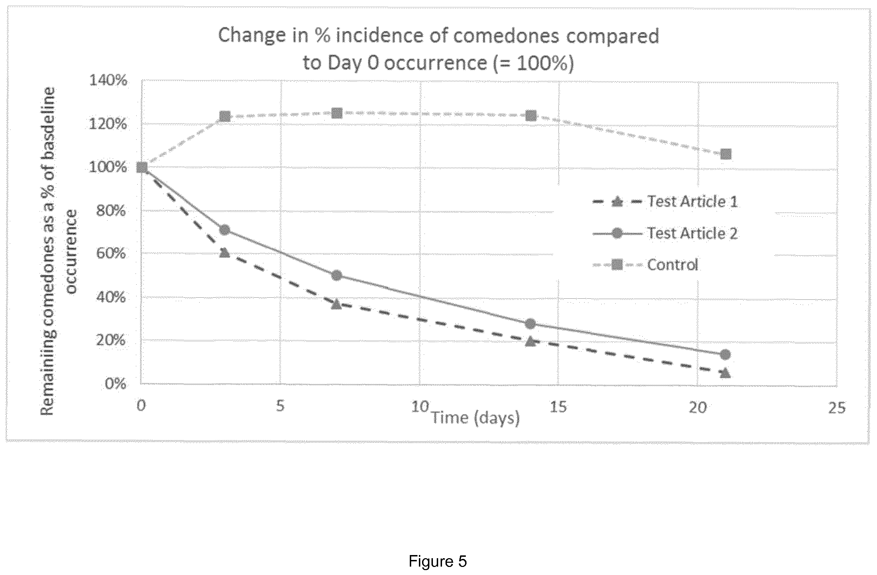

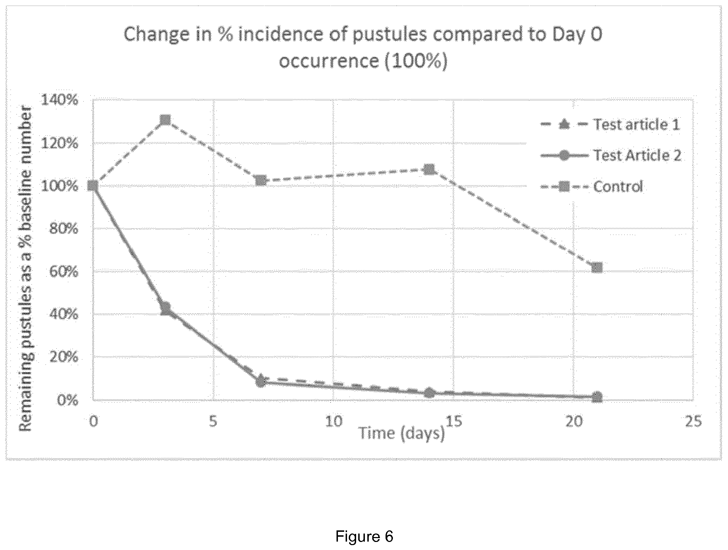

Sebum levels were reduced by over 20% from Baseline after 1 week of Formulation 4 and by more than 50% after 3 weeks' treatment (see Figure 4). Published data demonstrate that a reduction in sebum production of 30-50% correlates with reduced acne symptoms.1 The number of comedones (blocked pores) was also reduced with Formulation 4: by 50% from Baseline after 1 week and by more than 80% after 3 weeks (see Figure 5). Notably, the number of pustules was reduced by 90% from Baseline after 1 week and they were virtually cleared after 3 weeks' treatment (see Figure 6).

These objective measurements were further validated by the subjective assessments. Additional subjects were given formulations to try and questionnaires to fill in, such that the sample size for each of the groups rose to 102 subjects, of which:
- 82% reported less painful spots
- 80% said they had less shiny skin
- 76% felt their blackheads reduced
- 75% reported less swollen spots
- 75% found they had no spot recurrence.

Formulation 4 was well tolerated with no AEs reported and no erythema at the test site.

This research demonstrates that Formulation 4 is a very effective treatment for acne. The reduction in sebum and both spots and blackheads at 21 days also support the role of Formulation 4 in preventing future recurrence of the symptoms.

As mentioned, the objectively measured results demonstrated that against the control product the test products:
- Significantly reduced surface sebum (by up to 50%);
- Significantly reduced Comedones;
- Significantly reduced the numbers of papules and pustules.

**Table 4: summary of results**

| Results of objective assessment | Formulation 4.2 | Formulation 4 |
|---|---|---|
| Reduction in sebum after... | 1 week >20%; 3 weeks > 50% | 1 week >20%; 3 weeks > 50% |
| Reduction in comedones after... | 1 week > 40%; 3 weeks > 90% | 1 week = 50%; 3 weeks > 80% |
| Reduction in pustules after... | 1 week = 90%; 3 weeks > 98% | 1 week > 90%; 3 weeks > 98% |

| Results of subjective assessment: % agreeing / strongly agreeing | | |
|---|---|---|
| Product reduced blackheads | 64.7% | 76.5% |
| Product stopped spots recurring | 69.6% | 74.5% |
| Spots less painful (tender to touch) | 66.7% | 82.4% |
| Spots not as swollen | 73.5% | 75.5% |
| Skin less shiny | 59.8% | 80.4% |
| Skin less oily | 67.6% | 77.5% |
| Skin less greasy | 69.6% | 75.5% |

These results show the multifunctional nature of the formulation. It is believed that the colloidal particles in the formulations both assist in the clearing of the excess sebum from the skin surface, whilst their penetration into the skin drags the chlorhexidine in solution into the skin structure, killing bacteria. The tethered zinc may also have had a role in both reducing sebum production and/or having an anti-microbial function.

### Methods

**Table 5: Summary Protocol**

| | |
|---|---|
| Title: | An in-use study in healthy volunteers to investigate the anti-spot efficacy of two test formulations, using objective instrumental assessments of skin sebum levels and subjective visual assessments of lesion prevalence, against a placebo following a 3-week use period. |
| Study design: | Single-blind, within-subject comparison. |
| Test Groups: | **Formulation** **4.2.** CBL-DERM-14-005-E |
| | **Formulation** **4.** CBL-DERM-006-F |
| | The two test formulations were identical apart from containing colloidal particles that had slightly different SPC:Tween ratios |
| | |
| | **Control:** placebo. CBL-DERM-14-007/8-P |
| Dose regime: | The test groups followed a 12-week usage schedule according to treatment specific in-use regimes. |
| Duration of study: | 21 Days. |
| Number of subjects: | 36 subjects completed the active phase for group 1, 37 subjects for group 2 and 20 subjects completed assessments for group 3. |
| Duration of study: | Study Started: w/c 26th January 2015 |
| Study Ended: | w/e 20th February 2015 |
| Location: | Princeton Consumer Research Ltd. |
| | 307 College Road East |
| | Princeton |
| | New Jersey 08540 |

### Test Formulations

### 1. FORMULATION 4.2 AND FORMULATION 4 (CBL-DERM-14-005-E and CBL-DERM-006-F)

SOY PHOSPHATIDYLCHOLINE
BUTYLHYDROXYANISOLE
ETHANOL
ZINC STEARATE
METHYL-4-HYDROXYBENZOATE
ETHYL-4-HYDROXYBENZOATE
BENZYLALCOHOL
POLYSORBATE 80
CHLORHEXIDINE DIGLUCONATE
CITRIC ACID MONOHYDRATE
DI-SODIUM HYDROGEN ORTHOPHOSPHATE ANHYDROUS
SODIUM HYDROXIDE
CARBOPOL 974P
GLYCEROL
Linalool
WATER

The quantity of each component used is shown in the tables below. Formulation 4 (CBL-DERM-14-006-F) was the same as the exemplary composition set out in Example Formulation 4, above, except that Formulation 4 (CBL-DERM-14-006-F) contains carbopol instead of methyl cellulose (at the same quantity) and 0.11300g of sodium hydroxide (the amount of water in the gel phase is adjusted accordingly).

### 2. CONTROL (CBL-DERM-14-007/8-P)

METHYL-4-HYDROXYBENZOATE
ETHYL-4-HYDROXYBENZOATE
CITRIC ACID MONOHYDRATE
DI-SODIUM HYDROGEN ORTHOPHOSPHATE DODECAHYDRATE
SODIUM HYDROXIDE
CARBOPOL 974P (CARBOMER)
WATER

### Summary Formulation information

### Summary of Formulation 4 (CBL-DERM-14-006-F)

| **Ingredient** | **Quantity Required Per 100g Final Product (g)** |
|---|---|
| SPC (dry mass) | 6.870 |
| Polysorbate 80 | 0.850 |
| Benzylalcohol | 0.525 |
| Methyl-4-hydroxybenzoate | 0.250 |
| Ethyl-4-hydroxybenzoate | 0.250 |
| Butylhydroxyanisol | 0.020 |
| Linalool | 0.100 |
| Disodium hydrogen phosphate 12 H₂O | 0.530 |
| Citric acid monohydrate | 0.128 |
| Glycerol | 3.000 |
| Ethanol | 3.569 |
| Sodium hydroxide | 0.113 |
| Carbopol 974P NF | 0.750 |
| Water | 81.463 |

| Agent of interest - tethered to Tethersomes | |
|---|---|
| Zinc stearate | 0.082 |

| Agent of interest - present in continuous phase | |
|---|---|
| Chlorhexidine digluconate (20% solution) ** | 1.5 |
| | |
| pH | 5.5 |
| | |

| | |
|---|---|
| ** % with respect to the salt | |

### Summary of Formulation 4.2 (CBL-DERM-14-005-E)

| **Ingredient** | **Quantity Required Per 100g Final Product (g)** |
|---|---|
| SPC (dry mass) | 7.146 |
| Polysorbate 80 | 0.472 |
| Benzylalcohol | 0.525 |
| Methyl-4-hydroxybenzoate | 0.250 |
| Ethyl-4-hydroxybenzoate | 0.250 |
| Butylhydroxyanisol | 0.020 |
| Linalool | 0.100 |
| Disodium hydrogen phosphate 12 H₂O | 0.530 |
| Citric acid monohydrate | 0.128 |
| Glycerol | 3.000 |
| Ethanol | 3.418 |
| Sodium hydroxide | 0.113 |
| Carbopol 974P NF | 0.750 |
| Water | 81.716 |

| Agent of interest - tethered to Tethersomes | |
|---|---|
| Zinc stearate | 0.082 |

| Agent of interest - present in continuous phase | |
|---|---|
| Chlorhexidine digluconate (20% solution) ** | 1.5 |
| | |
| pH | 5.5 |

| | |
|---|---|
| ** % with respect to the salt | |

### Summary of Control (CBL-DERM-14-007/8-P)

| **Ingredient** | **Quantity Required Per 100g Final Product (g)** |
|---|---|
| SPC (dry mass) | |
| Polysorbate 80 | |
| Benzylalcohol | |
| Methyl-4-hydroxybenzoate | 0.250 |
| Ethyl-4-hydroxybenzoate | 0.250 |
| Butylhydroxyanisol | |
| Linalool | |
| Disodium hydrogen phosphate 12 H₂O | 0.530 |
| Citric acid monohydrate | 0.128 |
| Glycerol | |
| Ethanol | |
| Sodium hydroxide | 0.150 |
| Carbopol 974P NF | 1.000 |
| Water | 97.692 |

| Agent of interest - tethered to Tethersomes | |
|---|---|
| Zinc stearate | |

| Agent of interest - present in continuous phase | |
|---|---|
| Chlorhexidine digluconate (20% solution) ** | |
| | |
| pH | 5.5 |

| | |
|---|---|
| ** % with respect to the salt | |

### Detailed Formulation information

### Formulation 4 (CBL-DERM-14-006-F)

| **Organic Phase - Empty Sequessome Intermediate** | **Quantity Required Per 100g Final Product (g)** |
|---|---|
| SPC | 3.43500 |
| Ethanol | 1.82550 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.42500 |
| Linalool | 0.05000 |
| **Organic Phase Sub-Total** | **6.25800** |
| | |

| **Aqueous Phase** - **Empty Sequessome Intermediate** | |
|---|---|
| Citric acid monohydrate | 0.03600 |
| diNa hydrogen phos 12H2O | 0.14400 |
| Water | 22.72850 |
| **Aqueous Phase Sub-Total** | **22.90850** |
| | |
| **Empty Sequessome Intermediate Total** | **29.16650** |
| | |

| **Organic Phase - Zinc Stearate Intermediate** | |
|---|---|
| SPC | 3.43500 |
| Ethanol | 1.74350 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.42500 |
| Zinc stearate | 0.08200 |
| Linalool | 0.05000 |
| **Organic Phase Sub-Total** | **6.25800** |
| | |

| **Aqueous Phase - Zinc Stearate Intermediate** | |
|---|---|
| Citric acid monohydrate | 0.03600 |
| diNa hydrogen phos 12H2O | 0.14400 |
| Water | 22.72850 |
| **Aqueous Phase Sub-Total** | **22.90850** |
| **Zinc Tethersome Intermediate Total** | **29.16650** |
| | |
| **Sum of Sequessome and Tethersome Intermediates** | **58.33300** |
| | |
| **Gel Phase** - **Final Product** | |
| Citric acid monohydrate | 0.05600 |
| diNa hydrogen phos 12H2O | 0.24200 |
| Sodium hydroxide | 0.11300 |
| Carbopol 974P | 0.75000 |
| Glycerol | 3.00000 |
| Water | 36.00600 |
| **Gel Phase Sub-Total** | **40.16700** |
| | |
| **Chlorhexidine digluconate** | **1.50000** |
| | |
| **Overall Total** | **100.00000** |

### Formulation 4.2 (CBL-DERM-14-005-E)

| **Organic Phase - Empty Sequessome Intermediate** | **Quantity Required Per 100g Final Product (g)** |
|---|---|
| SPC | 3.57300 |
| Ethanol | 1.75000 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.23600 |
| Linalool | 0.05000 |
| **Organic Phase Sub-Total** | **6.13150** |

| **Aqueous Phase - Empty Sequessome Intermediate** | |
|---|---|
| Citric acid monohydrate | 0.06400 |
| diNa hydrogen phos 12H2O | 0.26500 |
| Water | 34.30000 |
| **Aqueous Phase Sub-Total** | **34.62900** |
| | |
| **Empty Sequessome Intermediate Total** | **40.76050** |
| | |

| **Organic Phase - Zinc Stearate Intermediate** | |
|---|---|
| SPC | 3.57300 |
| Ethanol | 1.66800 |
| BHA | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.23600 |
| Zinc stearate | 0.08200 |
| Linalool | 0.05000 |
| **Organic Phase Sub-Total** | **6.13150** |
| | |

| **Aqueous Phase** - **Zinc Stearate Intermediate** | |
|---|---|
| Citric acid monohydrate | 0.06400 |
| diNa hydrogen phos 12H2O | 0.26500 |
| Water | 34.30000 |
| **Aqueous Phase Sub-Total** | **34.62900** |
| **Zinc Tethersome Intermediate Total** | **40.76050** |
| | |
| **Sum of Sequessome and Tethersome Intermediates** | **81.52100** |
| | |

| **Gel Phase** - **Final Product** | |
|---|---|
| Sodium hydroxide | 0.11300 |
| Carbopol 974P | 0.75000 |
| Glycerol | 3.00000 |
| Water | 13.11600 |
| **Gel Phase Sub-Total** | **16.97900** |
| | |
| **Chlorhexidine digluconate** | **1.50000** |
| | |
| **Overall Total** | **100.00000** |

### Results

### Objectively measured parameters

### Sebum reduction

**Table 6: Average reduction in Sebum score from Day 0. A negative score indicates an increase in sebum.**

| | n | Day 3 | Day 7 | Day 14 | Day 21 |
|---|---|---|---|---|---|
| Formulation 4.2 | 36 | 20.47 | 34.94 | 71.64 | 90.11 |
| Formulation 4 | 37 | 14.51 | 36.30 | 85.35 | 98.49 |
| Control | 20 | -21.30 | -48.60 | -40.35 | -44.95 |
| 1 vs. 2 | t test p-values | 0.0163 | 0.6767 | 0.2065 | 0.3936 |
| 1 vs. Control | | 7.04E-16 | 5.27E-23 | 9.74E-11 | 5.69E-16 |
| 2 vs. Control | | 1.06E-14 | 1.62E-24 | 1.10E-14 | 4.63E-17 |

At all time points, both test formulations reduced sebum significantly more than the control at p<0.1%. On day 3 Formulation 4.2 was better than Formulation 4 at p<0.02. There was no significant difference in the effectiveness of the two test formulations at all later time points.

By Day 21, both test formulations had reduced the average sebum levels to less than 50% of the starting value (see Figure 4).

### Comedone reduction

**Table 7: Average reduction in occurrence of comedones from Day 0. A positive score indicates an increase in comedones.**

| | n | Day 3 | Day 7 | Day 14 | Day 21 |
|---|---|---|---|---|---|
| Formulation 4.2 | 36 | -1.44 | -2.31 | -2.92 | -3.44 |
| Formulation 4 | 37 | -1.14 | -1.97 | -2.84 | -3.38 |
| Control | 20 | 1.20 | 1.30 | 1.25 | 0.35 |
| 1 vs. 2 | t test p-values | 0.414 | 0.468 | 0.903 | 0.934 |
| 1 vs. Control | | 2.35E-06 | 2.98E-06 | 4.66E-05 | 6.63E-04 |
| 2 vs. Control | | 1.08E-07 | 2.93E-05 | 7.20E-05 | 8.97E-05 |

At all time points, both test formulations reduced comedones significantly more than the control at p<0.1%. There was no significant difference in the effectiveness of the two test formulations at all time points (see Figure 5).

### Papule reduction

**Table 8: Analysis of progression participants who had blemishes at day 0**

| | n | Day 0 | Day 3 | Day 7 | Day 14 | Day 21 |
|---|---|---|---|---|---|---|
| Formulation 4.2 | 5 | 13 | 5 | 1 | 0 | 0 |
| Formulation 4 | 7 | 16 | 9 | 5 | 0 | 0 |
| Control | 4 | 10 | 9 | 8 | 5 | 3 |

In those participants who began the trial with at least one papule, all treatments showed a reduction in papule numbers over 21 days.

**Table 9: Probabilities of U-statistic (Mann-Whitney) calculated on lesion reductions from Day 0**

| | D3 v D0 | D7 vs D0 | D14 vs D0 | D21 vs D0 |
|---|---|---|---|---|
| 1 vs. 2 | 0.146 | 0.044 | 0.373 | 0.373 |
| 1 vs. Control | 0.043 | 0.019 | 0.056 | 0.089 |
| 2 vs. Control | 0.110 | 0.093 | 0.110 | 0.254 |

The test statistic (U) shows that Formulation 4.2 was better than Control @ <5% on Day 3 and Day 7 and @ < 10% on Day 14 and Day 21 and better that Formulation 4 @<5% on Day 7. Formulation 4 was better than Control @ < 10% on Day 7.

### Pustule reduction

**Table 10: Reduction in pustule numbers from day 0. A positive score indicates an increase in numbers of pustules.**

| | n | Day 3 | Day 7 | Day 14 | Day 21 |
|---|---|---|---|---|---|
| Formulation 4.2 | 36 | -1.28 | -1.97 | -2.11 | -2.17 |
| Formulation 4 | 37 | -0.92 | -1.49 | -1.57 | -1.59 |
| Control | 20 | 0.60 | 0.05 | 0.15 | -0.75 |
| 1 vs. 2 | t test p-values | 0.127 | 0.221 | 0.228 | 0.226 |
| 1 vs. Control | | 2.71 E-08 | 2.00E-05 | 9.67E-05 | 1.37E-02 |
| 2 vs. Control | | 8.60E-07 | 7.48E-04 | 1.04E-03 | 9.15E-02 |

At all time points from Day 3 to Day 14, both test formulations reduced pustule numbers significantly more than the control at p<0.1%. At Day 21, Formulation 4.2 still outperformed control at p<2.0%; Formulation 4 outperformed control at p<10%. There was no significant difference in the effectiveness of the two test formulations at all time points (see Figure 6).

### References

1. Janiczek-Dolphin N1, Cook J, Thiboutot D, Harness J, Clucas A: Can sebum reduction predict acne outcome? Br J Dermatol. 2010 Oct;163(4):683-8. doi: 10.1111/j.1365-2133.2010.09878

### Reference Example 4: Anti-ageing trials. A summary of trials and results.

### Summary

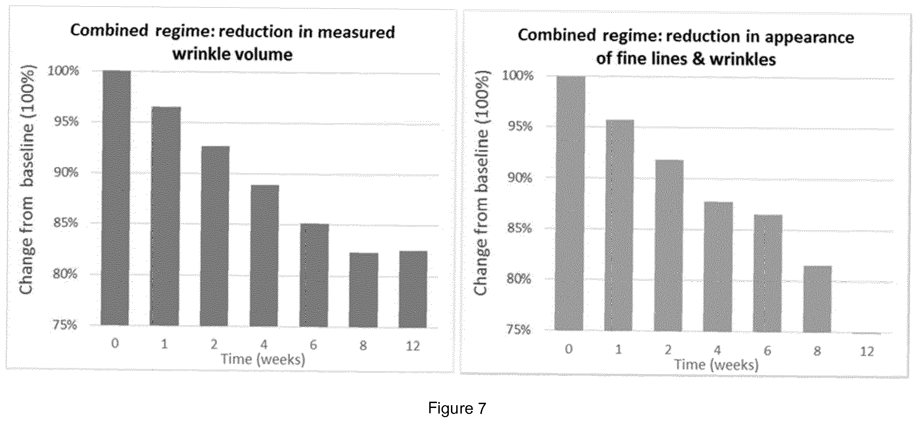

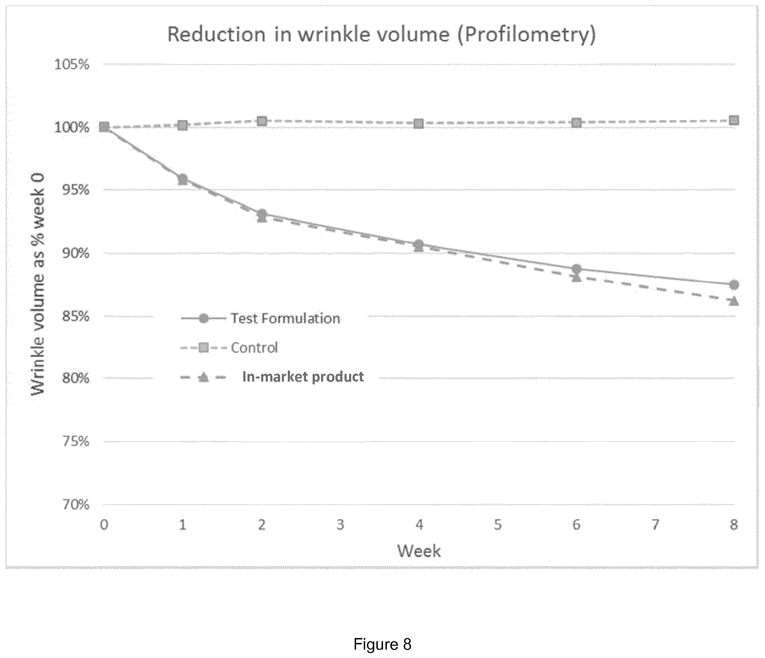

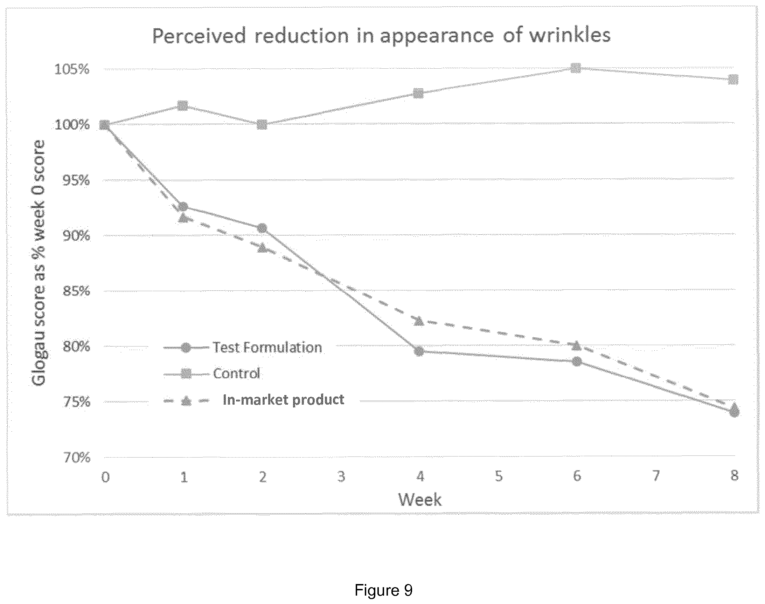

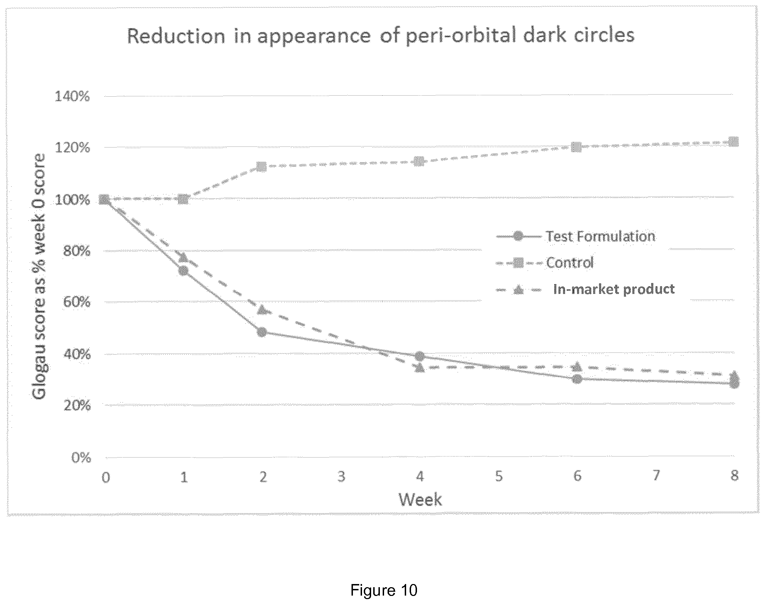

Two trials of novel products have been undertaken to assess their efficacy and how they were perceived and received by users. See Figures 7, 8, 9 and 10.

PROAWR1: A home-use study demonstrating the subjective and objective improvements in wrinkles and skin appearance after treatment with two different formulations of the same ingredients used with three different regimes

PROAWR2: home-use study in three parallel groups of a preferred formulation and regime against a marketed comparator (in-market product A) and a control (placebo) product.

The test formulas in the two trials were variants of the same ingredients, formulated by blending a mixture of Sequessome-based vesicles (i.e. colloidal particles), some of which had additional moieties tethered to the vesicle membrane.

### Headline results

### PROAWR1:

- 100% of all participants on all three regimes had measured reduction in wrinkle volume after the first week
- The average scores both wrinkle volume reduction from baseline measurements and the reduction in appearance of wrinkles from baseline scores were highly significant (p<0.001) from week one
- There was mean reduction in actual wrinkle volume of 17.46% at week 12; the mean Glogau (appearance) score had reduced by 25.86% over the same period
- In the subjective assessments:
   ∘ 100% reported that their skin felt smoother and healthier and looked and felt plumper after 8 weeks
   ∘ Over 90% reported that their skin looked smoother and healthier, felt more elastic and that the bags and circles under their eyes had reduced
   ∘ 80.8% of participants claimed that after 8 weeks their skin looked younger by between 5 to 15 years; 50% claimed their skin looked younger by between 10 to 15 years.

### PROAWR2:

For all three independently assessed metrics (actual wrinkle volume, the appearance of fine lines and wrinkles and the appearance of peri-orbital dark circles):
- Both the Test Formulation and the Commercial Product (in-market product A) were significantly better than the control in all three measures - no improvements were seen for the control group.
- The Test Formulation was equally effective as the in-market Commercial Product (in-market product A).

### PROAWR1: A home-use study demonstrating the subjective and objective improvements in wrinkles and skin appearance after treatment with a formulation in accordance with the present invention in healthy subjects with ageing and thinning skin

### Summary

This trial was conducted with two anti-ageing formulations in accordance with Example Formulation 1, "Dilute" and "Concentrated". In the Concentrated version, the tethered ascorbate and tethered peptides were present at twice the concentration that they were in the Dilute version.

The aim of this initial study was to examine the effectiveness of including these tethered moieties and whether the concentration of them in the formulations and the regime with which they were applied would affect their objective performance and the subjective perception of them by the participants.

Three regimes were followed:
- Dilute: Applied twice a day all over the face and neck
- Concentrated:Applied once a day to targeted lines and wrinkles
- Combined: Participants on this regime used both products, as directed above

The results showed that for the combined treatment:
- 100% of all participants on all three regimes had measured reduction in wrinkle volume after the first week.
- The average scores both wrinkle volume reduction from baseline measurements and the reduction in appearance of wrinkles from baseline scores were highly significant (p<0.001) from week one.
- There was mean reduction in actual wrinkle volume of 17.46% at week 12; the mean Glogau (appearance) score had reduced by 25.86% over the same period.
- In the subjective assessments:
   ∘ 100% reported that their skin felt smoother and healthier and looked and felt plumper after 8 weeks
   ∘ Over 90% reported that their skin looked smoother and healthier, felt more elastic and that the bags and circles under their eyes had reduced
   ∘ 80.8% of participants claimed that after 8 weeks their skin looked younger by between 5 to 15 years; 50% claimed their skin looked younger by between 10 to 15 years.

**Table 11: Summary Protocol**

| | | | |
|---|---|---|---|
| Title: | A randomised home-use study in three parallel groups of 30 healthy volunteers, with wrinkles, to assess the efficacy of two anti-ageing products and one anti-ageing regime. | | |
| Study design: | Single-blind, within-subject comparison, whole face design. | | |
| Test Groups: | **Group** | **Test product** | **Regime** |
| | **Group 1.** | DH3943 | Used twice a day, morning and evening, in an even layer to the face and neck |
| | n=26 | | |
| | **Group 2.** n=27 | DH3942 | Apply once a day in the evening to targeted lines or wrinkles |
| | **Group 3.** | DH3943 and DH3942 | Apply DH 3943 twice a day in an even layer to the face and neck |
| | n=26 | | Once a day apply a small quantity of DH3942 to targeted lines or wrinkles. |
| Duration of study: | 12 week treatment period, with assessments at Baseline, Weeks 1, 2, 4, 6, 8 and 12. | | |
| | Study Started: w/c 11th August 2014 | | |
| | Study Ended: w/e 21st November 2014 | | |
| Number of subjects: | 79 subjects completed the study up to Week 8. 62 subjects completed the study including the 4 week extension to week 12. | | |
| Method: | Subjects underwent Profilometry assessments after which they were issued with an instruction sheet, diary cards and the test article on Week 0 (baseline). Subjects returned to the Test Centre on Weeks 1, 2, 4, 6 and 8 for Profilometry assessments. Visual assessments were performed on both ageing and peri-orbital dark circles at baseline and Weeks 1, 2, 4, 6 and 8. The subjects completed SPQ's at Week 4 and Week 8. DIC (Digital Image Capture) was performed on approximately 40% of subjects in each group at baseline, Week 2, 4 and 8). A 4 week extension was authorised for the study in which groups 1 and 2 were instructed to continue use of the product and undergo further profilometery and visual assessments at week 12. 50% of Group 3 were instructed to continue use of the regimen whilst the remaining 50% were instructed to use a bland moisturiser. Following week 12 assessments subjects were compensated for their time and inconvenience and exited from the study. | | |
| Location: | Princeton Consumer Research Ltd. | | |
| | Harbour House | | |
| | 23 Chandlers Quay | | |
| | Maldon | | |
| | CM9 4LF | | |
| | United Kingdom | | |

**Table 12: Test Products**

| **DH3943 - "Dilute"** | **DH3942 - "Concentrated"** |
|---|---|
| Soy Phosphatidylcholine | Soy Phosphatidylcholine |
| Butylhydroxytoluene | Butylhydroxytoluene |
| Sodium metabisulphite | Sodium metabisulphite |
| Citric acid | Citric acid |
| di-Sodium hydrogen phosphate dodecahydrate | di-Sodium hydrogen phosphate dodecahydrate |
| Sodium EDTA | Sodium EDTA |
| Methyl-4-hydroxybenzoate | Methyl-4-hydroxybenzoate |
| Ethyl-4-hydroxybenzoate | Ethyl-4-hydroxybenzoate |
| Benzylalcohol | Benzylalcohol |
| Polysorbate 80 | Polysorbate 80 |
| Sodium hydroxide | Sodium hydroxide |
| Carbopol 974P | Carbopol 974P |
| Glycerol | Glycerol |
| Ethanol | Ethanol |
| Water | Water |
| Ascorbyl Palmitate | Ascorbyl Palmitate* |
| Palmitoyl Tripeptide-1 | Palmitoyl Tripeptide-1* |
| Palmitoyl Tetrapeptide-7 | Palmitoyl Tetrapeptide-7* |
| Linalool | Linalool |

| | |
|---|---|
| *In DH3942, these three components were present at double the concentration that they were in DH3943. DH3942 is the same as that in the exemplary composition set out in Example Formulation 1, above. | |

### Results

### Effect on wrinkle volume

100% of participants experienced reductions in wrinkle volume.

**Table 13: Average wrinkle volume at time t, expressed as a percentage of average volume at time 0 (=100%). Standard deviation in brackets. P value is the result of a t test comparison to the Week 0 data.**

| **Weeks** | **1** | **2** | **4** | **6** | **8** | **12** |
|---|---|---|---|---|---|---|
| DH3943 - "Dilute" | 96.9% (1.3%) p<0.001 | 93.8% (1.6%) p<0.001 | 91.9% (1.9%) p<0.001 | 90.5% (1.9%) p<0.001 | 89.0% (2.1%) p<0.001 | |
| DH3942 - "Concentrated" | 96.3% (2.1%) p<0.001 | 93.7% (2.7%) p<0.001 | 90.8% (2.7%) p<0.001 | 88.8% (2.7%) p<0.001 | 87.1% (2.7%) p<0.001 | |
| Dilute + Concentrated | 96.5% | 92.7% | 88.9% | 85.2% | 82.3% | 83% |
| | (0.9%) p<0.001 | (1.1%) p<0.001 | (1.5%) p<0.001 | (1.7%) p<0.001 | (1.9%) p<0.001 | (1.9%) p<0.001 |

### Effect on Glogau score (appearance of fine lines and wrinkles)

Glogau scores are assessments made by an independent assessor of the severity of appearance of lines and wrinkles.

**Table 14: Average Glogau score at time t, expressed as a percentage of average volume at time 0 (=100%). Standard deviation in brackets. P value is the result of a t test comparison to the Week 0 data.**

| **Weeks** | **1** | **2** | **4** | **6** | **8** | **12** |
|---|---|---|---|---|---|---|
| DH3943 - "Dilute" | 99.2% (3.9%) p=0.163 | 100% (0.0%) N/A | 99.6% (2.0%) p=0.163 | 95.4% (7.0%) p=0.001 | 88.8% (8.9%) p<0.001 | |
| DH3942 - "Concentrated" | 99.2% (4.3%) p=0.163 | 98.8% (4.3%) p=0.081 | 96.5% (7.3%) p=0.008 | 94.7% (8.9%) p=0.001 | 93.1% (12.0%) p=0.002 | |
| Dilute + Concentrated | 95.7% (6.3%) p<0.001 | 91.8% (7.7%) p<0.001 | 87.7% (7.6%) p<0.001 | 86.5% (8.6%) p<0.001 | 81.5% (10.0%) p<0.001 | 73.8% (10.4%) p<0.001 |

### Subjective questionnaire results

**Table 15: % of subjects Agreeing / Strongly agreeing with the statement**

| | DH 3942 - Conc | | DH 3943 - Dil | | Combined | |
|---|---|---|---|---|---|---|
| **Statement** | **Week 4** | **Week 8** | **Week 4** | **Week 8** | **Week 4** | **Week 8** |
| Felt smoother | 23.1% | 50.0% | 30.3% | 81.5% | 46.2% | 100.0 % |
| Looked smoother | 26.9% | 61.5% | 55.6% | 70.4% | 30.8% | 96.2% |
| Skin tone more even | 50.0% | 92.3% | 29.6% | 74.1% | 15.4% | 88.5% |
| Acne scarring reduced | 19.2% | 84.6% | 37.0% | 55.6% | 11.5% | 38.5% |
| Blemishes reduced | 26.9% | 73.1% | 29.6% | 100.0 % | 3.9% | 80.8% |
| Complexion improved | 30.8% | 61.5% | 22.2% | 100.0 % | 7.7% | 76.9% |
| Complexion more radiant | 19.2% | 38.5% | 29.6% | 96.3% | 23.1% | 88.5% |
| Age spots/hyper pigmentation reduced | 26.9% | 53.9% | 55.6% | 55.6% | 61.5% | 88.5% |
| Felt more firm | 38.5% | 50.0% | 55.6% | 92.6% | 42.3% | 80.8% |
| Looked more firm | 19.2% | 53.9% | 22.2% | 40.7% | 34.6% | 100.0 % |
| Felt more plump | 30.8% | 57.7% | 40.7% | 100.0 % | 19.2% | 100.0 % |
| Looked more plump | 19.2% | 46.2% | 59.3% | 92.6% | 34.6% | 100.0 % |
| Complexion looked healthier | 42.3% | 76.9% | 25.9% | 92.6% | 23.1% | 96.2% |
| Skin feels healthier | 53.9% | 61.5% | 74.1% | 88.9% | 42.3% | 100.0 |
| Skin felt more elastic | 46.2% | 73.1% | 55.6% | 70.4% | 26.9% | 92.3% |
| Bags/circles reduced | 30.8% | 57.7% | 44.4% | 96.3% | 30.8% | 92.3% |
| Most effective anti-ageing used | 42.3% | 76.9% | 55.6% | 81.5% | 30.8% | 76.9% |
| Would use instead of current | 46.2% | 69.2% | 55.6% | 92.6% | 65.4% | 92.3% |
| Easy to use alongside current products | 46.2% | 80.8% | 100.0 % | 96.3% | 34.6% | 61.5% |
| Would recommend to a friend | 65.4% | 76.9% | 81.5% | 100.0 | 73.1% | 100.0 |
| | | | | % | | % |
| Would buy the product | 38.5% | 76.9% | 18.5% | 81.5% | 84.6% | 92.3% |

### Skin looked younger by

| | | | | | | |
|---|---|---|---|---|---|---|
| 20+ years | 0.0% | 0.0% | 0.0% | 0.0% | 0.0% | 0.0% |
| 15+ years | 0.0% | 3.9% | 0.0% | 3.7% | 0.0% | 7.7% |
| 10+ years | 3.9% | 19.2% | 7.4% | 22.2% | 3.9% | 42.3% |
| 5+ years | 23.1% | 42.3% | 81.5% | 55.6% | 42.3% | 30.8% |
| Same | 73.1% | 34.6% | 11.1% | 18.5% | 50.0% | 19.2% |

### PROAWR2: A randomised home-use study in three parallel groups of 30 healthy volunteers, with wrinkles, to assess the efficacy of one anti-ageing product against a placebo and a comparator with an additional 73 subjects to complete a subjective user-trial using only the product

### Summary

This trial tested a preferred test anti-ageing formulation in accordance with the present invention (the "Concentrated" formulas from PROAWR1), against an in-market anti-ageing Commercial Product (in-market product A) and a control formulation.

The aim of this study was to test:
a) That previous results seen with the preferred formulation were real (control comparison)
b) To assess the effectiveness against a currently marketed formulation for anti-ageing.

The results showed that:
For all three independently assessed metrics (actual wrinkle volume, the appearance of fine lines and wrinkles and the appearance of peri-orbital dark circles):
- Both the Test Formulation and the Commercial Product were significantly better than the control in all three measures - no improvements were seen for the control group.
- The Test Formulation was equally effective as the in-market Commercial Product.

For the subjective assessment of the products:
- Both the Test Formulation and the Commercial Product were significantly better than the control in all measures
- The Test Formulation was rated higher than the Commercial Product for:
   ∘ Skin looked smoother
   ∘ Skin felt more plump
   ∘ Skin felt healthier
   ∘ Skin felt more elastic
   ∘ Complexion looked healthier
   ∘ Skin tone looked more even
   ∘ Eye puffiness reduction
   ∘ Effectiveness in anti-ageing
   ∘ Lifted sagging skin
   ∘ Hydration of skin
   ∘ Reducing pore size

**Table 16: Summary Protocol**

| | | | |
|---|---|---|---|
| Title: | A randomised home-use study in three parallel groups of 30 healthy volunteers, with wrinkles, to assess the efficacy of one anti-ageing product against a placebo and a comparator with an additional 70 subjects to complete a subjective user-trial using only the product. | | |
| Study design: | Single-blind, within-subject comparison, whole face design. | | |
| Test Groups: | **Group** | **Test product** | **Regime** |
| | **Group 1.** | Test Formulation | • Apply twice a day |
| | | | • Apply all over to |
| | **Group 2.** | Control | face and neck |
| | | | • Apply additional amounts to areas where wrinkles/ageing is more noticeable |
| | **Group 3.** | In-market product A | |
| | | | • Apply a thin layer to the sub-orbital eye sacks |
| Duration of study: | 8 week treatment period, with assessments at Baseline, Week 4, and Week 8. | | |
| | Study Started: w/c 16th February 2015 | | |
| | Study Ended: w/e 24th April 2015 | | |
| Method: | Subjects underwent Profilometry assessments after which they were issued with an instruction sheet, diary cards and the test article on Week 0 (baseline). Subjects returned to the Test Centre on Weeks 1, 2, 4, 6 and 8 for Profilometry assessments. When the subjects returned to the Test Centre on Week 8, they returned any unused test articles and their completed diary cards. Visual assessments were performed on both ageing and peri-orbital dark circles at baseline and Weeks 1, 2, 4, 6 and 8. The subjects completed SPQ's at Week 8. Professional photography was performed on approximately 40% of subjects in each group at baseline, and Weeks 1, 2, 4 and 8 with as many subjects as possible for group 1 being photographed above and beyond 40%. 70 subjects were also issued the product to use at home for the duration of the test period and completed an SPQ at the same time points as the active groups. | | |
| Location: | Princeton Consumer Research Ltd. | | |
| | Harbour House | | |
| | 23 Chandlers Quay | | |
| | Maldon | | |
| | CM9 4LF | | |
| | United Kingdom | | |

**Table 17: Test Products**

| **Test Formulation** | **Control** |
|---|---|
| Soy Phosphatidylcholine | |
| Butylhydroxytoluene | |
| Ethanol | |
| Methyl-4-hydroxybenzoate | Methyl-4-hydroxybenzoate |
| Ethyl-4-hydroxybenzoate | Ethyl-4-hydroxybenzoate |
| Benzylalcohol | |
| Polysorbate 80 | |
| Linalool | |
| Ascorbyl Palmitate | |
| Palmitoyl Tripeptide-1 | |
| Palmitoyl Tetrapeptide-7 | |
| Sodium metabisulphite | |
| Citric acid monohydrate | Citric acid monohydrate |
| di-Sodium hydrogen orthophosphate anhydrous | di-Sodium hydrogen orthophosphate anhydrous |
| Sodium EDTA | |
| Sodium hydroxide | Sodium hydroxide |
| Carbopol 974P | Carbopol 974P |
| Glycerol | |
| Water | Water |

### Results

### Effect on wrinkle volume

**Table 18: Average wrinkle volume at time t, expressed as a percentage of average volume at time 0 (= 100%). Standard deviation in brackets. P value is the result of a t test comparison to the Week 0 data.**

| | **n** | **Week 1** | **Week 2** | **Week 4** | **Week 6** | **Week 8** |
|---|---|---|---|---|---|---|
| Test Formulation | 27 | 95.9% (2.0%) p<0.001 | 93.1% (2.9%) p<0.001 | 90.7% (3.6%) p<0.001 | 88.8% (3.9%) p<0.001 | 87.5% (4.1%) p<0.001 |
| **Control** | 28 | 100.2% (1.0%) P=0.31 | 100.5% (2.0%) P=0.17 | 100.3% (2.9%) P=0.57 | 100.4% (3.0%) P=0.51 | 100.5% (3.3%) P=0.39 |
| **In-market product A** | 29 | 95.8% (2.5%) p<0.001 | 92.9% (3.3%) p<0.001 | 90.5% (3.8%) p<0.001 | 88.1% (4.1%) p<0.001 | 86.2% (4.3%) p<0.001 |

**Table 19: p values for t-tests between different treatments**

| | **Week 1** | **Week 2** | **Week 4** | **Week 6** | **Week 8** |
|---|---|---|---|---|---|
| Test Formulation vs. Control | p<0.001 | p<0.001 | p<0.001 | p<0.001 | p<0.001 |
| Test Formulation vs. in-market product A | p=0.554 | p=0.623 | p=0.604 | p=0.817 | p=0.947 |
| In-market product A vs. Control | p<0.001 | p<0.001 | p<0.001 | p<0.001 | p<0.001 |

- Test Formulation and in-market product A significantly different from Control.
- No significant difference between Test Formulation and in-market product A.

### Effect on Glogau score (appearance of fine lines and wrinkles)

Glogau scores are assessments made by an independent assessor of the severity of appearance of lines and wrinkles.

**Table 20: Average Glogau score at time t, expressed as a percentage of average volume at time 0 (=100%). Standard deviation in brackets. P value is the result of a t test comparison to the Week 0 data.**

| | **n** | **Week 1** | **Week 2** | **Week 4** | **Week 6** | **Week 8** |
|---|---|---|---|---|---|---|
| **Test Formulation** | 27 | 92.6% | 90.7% | 79.5% | 78.5% | 73.9% |
| | | (8.5%) | (8.3%) | (9.8%) | (8.9%) | (7.4%) |
| | | p<0.001 | p<0.001 | p<0.001 | p<0.001 | p<0.001 |
| **Control** | 28 | 101.7% | 100.0% | 102.8% | 105.0% | 103.9% |
| | | (10.8%) | (10.7%) | (8.0%) | (13.4%) | (12.8%) |
| | | p=0.81 | p=0.79 | p=0.21 | p=0.12 | p=0.31 |
| **In-market product A** | 29 | 91.6% | 88.9% | 82.2% | 79.9% | 74.3% |
| | | (8.1%) | (8.4%) | (8.5%) | (9.5%) | (10.5%) |
| | | p<0.001 | p<0.001 | p<0.001 | p<0.001 | p<0.001 |

**Table 21: p values for t-tests between different treatments**

| | **Week 1** | **Week 2** | **Week 4** | **Week 6** | **Week 8** |
|---|---|---|---|---|---|
| Test Formulation vs. Control | p=0.266 | p=0.198 | p=0.004 | p<0.001 | p<0.001 |
| Test Formulation vs. in-market product A | p=0.976 | p=0.998 | p=0.681 | p=0.698 | p=0.799 |
| in-market product A vs. Control | p=0.318 | p=0.245 | p=0.018 | p=0.005 | p=0.001 |

- Test Formulation and in-market product A significantly different from Control from week 4.
- No significant difference between Test Formulation and in-market product A.

### Effect on Peri-orbital dark circles

**Table 22: Average PO Dark circle score at time t, expressed as a percentage of average volume at time 0 (=100%). Standard deviation in brackets. P value is the result of a t test comparison to the Week 0 data.**

| | **n** | **Week 1** | **Week 2** | **Week 4** | **Week 6** | **Week 8** |
|---|---|---|---|---|---|---|
| **Test Formulation** | 27 | 72.2% | 48.1% | 38.9% | 29.6% | 27.8% |
| | | (34.9%) | (37.9%) | (42.4%) | (42.2%) | (37.6%) |
| | | p<0.001 | p<0.001 | p<0.001 | p<0.001 | p<0.001 |
| **Control** | 28 | 100% | 112.5% | 114.3% | 119.6% | 121.4% |
| | | (0%) | (44.4%) | (54.2%) | (59.8%) | (58.4%) |
| | | p=N/A | p=0.49 | p=0.79 | p=0.63 | p=0.45 |
| **In-market product A** | 29 | 77.6% | 56.9% | 34.5% | 34.5% | 31.0% |
| | | (34.3%) | (43.8%) | (40.3%) | 42.5%) | (41.0%) |
| | | p<0.001 | p<0.001 | p<0.001 | p<0.001 | p<0.001 |

**Table 23: p values for t-tests between different treatments**

| | **Week 1** | **Week 2** | **Week 4** | **Week 6** | **Week 8** |
|---|---|---|---|---|---|
| Test Formulation vs. Control | p=0.013 | p<0.001 | p<0.001 | p<0.001 | p<0.001 |
| Test Formulation vs. in-market product A | p=0.949 | p=0.589 | p=0.794 | p=0.562 | p=0.962 |
| in-market product A vs. Control | p=0.008 | p<0.001 | p<0.001 | p<0.001 | p<0.001 |

- Test Formulation and in-market product A significantly different from Control from week 1.
- No significant difference between Test Formulation and in-market product A.

### Subjective questionnaire results

**Table 24:**

| | **Test Formulation N = 27+73*** | **In market Product A N=26** | **Control N=28** |
|---|---|---|---|
| After using the product... | | | |
| my skin felt significantly smoother | 82.00% | 89.66% | 28.57% |
| my skin looked significantly smoother | 85.00% | 55.17% | 46.43% |
| my skin felt significantly more firm | 89.00% | 89.66% | 42.86% |
| my skin looked significantly more firm | 54.00% | 58.62% | 46.43% |
| my skin felt significantly more plump | | 86.21% | 25.00% |
| my skin looked significantly more plump | 56.00% | 68.97% | 25.00% |
| my skin feels significantly healthier | 86.00% | 79.31% | 46.43% |
| my skin felt more elastic | 83.00% | 82.76% | 28.57% |
| my complexion was visibly improved | 58.00% | 65.52% | 25.00% |
| my complexion was visibly more radiant | 61.00% | 82.76% | 21.43% |
| my complexion looked significantly healthier | 90.00% | 86.21% | 32.14% |
| my skin tone looked significantly more even | 90.00% | 79.31% | 25.00% |
| any age spots / hyperpigmentation were significantly reduced | 67.00% | 72.41% | 46.43% |
| the bags under my eyes were significantly reduced | 89.00% | | 35.71% |
| the puffiness all around my eyes was significantly reduced | 88.00% | 86.21% | 39.29% |
| the dark circles under my eyes were significantly reduced | 86.00% | 89.66% | 42.86% |
| sagging skin was lifted | 89.00% | 86.21% | 46.43% |
| my skin looked: | | | |
| *at least 5 years younger* | 76.00% | 82.76% | 39.29% |
| *at least 10 years younger* | 35.00% | 34.48% | 0.00% |
| The product... | | | |
| redefined sagged skin | 58.00% | 75.86% | 28.57% |
| has a pleasant fragrance | 62.00% | 58.62% | 42.86% |
| has a pleasant consistency and texture | | 79.31% | 32.14% |
| has a pleasant appearance | 61.00% | 65.52% | 28.57% |
| felt good on my skin after or upon application | 66.00% | 65.52% | 32.14% |
| dried quickly | 89.00% | | 32.14% |
| was easy to use alongside my usual skincare products | | 86.21% | 28.57% |
| was the most effective anti-ageing serum I have used | 86.00% | 62.07% | 39.29% |
| redefined facial contours that were sagged | 67.00% | 72.41% | 39.29% |
| hydrated my skin | | 86.21% | 25.00% |
| has visibly reduced the size of my pores | 85.00% | 68.97% | 17.86% |
| has visibly reduced the appearance of any thread veins | 69.00% | 75.86% | 25.00% |
| I would... | | | |
| recommend the product to a friend | 79.00% | | 32.14% |
| buy this product in place of my usual anti-ageing serum | | | 57.14% |

| | | | |
|---|---|---|---|
| * An additional 73 subjects used the Test Formulation and answered the self-assessment questionnaire but were not independently assessed for wrinkle volume, wrinkle appearance or appearance of peri-orbital dark circles. | | | |

### Reference Example 5: A study in volunteers to investigate the sensation produced using a formulation comprising capsaicin

### Background

There are several products on the market that provide a warming sensation when applied to the joint or muscle, to relieve aches or pains. These products contain chemicals such as capsaicin which stimulate the nerve endings in the skin to create the warming sensations (without actually being related to a physical temperature change).

The inventors have developed a capsaicin-containing formulation in accordance with the present invention, so that a warming sensation is associated with the product, either at application or sometime after. The formulation provides a combined effect of the joint-pain relieving property of drug-free colloidal dispersions, combined with a warming feeling.

The formulation (TP2) comprised insoluble and inflexible liposomes comprising capsaicin and menthol in combination with Transfersomes™ comprising menthol.

Transfersomes™/Sequessomes™/Tethersomes are flexible, deformable colloidal particles that make their own way into the skin, whereas liposomes are rigid vesicles that would not ordinarily be expected to penetrate the skin by themselves unless some other force is applied (e.g. Sequessomes™/Transfersomes™/Tethersomes in the same mixture opening pores and pulling/pushing the liposomes through).

The starting material used to make TP2 is set out below.

TP2 (PD-14-0073) was formed by taking 90g of Start Material 1 and adding/mixing in 10g of Start Material 2.

### Start Material 1 (Flexible, empty, menthol-fragranced vesicles):

| **Organic Phase - Empty Transfersome Intermediate** | **Quantity Required Per 100g Final Product (g)** |
|---|---|
| SPC (Dry Mass) | 6.87000 |
| Ethanol | 3.65100 |
| BHT | 0.02000 |
| Methyl-4-hydroxybenzoate | 0.25000 |
| Ethyl-4-hydroxybenzoate | 0.25000 |
| Benzyl alcohol | 0.52500 |
| Polysorbate 80 | 0.85000 |
| Capsaicin | 0.00000 |
| Menthol | 0.10000 |
| **Organic Phase Sub-Total** | **12.51600** |

| **Aqueous Phase - Empty Transfersome Intermediate** | |
|---|---|
| Sodium dihydrogen orthophosphate 2 H₂O | 0.03700 |
| disodium hydrogen orthophosphate 12 H₂O | 0.45300 |
| Sodium EDTA | 0.10000 |
| Water | 45.22700 |
| **Aqueous Phase Sub-Total** | **45.81700** |
| **Intermediate Transfersome Total** | **58.33300** |
| | |

| **Gel Phase** - **Final Product** | |
|---|---|
| Sodium dihydrogen orthophosphate 2 H₂O | 0.02400 |
| disodium hydrogen orthophosphate 12 H₂O | 0.30200 |
| Sodium hydroxide | 0.63000 |
| Carbopol 974P NF | 1.25000 |
| Glycerol | 3.00000 |
| Water | 36.46100 |
| **Gel Phase Sub-Total** | **41.66700** |
| **Overall Total** | **100.00000** |

### Start Material 2 (Capsaisin liposomes):

| **Organic Phase** - **Liposome Intermediate** | **Quantity Required Per 100g Final Product (g)** |
|---|---|
| SPC (Dry Mass) | 6.87000 |
| Ethanol | 4.25100 |
| BHT | 0.02000 |
| Methyl-4-hydroxybenzoate | 0.25000 |
| Ethyl-4-hydroxybenzoate | 0.25000 |

| | |
|---|---|
| Benzyl alcohol | 0.52500 |
| Polysorbate 80 | 0.00000 |
| Capsaicin | 0.25000 |
| Menthol | 0.10000 |
| **Organic Phase Sub-Total** | **12.51600** |

| **Aqueous Phase - Liposome Intermediate** | |
|---|---|
| Sodium dihydrogen orthophosphate 2 H₂O | 0.03700 |
| disodium hydrogen orthophosphate 12 H₂O | 0.45300 |
| Sodium EDTA | 0.10000 |
| Water | 45.22700 |
| **Aqueous Phase Sub-Total** | **45.81700** |
| **Liposome Intermediate Total** | **58.33300** |
| | |

| **Gel Phase** - **Final Product** | |
|---|---|
| Sodium dihydrogen orthophosphate 2 H₂O | 0.02400 |
| disodium hydrogen orthophosphate 12 H₂O | 0.30200 |
| Sodium hydroxide | 0.63000 |
| Carbopol 974P NF | 1.25000 |
| Glycerol | 3.00000 |
| Water | 36.46100 |
| **Gel Phase Sub-Total** | **41.66700** |
| **Overall Total** | **100.00000** |

### Summary formulae:

| **Title** | **Start Material 1** | **Start Material 2** | **PD-14-0073** |
|---|---|---|---|
| **Ingredient** | | | **(90% Start Material 1 plus 10% Start Material 2** |
| SPC (dry mass) | 6.870g | 6.870g | 6.870g |
| Polysorbate 80 | 0.850g | - | 0.765g |
| Benzylalcohol | 0.525g | 0.525g | 0.525g |
| Methyl-4-hydroxybenzoate | 0.250g | 0.250g | 0.250g |
| Ethyl-4-hydroxybenzoate | 0.250g | 0.250g | 0.250g |
| Butylhydroxytoluene | 0.020g | 0.020g | 0.020g |
| Disodium EDTA | 0.100g | 0.100g | 0.100g |
| Disodium hydrogen phosphate 12 H₂O | 0.755g | 0.755g | 0.755g |
| Sodium dihydrogen phosphate 2 H₂O | 0.061g | 0.061g | 0.061g |
| Glycerol | 3.000g | 3.000g | 3.000g |
| Ethanol | 3.651g | 4.251g | 3.711g |
| Sodium hydroxide | 0.630g | 0.630g | 0.630g |
| Carbopol 974P NF | 1.250g | 1.250g | 1.250g |
| Water | 81.688g | 81.688g | 81.688g |

| Agent of interest - in continuous phase | | | |
|---|---|---|---|
| Capsaicin | - | 0.250g | 0.025g |

| Agent of interest - in Transfersome | | | |
|---|---|---|---|
| Menthol | 0.100g | 0.100g | 0.100g |
| Total mass | 100.000g | 100.000g | 100.000g |

### Results

The formulation was tried by three individuals. The results are presented in the table below.

**Table 26**

| **Test product** | **TP2 (PD-14-0073)** |
|---|---|
| **Capsaicin** | In liposomes |
| **Menthol** | In membranes of Transfersomes™ and of liposomes |

| CAPSAICIN: | |
|---|---|
| Onset of sensation | 5 to 30 mins |
| Onset of optimal sensation | 15 to 60 mins |
| Duration of sensation | 45 to 180 mins |
| Intensity of sensation | Pleasant |

| MENTHOL: | |
|---|---|
| Duration of sensation | 15 to 30 mins |
| Intensity of sensation | Enough |

### Conclusion / Summary

These early results are useful in showing that:
- The warming sensation of capsaicin can be felt:
   ∘ In Transfersomes™ - in the same "phase" as a joint-targeting colloidal particle
   ∘ In liposomes - separate "phase" to the joint-targeting colloidal particles

### Reference Example 6: A user trial study in healthy volunteers to investigate the efficacy of a formulation comprising caffeine in improving the appearance of periorbital skin

This study tested the efficacy of a formulation comprising caffeine and tocopherol in the continuous phase and three types of deformable colloidal particles: 1) Tethersomes to which palmitoyl ascorbate is tethered, 2) Tethersomes to which palmitoyl tripeptide-1 is tethered, and 3) Tethersomes to which palmitoyl tetrapeptide-7 is tethered.

### Methods

### Summary protocol

| | |
|---|---|
| Study design: | Single-blind |
| Test article: | CBL-DERM-15-013 Periorbital skin serum |
| Duration of treatment: | 4 weeks |
| Number of subjects: | 102 |
| Type of subjects: | Healthy male and female subjects (from a breadth of ethnicities) aged between 40 and 70 years old (in equal proportions, 33% each 10 year age group), from a breadth of ethnicities and with a variety of skin types (normal, combination, dry, oily, sensitive) who suffer from the appearance of two of the following characteristics: |
| | Periorbital puffiness - swelling around the eyes |
| | Puffiness below the lower eyelids - 'eye bags' |
| | Periorbital dark circles caused by a) aging b) heredity and c) hyperpigmentation (dark skinned people) |
| | (There must be an equal representation of all three characteristics to ensure claims objectivity). |
| | All subjects must have visible wrinkles, fine lines and crow's feet around the eye area |
| Observations: | Subjects were asked to apply the product as per usage instructions. They were then asked to complete a Self-Perception Questionnaire (SPQ) after two weeks and at the end of the study after four weeks. |
| Treatment: | Subjects were issued with samples of the test article and directions for use following standard in-use application regime. |
| Location | Princeton Consumer Research |
| | Harbour House |
| | 23 Chandlers Quay |
| | Maldon |
| | CM9 4LF |
| | United Kingdom |

### Formulation (CBL-DERM-15-013)

| **Organic Phase - 1600ppm PAA Intermediate** | **Quantity Required Per 100g Final Product (g)** |
|---|---|
| Soy phosphatidylcholine (SPC) | 4.12200 |
| Ethanol | 1.99660 |
| Butylhydroxyanisole (BHA) | 0.01200 |
| Methyl-4-hydroxybenzoate | 0.15000 |
| Ethyl-4-hydroxybenzoate | 0.15000 |
| Benzyl alcohol | 0.31500 |
| Polysorbate 80 | 0.40800 |
| Palmitoyl ascorbic acid | 0.09600 |
| +/- alpha tocopherol | 0.20000 |
| Linalool | 0.06000 |
| **Organic Phase Sub-Total** | **7.50960** |

| **Aqueous Phase - 1600ppm PAA intermediate** | |
|---|---|
| Sodium metabisulphite | 0.03000 |
| Citric acid monohydrate | 0.04320 |
| Disodium EDTA Disodium hydrogen orthophosphate | 0.06000 |
| dodecahydrate | 0.17280 |
| Caffeine | 0.05000 |
| Water | 27.13420 |
| **Aqueous Phase Sub-Total** | **27.49020** |
| **PAA Tethersome Intermediate Total** | **34.99980** |
| | |

| **Organic Phase - 300ppm Tetrapeptide Intermediate** | |
|---|---|
| Soy phosphatidylcholine (SPC) | 1.37400 |
| Ethanol | 0.74120 |
| Butylhydroxyanisole (BHA) | 0.00400 |
| Methyl-4-hydroxybenzoate | 0.05000 |
| Ethyl-4-hydroxybenzoate | 0.05000 |
| Benzyl alcohol | 0.10500 |
| Polysorbate 80 | 0.15300 |
| Palmitoyl peptide | 0.00600 |
| Linalool | 0.02000 |
| **Organic Phase Sub-Total** | **2.50320** |

| **Aqueous Phase** - **300ppm Tetrapeptide Intermediate** | |
|---|---|
| Sodium metabisulphite | 0.01000 |
| Citric acid monohydrate | 0.01440 |
| Disodium EDTA Disodium hydrogen orthophosphate | 0.02000 |
| dodecahydrate | 0.05760 |
| Water | 9.06140 |
| **Aqueous Phase Sub-Total** | **9.16340** |
| **Tetrapeptide Tethersome Intermediate Total** | **11.66660** |

| **Organic Phase** - **300ppm Tripeptide Intermediate** | |
|---|---|
| Soy phosphatidylcholine (SPC) | 1.37400 |
| Ethanol | 0.74120 |
| Butylhydroxyanisole (BHA) | 0.00400 |
| Methyl-4-hydroxybenzoate | 0.05000 |
| Ethyl-4-hydroxybenzoate | 0.05000 |
| Benzyl alcohol | 0.10500 |
| Polysorbate 80 | 0.15300 |
| Palmitoyl peptide | 0.00600 |
| Linalool | 0.02000 |
| **Organic Phase Sub-Total** | **2.50320** |

| **Aqueous Phase** - **300ppm Tripeptide Intermediate** | |
|---|---|
| Sodium metabisulphite | 0.01000 |
| Citric acid monohydrate | 0.01440 |
| Disodium EDTA Disodium hydrogen orthophosphate | 0.02000 |
| dodecahydrate | 0.05760 |
| Water | 9.06140 |
| **Aqueous Phase Sub-Total** | **9.16340** |
| **Tripeptide Tethersome Intermediate Total** | **11.66660** |
| **Sum of Sequessome and Tethersome Intermediates** | **58.33300** |

| **Gel Phase - Final Product** | |
|---|---|
| Sodium hydroxide | 0.16000 |
| Carbopol 974P NF | 0.75000 |
| Glycerol | 3.00000 |
| Citric acid monohydrate Disodium hydrogen orthophosphate | 0.05600 |
| dodecahydrate | 0.24200 |
| Water | 37.45900 |
| **Gel Phase Sub-Total** | **41.66700** |
| **Overall Total** | **100.00000** |

### Summary formulae of CBL-DERM-15-013

| **Ingredient (percentage; g per 100g)** | **Periorbital Skin** |
|---|---|
| SPC (dry mass) | 6.870 |
| Polysorbate 80 | 0.714 |
| Benzylalcohol | 0.525 |
| Methyl-4-hydroxybenzoate | 0.250 |
| Ethyl-4-hydroxybenzoate | 0.250 |
| Butylhydroxyanisole | 0.020 |
| Linalool | 0.100 |
| Sodium metabisulphite | 0.050 |
| Disodium EDTA | 0.100 |
| Disodium hydrogen phosphate 12 H₂O | 0.530 |
| Citric acid monohydrate | 0.128 |
| Glycerol | 3.000 |
| Ethanol *** | 3.479 |
| Sodium hydroxide | 0.160 |
| Carbopol 974P NF | 0.750 |
| Water | 82.716 |

| Agent of interest - tethered to Tethersomes | |
|---|---|
| Palmitoyl tripeptide-1 | 0.006 |
| Palmitoyl tetrapeptide-7 | 0.006 |
| Palmitoyl ascorbate | 0.096 |

| Agent of interest - in continuous phase | |
|---|---|
| Tocopherol | 0.200 |
| Caffeine | 0.050 |
| | |
| pH | 5.5* |

| | |
|---|---|
| * Final pH approximate; to be confirmed | |

### Results

Within-treatment analysis (p<0.05) of periorbital clinical assessment shows there to be a statistically significant reduction in wrinkles (15.69%), fine lines (31.22%), crow's feet (21.47%), puffiness (38.07%), dark circles (26.01%) and eye bags (38.94%) after 4 weeks of product usage.

The product performed statistically favourably over the 4 week study in the majority of attributes under Clearcast guidelines of advertising. The product showed a preference or favourability in the majority of attributes:
- After using the product, 96.08% of users agreed, or strongly agreed the product reduced the appearance of puffiness and swelling around their eyes.
- After using the product, 96.08% of users agreed, or strongly agreed their skin felt and looked less thin.
- After using the product, 93.14% of users agreed, or strongly agreed their skin felt more elastic.
- After using the product, 96.08% of users agreed, or strongly agreed the product reduced the appearance of swelling and puffiness below the lower eyelids (eye bags).
- After using the product, 90.20% of users agreed, or strongly agreed the product lifted sagged skin (reduction in skin droopiness and sagging).
- After using the product, 92.16% of users agreed, or strongly agreed the product reduced the appearance of under eye dark circles.
- After using the product, 95.10% of users agreed, or strongly agreed their skin looked and felt more firm.
- After using the product, 90.20% of users agreed, or strongly agreed there was a reduction in fine lines (inc. crow's feet) around the eye area.
- After using the product, 87.25% of users agreed, or strongly agreed there was a reduction in deep wrinkles (inc. crow's feet) around the eye area.
- After using the product, 93.14% of users agreed, or strongly agreed their skin tone looked more even.
- After using the product, 90.20% of users agreed, or strongly agreed their eye contor looked and felt tighter.
- After using the product, 90.20% of users agreed, or strongly agreed their eye contour looked more toned/lifted.
- After using the product, 96.08% of users agreed, or strongly agreed their eyes looked rested/less tired.
- After using the product, 97.06% of users agreed, or strongly agreed their eye skin was more hydrated.
- After using the product, 95.10% of users agreed, or strongly agreed their skin looked smoother.
- After using the product, 99.02% of users agreed, or strongly agreed the product was gentle and well tolerated.
- After using the product, 75.49% of users stated, yes, they would buy this product instead of their usual product.
- After using the product, 86.27% of users stated, yes, they would recommend this product to a friend.
- After using the product, 46.08% of users stated, they looked at least 5 years younger.

### Reference Example 7: A user trial study in healthy volunteers to investigate the efficacy of a formulation comprising salicylate and tocopherol in improving skin tone.

This study tested the efficacy of a formulation comprising tocopherol in the continuous phase and two types of colloidal particles: 1) Tethersomes to which tridecyl salicylate is tethered and 2) Tethersomes to which palmitoyl ascorbate and tocopheryl linoleate are tethered.

### Methods

### Summary protocol

| Study design: | Single-blind |
|---|---|
| Test article: | CBL-DERM-15-014 skin tone serum |
| Duration of treatment: | 6 weeks |
| Number of subjects: | 110 |
| Type of subjects: | A user trial study in 110 healthy male (20%) and female (80%) subjects aged between 20 and 70 years old (equal proportions of each 10 year age group; 20's, 30's, 40's, 50's, 60's, 70's); from a breadth of ethnicities with a variety of skin types who suffer |
| | from one pronounced or two out of the following six conditions; melasma or chloasma spots, uneven skin tone, age, sun or "liver" spots, freckles, post-inflammatory hyperpigmentation or periorbital hyperpigmentation (dark circles). |
| Observations: | Subjects were asked to apply the product as per usage instructions. They were asked to complete a Self-Perception Questionnaire (SPQ) at the end of Weeks 3 and 6. |
| Treatment: | Subjects were issued with samples of the test article and directions for use following standard in-use application regime. |
| Location | Princeton Consumer Research |
| | Harbour House |
| | 23 Chandlers Quay |
| | Maldon |
| | CM9 4LF |
| | United Kingdom |

### Formulation (CBL-DERM-15-014)

| **Organic Phase - Tridecyl Salicylate Intermediate** | **Quantity Required Per 100g Final Product (g)** |
|---|---|
| Soy phosphatidylcholine (SPC) | 3.43500 |
| Ethanol | 1.67550 |
| Butylhydroxyanisole (BHA) | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.42500 |
| Tridecyl Salicylate | 0.10000 |
| +/- alpha tocopherol | 0.05000 |
| Linalool | 0.05000 |
| **Organic Phase Sub-Total** | **6.25800** |
| | |

| **Aqueous Phase - Tridecyl Salicylate Intermediate** | |
|---|---|
| Citric acid monohydrate | 0.03600 |
| Disodium EDTA | 0.05000 |
| Sodium metabisulphite Disodium hydrogen orthophosphate | 0.02500 |
| dodecahydrate | 0.14400 |
| Water | 22.65350 |
| **Aqueous Phase Sub-Total** | **22.90850** |
| | |

| **Tridecyl Salicylate Tethersome Intermediate** | |
|---|---|
| **Total** | **29.16650** |
| | |

| **Organic Phase** - **PAA/Tocopheryl Linoleate Intermediate** | |
|---|---|
| Soy phosphatidylcholine (SPC) | 3.43500 |
| Ethanol | 1.57950 |
| Butylhydroxyanisole (BHA) | 0.01000 |
| Methyl-4-hydroxybenzoate | 0.12500 |
| Ethyl-4-hydroxybenzoate | 0.12500 |
| Benzyl alcohol | 0.26250 |
| Polysorbate 80 | 0.42500 |
| Palmitoyl ascorbic acid | 0.09600 |
| Tocopheryl linoleate | 0.10000 |
| +/- alpha tocopherol | 0.05000 |
| Linalool | 0.05000 |
| **Organic Phase Sub-Total** | **6.25800** |
| | |

| **Aqueous Phase** - **PAA/Tocopheryl Linoleate Intermediate** | |
|---|---|
| Citric acid monohydrate | 0.03600 |
| Disodium EDTA | 0.05000 |
| Sodium metabisulphite | 0.02500 |
| Disodium hydrogen orthophosphate dodecahydrate | 0.14400 |
| Water | 22.65350 |
| **Aqueous Phase Sub-Total** | **22.90850** |
| | |
| **PAA/Tocopheryl Linoleate Tethersome** | |
| **Intermediate Total** | **29.16650** |
| **Sum of Sequessome and Tethersome Intermediates** | **58.33300** |
| **Gel Phase - Final Product** | |
| Sodium hydroxide | 0.16000 |
| Carbopol 974P NF | 0.75000 |
| Glycerol | 3.00000 |
| Citric acid monohydrate | 0.05600 |
| Disodium hydrogen orthophosphate dodecahydrate | 0.24200 |
| Water | 37.45900 |
| **Gel Phase Sub-Total** | **41.66700** |
| **Overall Total** | **100.00000** |

### Summary formulae of CBL-DERM-15-014

| **Ingredient (percentage; g per 100g)** | **Uneven Skin Tone **** |
|---|---|
| SPC (dry mass) | 6.870 |
| Polysorbate 80 | 0.850 |
| Benzylalcohol | 0.525 |
| Methyl-4-hyd roxybenzoate | 0.250 |
| Ethyl-4-hyd roxybenzoate | 0.250 |
| Butylhydroxyanisole | 0.020 |
| Linalool | 0.100 |
| Sodium metabisulphite | 0.050 |
| Disodium EDTA | 0.100 |
| Disodium hydrogen phosphate 12 H₂O | 0.530 |
| Citric acid monohydrate | 0.128 |
| Glycerol | 3.000 |
| Ethanol *** | 3.255 |
| Sodium hydroxide | 0.160 |
| Carbopol 974P NF | 0.750 |
| Water | 82.766 |
| Agent of interest - tethered to Tethersomes | |
| Palmitoyl ascorbate | 0.096 |
| Tocopheryl Linoleate | 0.100 |
| Tridecyl Salicylate | 0.100 |
| Agent of interest - in continuous phase | |
| Tocopherol | 0.100 |
| pH | 5.5* |

| | |
|---|---|
| * Final pH approximate; to be confirmed ** The source of SC dry mass for Uneven Skin Tone will be lipoid Phospholipon 90K (P90K). The quantity of P90K and ethanol to be added should be calculated accordingly. | |

### Results

The product performed statistically favourably over the 6 week study in the majority of attributes under Clearcast guidelines of advertising. The product did show a preference or favourability in the majority of attributes:
- After using the product, 100% of users agreed, or strongly agreed the product reduced the appearance of fine lines and wrinkles.
- After using the product, 100% of users agreed, or strongly agreed their skin looked significantly smoother.
- After using the product, 100% of users agreed, or strongly agreed their skin felt significantly more healthy.
- After using the product, 96.36% of users agreed, or strongly agreed their skin felt more elastic.
- After using the product, 99.09% of users agreed, or strongly agreed their complexion looks younger.
- After using the product, 99.09% of users agreed, or strongly agreed their complexion is visibly improved.
- After using the product, 100% of users agreed, or strongly agreed their complexion was visibly more radiant.
- After using the product, 100% of users agreed, or strongly agreed their complexion looked significantly healthier.
- After using the product, 98.18% of users agreed, or strongly agreed their skin tone looked significantly more even.
- After using the product, 96.36% of users agreed, or strongly agreed that the amount of hyperpigmentation/pigmented skin blemishes were significantly reduced.
- After using the product, 97.27% of users agreed, or strongly agreed that the size of hyperpigmentation/pigmented skin blemishes were significantly reduced.
- After using the product, 99.09% of users agreed, or strongly agreed that hyperpigmentation/pigmented skin blemishes were significantly lighter.
- After using the product, 20.00% of users agreed, or strongly agreed that hyperpigmented/pigmented skin blemishes totally disappeared.
- After using the product, 100% of users agreed, or strongly agreed their periorbital dark circles got significantly lighter.
- After using the product, 95.45% of users agreed, or strongly agreed this was the most effective hyperpigmentation solution they had used.
- After using the product, 92.73% of users stated, yes, they would buy this product instead of their usual product.
- After using the product, 100% of users stated, yes, they would recommend this product to a friend.

With reference to packaging / promotional claims, any results with a top 3 & 4 scoring greater than 80% are highly favourable.

## Claims

1. A formulation comprising a first colloidal dispersion and a second colloidal dispersion,
wherein each colloidal dispersion comprises deformable colloidal particles comprising a surfactant and optionally a phospholipid, and
wherein one or more of the deformable colloidal particles of the first colloidal dispersion comprise a modified component comprising a first Agent of Interest (AOI), wherein the modified component is a lipid or surfactant covalently bonded to an AOI, such that the entire AOI is outside the particle membrane, and
wherein the first AOI is zinc.

2. A formulation as claimed in claim 1, wherein one or more of the deformable colloidal particles of the second colloidal dispersion comprise a modified component comprising a second AOI and wherein the first AOI and the second AOI are different, wherein the modified component is a lipid or surfactant bonded to an AOI.

3. A formulation as claimed in claim 1 or claim 2, wherein one or more of the deformable colloidal particles of the first colloidal dispersion and/or one or more of the deformable colloidal particles of the second colloidal dispersion comprises modified components comprising one or more further AOIs and wherein each AOI is different, wherein the modified component is a lipid or surfactant bonded to an AOI.

4. A formulation as claimed in claim 1, claim 2 or claim 3, wherein the formulation comprises one or more further colloidal dispersions comprising deformable colloidal particles comprising a surfactant and optionally a phospholipid, and wherein one or more of the particles of at least two of the dispersions comprises modified components comprising one or more AOIs and wherein the first AOI, second AOI and each further AOI is different, wherein the modified component is a lipid or surfactant bonded to an AOI.

5. A formulation as claimed in any preceding claim, wherein the particle comprises both a modified surfactant component of the vesicle membrane and a modified lipid component of the vesicle membrane.

6. A formulation as claimed in any preceding claim, wherein the second AOI is selected from the group consisting of an element, an ion, an inorganic salt, a small molecule, an amino acid, a peptide, a protein, a carbohydrate, a lipid, a micronutrient, a macromolecule or a macrocyclic molecule, optionally wherein the second AOI and/or each further AOI is selected from the group consisting of zinc, ascorbate, tetrapeptide, tripeptide, salicylic acid, Vitamin D, tocopherol or menthol.

7. A formulation as claimed in any preceding claim, wherein the formulation comprises chlorhexidine, salicylic acid, capsaicin, caffeine or tocopherol, optionally wherein the chlorhexidine, salicylic acid, capsaicin, caffeine or tocopherol is not associated with the deformable colloidal particles.

8. A formulation as claimed in any preceding claim, wherein the formulation is topically applied.

9. A formulation as claimed in any preceding claim for use in treating or preventing a disease.

10. A formulation as claimed in any preceding claim for use in:
(i) treating or preventing acne, or
(ii) treating or preventing dry skin, itchy skin, red skin, irritated skin, scaly skin, ageing skin, thinning skin, uneven skin tone, periorbital skin, photo-aged skin or dermatitis.

11. A formulation as claimed in any of claims 1 to 8 for use in skin care and/or cosmetics.

12. A formulation as claimed in claim 11 for use in improving the appearance of the skin, wherein the formulation comprises chlorhexidine or a salt thereof, optionally wherein the composition improves the appearance of the skin by one or more of treating or preventing acne, treating or preventing the formation of spots, blackheads and blemishes, reducing skin impurities and tightening and constricting pores in the skin.

13. A formulation as claimed in claim 11 for use in improving the appearance of uneven skin tone, wherein the formulation comprises salicylic acid or a salt or ester thereof, optionally wherein the formulation further comprises ascorbic acid and tocopherol or a derivative thereof, optionally wherein the formulation improves the appearance of uneven skin tone in one or more of the following ways; reducing the appearance of wrinkles, hyperpigmentation, blemishes, dark under-eye circles, improving the appearance of skin texture and increasing skin elasticity.

14. A formulation as claimed in claim 11 for use in improving the appearance of periorbital skin, wherein the formulation comprises caffeine, optionally wherein the formulation improves the appearance of periorbital skin in one or more of the following ways; reducing the appearance of wrinkles, eye puffiness, lower eyelid bags or sagging and dark under-eye circles, improving the appearance of skin texture and preventing sun damage.

15. A formulation as claimed in any of claims 1 to 8 for cosmetic treatment of a subject.

## Patentansprüche

1. Formulierung, umfassend eine erste kolloidale Dispersion und eine zweite kolloidale Dispersion, wobei die kolloidalen Dispersionen jeweils verformbare kolloidale Partikel umfassen, die ein Tensid und gegebenenfalls ein Phospholipid umfassen, und
wobei eines oder mehrere der verformbaren kolloidalen Partikel der ersten kolloidalen Dispersion eine modifizierte Komponente umfassen, die ein erstes Agens von Interesse (Agent of Interest, AOI) umfasst, wobei es sich bei der modifizierten Komponente um ein Lipid oder Tensid handelt, das kovalent an ein AOI so gebunden ist, dass sich das gesamte AOI außerhalb der Partikelmembran befindet, und
wobei es sich bei dem ersten AOI um Zink handelt.

2. Formulierung nach Anspruch 1, wobei eines oder mehrere der verformbaren kolloidalen Partikel der zweiten kolloidalen Dispersion eine modifizierte Komponente umfassen, die ein zweites AOI umfasst, und wobei das erste AOI und das zweite AOI verschieden sind, wobei es sich bei der modifizierten Komponente um ein Lipid oder Tensid handelt, das an ein AOI gebunden ist.

3. Formulierung nach Anspruch 1 oder Anspruch 2, wobei eines oder mehrere der verformbaren kolloidalen Partikel der ersten kolloidalen Dispersion und/oder eines oder mehrere der verformbaren kolloidalen Partikel der zweiten kolloidalen Dispersion modifizierte Komponenten umfassen, die ein oder mehrere weitere AOI umfassen, und wobei die AOI jeweils verschieden sind, wobei es sich bei der modifizierten Komponente um ein Lipid oder Tensid handelt, das an ein AOI gebunden ist.

4. Formulierung nach Anspruch 1, Anspruch 2 oder Anspruch 3, wobei die Formulierung eine oder mehrere weitere kolloidale Dispersionen umfassen, die verformbare kolloidale Partikel umfassen, die ein Tensid und gegebenenfalls ein Phospholipid umfassen, und wobei eines oder mehrere der Partikel von wenigstens zwei der Dispersionen modifizierte Komponenten umfassen, die ein oder mehrere AOI umfassen, und wobei das erste AOI, zweite AOI und alle weiteren AOI jeweils verschieden sind, wobei es sich bei der modifizierten Komponente um ein Lipid oder Tensid handelt, das an ein AOI gebunden ist.

5. Formulierung nach einem vorhergehenden Anspruch, wobei das Partikel sowohl eine modifizierte Tensidkomponente der Vesikelmembran als auch eine modifizierte Lipidkomponente der Vesikelmembran umfasst.

6. Formulierung nach einem vorhergehenden Anspruch, wobei das zweite AOI ausgewählt ist aus der Gruppe bestehend aus einem Element, einem Ion, einem anorganischen Salz, einem kleinen Molekül, einer Aminosäure, einem Peptid, einem Protein, einem Kohlenhydrat, einem Lipid, einem Mikronährstoff, einem Makromolekül oder einem makrocyclischen Molekül, gegebenenfalls wobei das zweite AOI und/oder jedes weitere AOI ausgewählt ist aus der Gruppe bestehend aus Zink, Ascorbat, Tetrapeptid, Tripeptid, Salicylsäure, Vitamin D, Tocopherol oder Menthol.

7. Formulierung nach einem vorhergehenden Anspruch, wobei die Formulierung Chlorhexidin, Salicylsäure, Capsaicin, Coffein oder Tocopherol umfasst, gegebenenfalls wobei das/die Chlorhexidin, Salicylsäure, Capsaicin, Coffein bzw. Tocopherol nicht mit den verformbaren kolloidalen Partikeln assoziiert ist.

8. Formulierung nach einem vorhergehenden Anspruch, wobei die Formulierung topisch angewandt wird.

9. Formulierung nach einem vorhergehenden Anspruch zur Verwendung bei der Behandlung oder Vorbeugung einer Krankheit.

10. Formulierung nach einem vorhergehenden Anspruch zur Verwendung bei:
(i) Behandlung oder Vorbeugung von Akne oder
(ii) Behandlung oder Vorbeugung von trockener Haut, juckender Haut, Hautrötungen, gereizter Haut, schuppiger Haut, alternder Haut, dünner werdender Haut, ungleichmäßiger Hauttönung, Periorbitalhaut, lichtgealterter Haut oder Dermatitis.

11. Formulierung nach einem der Ansprüche 1 bis 8 zur Verwendung in der Hautpflege und/oder Kosmetik.

12. Formulierung nach Anspruch 11 zur Verwendung beim Verbessern des Aussehens der Haut, wobei die Formulierung Chlorhexidin oder ein Salz davon umfasst, gegebenenfalls wobei die Zusammensetzung das Aussehen der Haut durch eine oder mehrere von Behandlung oder Vorbeugung von Akne, Behandlung oder Vorbeugung der Ausbildung von Pickeln, Mitessern und Makeln, Verringerung von Hautunreinheiten und Zusammenziehen und Verengen der Poren in der Haut.

13. Formulierung nach Anspruch 11 zur Verwendung beim Verbessern des Aussehens ungleichmäßiger Hauttönung, wobei die Formulierung Salicylsäure oder ein Salz bzw. einen Ester davon umfasst, gegebenenfalls wobei die Formulierung ferner Ascorbinsäure und Tocopherol oder ein Derivat davon umfasst, gegebenenfalls wobei die Formulierung das Aussehen ungleichmäßiger Hauttönung auf eine oder mehrere der folgenden Arten und Weisen verbessert: Vermindern des Auftretens von Falten, Hyperpigmentierung, Makeln, dunklen Ringen unter dem Auge, Verbessern des Hautbilds und Erhöhen der Hautelastizität.

14. Formulierung nach Anspruch 11 zur Verwendung beim Verbessern des Aussehens von Periorbitalhaut, wobei die Formulierung Coffein umfasst, gegebenenfalls wobei die Formulierung das Aussehen von Periorbitalhaut auf eine oder mehrere der folgenden Arten und Weisen verbessert: Vermindern des Auftretens von Falten, Augenschwellungen, Schlupflidern oder schlaffen unteren Lidern und dunklen Ringen unter dem Auge, Verbessern des Hautbilds und Vorbeugen von Schäden durch Sonneneinwirkung.

15. Formulierung nach einem der Ansprüche 1 bis 8 zur kosmetischen Behandlung eines Individuums.

## Revendications

1. Formulation comprenant une première dispersion colloïdale et une deuxième dispersion colloïdale,
dans laquelle chaque dispersion colloïdale comprend des particules colloïdales déformables comprenant un tensioactif et éventuellement un phospholipide, et
dans laquelle une ou plusieurs des particules colloïdales déformables de la première dispersion colloïdale comprennent un composant modifié comprenant un premier agent d'intérêt (AOI), le composant modifié étant un lipide ou un tensioactif lié par liaison covalente à un AOI, de telle sorte que l'AOI dans sa totalité soit à l'extérieur de la membrane des particules, et
dans laquelle le premier AOI est le zinc.

2. Formulation selon la revendication 1, dans laquelle une ou plusieurs des particules colloïdales déformables de la deuxième dispersion colloïdale comprennent un composant modifié comprenant un deuxième AOI et dans laquelle le premier AOI et le deuxième AOI sont différents, le premier composant étant un lipide ou un tensioactif lié à un AOI.

3. Formulation selon la revendication 1 ou la revendication 2, dans laquelle une ou plusieurs des particules colloïdales déformables de la première dispersion colloïdale et/ou une ou plusieurs des particules colloïdales déformables de la deuxième dispersion colloïdale comprennent des composants modifiés comprenant un ou plusieurs AOI supplémentaires, et dans laquelle chaque AOI est différent, le composant modifié étant un lipide ou un tensioactif lié à un AOI.

4. Formulation selon la revendication 1, la revendication 2 ou la revendication 3, la formulation comprenant une ou plusieurs dispersions colloïdales supplémentaires comprenant des particules colloïdales déformables comprenant un tensioactif et éventuellement un phospholipide, et dans laquelle une ou plusieurs des particules d'au moins deux des dispersions comprennent des composants modifiés comprenant un ou plusieurs AOI, et le premier AOI, le deuxième AOI et chaque AOI supplémentaire étant différents, le composant modifié étant un lipide ou un tensioactif lié à un AOI.

5. Formulation selon l'une quelconque des revendications précédentes, dans laquelle la particule comprend tant un composant tensioactif modifié de la membrane vésiculaire qu'un composant lipide modifié de la membrane vésiculaire.

6. Formulation selon l'une quelconque des revendications précédentes, dans laquelle le deuxième AOI est choisi dans le groupe consistant en un élément, un ion, un sel inorganique, une petite molécule, un acide aminé, un peptide, une protéine, un glucide, un lipide, un micronutriment, une macromolécule ou une molécule macrocyclique, éventuellement dans laquelle le deuxième AOI et/ou chaque AOI supplémentaire sont choisis dans le groupe consistant en le zinc, un ascorbate, un tétrapeptide, un tripeptide, l'acide salicylique, la vitamine D, le tocophérol ou le menthol.

7. Formulation selon l'une quelconque des revendications précédentes, dans laquelle la formulation comprend de la chlorhexidine, de l'acide salicylique, de la capsaïcine, de la caféine ou du tocophérol, éventuellement dans laquelle la chlorhexidine, l'acide salicylique, la capsaïcine, la caféine ou le tocophérol ne sont pas associés aux particules colloïdales déformables.

8. Formulation selon l'une quelconque des revendications précédentes, la formulation étant appliquée par voie topique.

9. Formulation selon l'une quelconque des revendications précédentes pour une utilisation dans le traitement et la prévention d'une maladie.

10. Formulation selon l'une quelconque des revendications précédentes pour une utilisation dans
(i) le traitement ou la prévention de l'acné, ou
(ii) le traitement ou la prévention de la peau sèche, de la peau prurigineuse, de la peau rouge, de la peau irritée, de la peau écailleuse, de la peau vieillissante, de la peau amincissante, du teint irrégulier, de la peau périorbitaire, de la peau photo-vieillie ou de la dermatite.

11. Formulation selon l'une quelconque des revendications 1 à 8 pour une utilisation dans les soins de la peau et/ou en cosmétique.

12. Formulation selon la revendication 11 pour une utilisation dans l'amélioration de l'aspect de la peau, la formulation comprenant de la chlorhexidine ou un sel de celle-ci, éventuellement dans laquelle la composition améliore l'aspect de la peau par un ou plusieurs parmi le traitement ou la prévention de l'acné, le traitement ou la prévention des taches, des comédons et des imperfections, la réduction des impuretés cutanées et le resserrement et la constriction des pores de la peau.

13. Formulation selon la revendication 11 pour une utilisation dans l'amélioration de l'aspect de teint irrégulier, la formulation comprenant de l'acide salicylique ou un sel ou un ester de celui-ci, éventuellement la formulation comprenant en outre de l'acide ascorbique et du tocophérol ou un dérivé de ceux-ci, éventuellement la formulation améliorant l'aspect d'un teint irrégulier par une ou plusieurs des techniques suivantes : réduction de l'aspect des rides, d'une hyperpigmentation, des imperfections, des cernes foncés sous les yeux, amélioration de l'aspect de la texture de la peau et augmentation de l'élasticité de la peau.

14. Formulation selon la revendication 11 pour une utilisation dans l'amélioration de l'aspect de la peau périorbitaire, la formulation comprenant de la caféine, éventuellement la formulation améliorant l'aspect de la peau périorbitaire par une ou plusieurs des techniques suivantes : réduction de l'aspect des rides, du gonflement des yeux, des poches des paupières inférieures, ou de l'affaissement, et des cernes foncés sous les yeux, l'amélioration de l'aspect de la texture de la peau et la prévention des lésions dues au soleil.

15. Formulation selon l'une quelconque des revendications 1 à 8 pour le traitement cosmétique d'un sujet.
